# EUROPEAN PATENT SPECIFICATION

(11) **EP 2 101 813 B1**
(45) Date of publication and mention of the grant of the patent: **02.04.2014**
(21) Application number: 07874550.2
(22) Date of filing: 27.11.2007
(51) Int. Cl.: A61K 39/00

(54) **NOVEL GLYCOSYLATED PEPTIDE TARGET IN NEOPLASTIC CELLS**
NEUES GLYKOSYLIERTES ZIELPEPTID IN NEOPLASTISCHEN ZELLEN
NOUVELLE CIBLE DE PEPTIDE GLYCOSYLÉ DANS DES CELLULES NÉOPLASIQUES

(30) Priority: 27.11.2006 US 867285 P
(43) Date of publication of application: 23.09.2009
(60) Divisional application: 11186480.7
(73) Proprietor: PATRYS LIMITED, Melbourne VIC 3000 (AU)
(72) Inventor: VOLLMERS, Heinz Peter, 97078 Würzburg (DE)
(74) Representative: Cohausz & Florack
(86) International application number: PCT/IB2007/004632
(87) International publication number: WO 2008/152446

(56) References cited:
- WO-A-2005/047332
- T. POHLE ET AL: "Lipoptosis: Tumor specific cell death by antibody-induced intracellular lipid accumulation" CANCER RESEARCH, vol. 64, 1 June 2004 (2004-06-01), pages 3900-3906, XP002520042
- VOLLMERS ET AL: "Natural antibodies and cancer" JOURNAL OF AUTOIMMUNITY, LONDON, GB, vol. 29, no. 4, 26 October 2007 (2007-10-26), pages 295-302, XP022317196 ISSN: 0896-8411
- RAUSCHERT N. ET AL.: "A new tumor specific variant of GRP78 as target for antibody-based therapy" LABORATORY INVESTIGATION, vol. 88, April 2008 (2008-04), pages 375-386, XP002520043
- J. HONG ET AL: "A two dimensional gel database of human carcinoma proteins" ELECTROPHORESIS, vol. 18, 1997, pages 605-613, XP002520136
- DATABASE NCBI 1 July 1989 (1989-07-01), "78 Kda glucose related protein precursor (GRP78) (Heat shock 70 Kda protein 5) (HSPA5) (Immunoglobulin heavy chain binding protein) (BiP)" XP002520137 Database accession no. p11021

## Description

### Field of the Invention

The invention relates to a glycosylated peptide or glycoprotein that binds to an antibody, known as SAM-6. The glycoprotein, denoted SAM-6 Receptor (SAM-6/R) or SAM-6/R glycoprotein has apparent sequence homology/shares identity with Grp78 (glucose regulated protein 78) polypeptide sequence.

### Introduction

There is a need for methods of treating undesirable or aberrant cell proliferation, such as cellular hyperproliferative disorders, including neoplasias, tumors and cancers. The invention addresses this need and provides related benefits.

CANCER RESEARCH 64, 3900-3906, June 1, 2004 describes the tumor-specific cell death by antibody-induced intracellular lipid accumulation. The protein disclosed in this document has a molecular weight of 140 kDa. WO 2005/047332 A1 shows an antibody which however does not have a heavy chain sequence identical to the heavy chain sequence as disclosed herein. Electrophoresis 1997, 18, 605-613 discloses a human GRP 78 Protein identified in a two-dimensional gel of a human colon carcinoma cell line LIM 1215. This protein has a molecular mass of 76.0 kDa and the amino acid sequence is only 22 amino acid residues long.

### Summary

The invention provides an isolated or purified glycoprotein denoted as SAM-6 Receptor (SAM-6/R), wherein said SAM-6/R glycoprotein has an apparent molecular weight in a range of about 80-82 kilodaltons (kDa) as determined by denaturing gel electrophoresis, a sequence comprising SEQ ID NO:1, has at least one nitrogen (N)- or oxygen (O)-linked carbohydrate moiety distinct from Grp78, wherein an antibody denoted SAM-6 specifically binds to the glycoprotein, and wherein treatment of the SAM-6/R glycoprotein with an O-glycosidase reduces binding of the SAM-6 antibody to the glycoprotein, wherein the SAM-6 antibody comprises:
(a) a heavy chain variable region sequence, wherein the heavy chain variable region sequence comprises the amino acid sequence shown in SEQ ID NO:15, and
(b) a light chain variable region sequence, wherein the light chain variable region sequence comprises the amino acid sequence shown in SEQ ID NO:13.
The SAM-6/R glycoprotein has an apparent molecular weight in a range of about 80-82 kilodaltons 20 (kDa) as determined by denaturing gel electrophoresis, at least one nitrogen (N)- or oxygen (0)-linked carbohydrate moiety distinct from Grp78, and an antibody denoted SAM-6 specifically binds to the SAM-6/R glycoprotein. In another embodiment, a SAM-6/R glycoprotein has an apparent molecular weight in a range of about 80-82 kilodaltons (kDa) as determined by denaturing gel electrophoresis, at least one 25 nitrogen (N)- or oxygen (0)-linked carbohydrate moiety distinct from a carbohydrate moiety of Grp78, and polypeptide sequence homology to Grp78 as set forth in SEQ ID NO:1 (*e.g*., at least 60%, 70%, 80%, 90%, 95% or more identity).

In various aspects, isolated and purified glycoproteins denoted as SAM-6 Receptor (SAM-6/R) or SAM-6/R glycoprotein include a sequence of about 655 amino acids, have a transmembrane domain of about 17 amino acids, have an extracellular domain of about 220 amino acids, or have an intracellular domain of about 411 amino acids. In various additional aspects, SAM-6 Receptor (SAM-6/R) or SAM-6/R glycoprotein is characterized as being expressed on a neoplastic, cancer or tumor cell, for example, a pancreas carcinoma cell line or gastric carcinoma cell line denoted respectively as BXPC-3 (ATCC Deposit No. CRL-1687) and 23132/87 (DSMZ Deposit No. ACC 201).

In another aspect, the linked carbohydrate moiety of isolated and purified glycoproteins denoted as SAM-6 Receptor (SAM-6/R) or SAM-6/R glycoprotein is linked to an asparagine, serine or threonine residue (e.g. of SEQ ID NO:1). In a further aspect, the SAM-6 antibody binds to a portion of the glycoprotein that includes the N- or O-linked carbohydrate moiety, or binds to the N- or O-linked carbohydrate moiety. In additional aspects, treatment of SAM-6 Receptor (SAM-6/R) or SAM-6/R glycoprotein with a glycosidase enzyme (*e.g*., an O-glycosidase or an N-glycosidase, such as endoglycosidase H or endoglycosidase F) reduces the apparent molecular weight of the glycoprotein by about 1-5 kilodaltons (KDa), or reduces binding of SAM-6 antibody to the SAM-6 Receptor (SAM-6/R) or SAM-6/R glycoprotein. In a still further aspect, the N- or O-linked carbohydrate moiety includes one or more of galactose, acetylgalactose, mannose, fucose, glucose, acetylglucose, sialic acid, N-acetylgalactosamine, or N-acetylglucosamine.

The invention further provides nucleic acid sequences that encode glycoprotein SAM-6 Receptor (SAM-6/R) or SAM-6/R glycoprotein. In one embodiment, a nucleic acid sequence is at least 75-90%, or more, complementary or homologous to a nucleic acid sequence that encodes SEQ ID NO:1, or a subsequence thereof. In another embodiment, a nucleic acid sequence encodes a SAM-6 Receptor (SAM-6/R) or SAM-6/R glycoprotein capable of having linked thereto at least one nitrogen (N)- or oxygen (O)-linked carbohydrate moiety distinct from a carbohydrate moiety of Grp78. In particular aspects a nucleic acid encodes a subsequence of SAM-6 Receptor (SAM-6/R) or SAM-6/R glycoprotein. In other aspects, a nucleic acid sequence has a length from about 10-20, 20-30, 30-50, 50-100, 100-150, 150-200, 200-250, 250-300, 300-400, 400-500, 500-1000, 1000-2000 nucleotides. In additional aspects, a nucleic acid sequence specifically hybridizes to a nucleic acid that encodes SEQ ID NO:1, or a subsequence thereof, or specifically hybridizes to a nucleic acid sequence complementary to a nucleic acid that encodes SEQ ID NO:1, or a subsequence thereof. In further aspects, a nucleic acid is an antisense polynucleotide, a small interfering RNA, or a ribozyme nucleic acid that specifically hybridizes to a nucleic acid sequence encoding SAM-6 Receptor (SAM-6/R) or SAM-6/R glycoprotein and reduces expression of the SAM-6 Receptor (SAM-6/R) or SAM-6/R glycoprotein. Antisense polynucleotides, small interfering RNA, and ribozyme polynucleotides can have a length from about 10-20, 20-30, 30-50, 50-100, 100-150, 150-200, 200-250, 250-300, 300-400, 400-500, 500-1000, 1000-2000 nucleotides, and be at least 90% complementary or homologous to a nucleic acid sequence that encodes SEQ ID NO:1, or a subsequence thereof. In still further aspects, nucleic acid sequence can include an expression control sequence or a vector (*e.g*., a viral, bacterial, fungal or mammalian vector).

The invention additionally provides host cells transformed with nucleic acid that encodes SAM-6 Receptor (SAM-6/R) or SAM-6/R glycoprotein or a subsequence thereof. Host cells include eukaryotic (*e.g*., a hyperproliferative cell, immortalized cell, neoplastic cell, tumor cell or cancer cell) and non-eukaryotic cells, which can be stably or transiently transformed with the nucleic acid or vector.

The invention moreover provides isolated and purified polyclonal and monoclonal antibodies that specifically bind to SAM-6 Receptor (SAM-6/R) or SAM-6/R glycoprotein or a subsequence thereof. The antibody of the invention is directed to an isolated or purified antibody or subsequence thereof comprising:
(a) a heavy chain variable region sequence, wherein the heavy chain variable region sequence comprises:
   (i) an amino acid sequence which is at least 90% identical to the sequence shown in SEQ ID NO:18, and
   (ii) a complementarity determining region (CDR) with 100% identity to the CDR3 (ARDRLAVAGRPFDY) shown in SEQ ID NO:17; and
(b) a light chain variable region sequence, wherein the light chain variable region sequence comprises an amino acid sequence which is at least 90% identical to the sequence shown in SEQ ID NO:13.

In one embodiment, an antibody specifically binds to a SAM-6 Receptor (SAM-6/R) or SAM-6/R glycoprotein epitope including at least one N- or O-linked carbohydrate moiety (*e.g*., extracellular domain). In another embodiment, an antibody does not bind to an epitope comprising an N- or O-linked carbohydrate moiety. In a further embodiment, an antibody binds to LDL (*e.g*., oxidized LDL, "oxLDL"). In an additional embodiment, an antibody competes for binding of SAM-6 antibody to the SAM-6 Receptor (SAM-6/R) or SAM-6/R glycoprotein or a subsequence thereof, or inhibits or blocks binding of SAM-6 antibody (*e.g*., at least 50% of the binding of SAM-6 antibody) to SAM-6 Receptor (SAM-6/R) or SAM-6/R glycoprotein or a subsequence thereof (*e.g*., as determined in an ELISA assay), or competes for binding of SAM-6 antibody to LDL (*e.g*., oxLDL), or inhibits or blocks binding of SAM-6 antibody (*e.g*., at least 50% of the binding of SAM-6 antibody) to LDL or oxLDL (*e.g*., as determined in an ELISA assay). In still additional embodiments, an antibody binds to cells expressing SAM-6 Receptor (SAM-6/R) or SAM-6/R glycoprotein (*e.g*., a neoplastic, cancer or tumor cell or cell line such as BXPC-3 or 23132/87 cells) and stimulates or induces cell death, lysis or apoptosis, or activation of a caspase (e.g. caspase-3, caspase-8 or *caspase-9) in vitro* or *in vivo.* In still another embodiment, treatment of SAM-6 Receptor (SAM-6/R) or SAM-6/R glycoprotein with an O-glycosidase reduces binding of the antibody to the SAM-6 Receptor (SAM-6/R) or SAM-6/R glycoprotein, or to LDL or oxLDL. In still additional embodiments, the antibody has a binding affinity for SAM-6 Receptor (SAM-6/R) or SAM-6/R glycoprotein within about 1-5000 fold of the binding affinity of SAM-6, or a binding affinity for SAM-6 Receptor (SAM-6/R) or SAM-6/R glycoprotein within about KD 10⁻⁶ M to about KD 10⁻¹³ M of SAM-6. In yet further embodiments, the antibody has a binding affinity for LDL or oxLDL within about 1-5000 fold of the binding affinity of SAM-6, or a binding affinity for LDL or oxLDL within about KD 10⁻⁶ M to about KD 10⁻¹³ M of SAM-6.

Antibodies of the invention are distinct from SAM-6 antibody. In one embodiment, an antibody does not have heavy and light chain sequences identical to heavy and light chain sequences of SAM-6. In another embodiment, an antibody does not have heavy or light chain variable sequences identical to heavy or light chain variable sequences of SAM-6. In a further embodiment, an antibody does not have 90%-95%, 96%, 97%, 98%, 99%, or more identity to heavy or light chain variable region sequence of SAM-6 antibody. In a particular embodiment, an antibody that is distinct from SAM-6 antibody has a heavy chain sequence with 100% identity to a heavy chain variable region sequence or a CDR (CDR3; ARDRLAVAGRPFDY; SEQ ID NO:17) within a heavy chain variable region amino acid sequence set forth as SEQ ID NO: 18.

Antibodies of the invention include IgG, IgA, IgM, IgE and IgD. In various aspects, an IgG is an IgG1, IgG2, IgG3, or IgG4.

Antibodies of the invention also include antibody subsequences that bind to SAM-6 Receptor (SAM-6/R) or SAM-6/R glycoprotein or a subsequence (e.g., immunogenic fragment) thereof. In various aspects, a subsequence is an Fab, Fab', F(ab')₂, Fv, Fd, single-chain Fvs (scFv), disulfide-linked Fvs (sdFv), V_{L} or V_{H}.

Further described herein is a SAM-6 Receptor (SAM-6/R) or SAM-6/R glycoprotein and subsequences thereof, antibodies and subsequences that bind to SAM-6 Receptor (SAM-6/R) or SAM-6/R glycoprotein that include a heterologous domain. In one embodiment, a heterologous domain includes a detectable label, tag or cytotoxic agent. In particular aspects, a detectable label or tag is an enzyme, enzyme substrate, ligand, receptor, radionuclide, a T7-, His-, myc-, HA- or FLAG-tag, electron-dense reagent, energy transfer molecule, paramagnetic label, fluorophore, chromophore, chemi-luminescent agent, or a bio-luminescent agent.

Further described herein are kits. In one embodiment, a kit includes an antibody that binds to SAM-6 Receptor (SAM-6/R) or SAM-6/R glycoprotein and instructions for detecting SAM-6 Receptor (SAM-6/R) or SAM-6/R glycoprotein. In another embodiment, a kit includes an antibody that binds to SAM-6 Receptor (SAM-6/R) or SAM-6/R glycoprotein and instructions for treating a condition treatable with an antibody that binds to bind to SAM-6 Receptor (SAM-6/R) or SAM-6/R glycoprotein. In additional embodiments, a kit also includes an anti-cell proliferative or immune enhancing treatment or therapeutic agent, or an anti-neoplastic, anti-cancer or anti-tumor agent, or an article of manufacture (*e.g*., for delivering the antibody, anti-cell proliferative or immune enhancing treatment or therapy into a subject locally, regionally or systemically). In particular aspects, the instructions are for treating undesirable cell proliferation or hyperproliferation or treating a neoplasia, tumor or cancer. In additional aspects, the instructions are on a label or packaging insert.

The invention yet additionally provides pharmaceutical compositions. In one embodiment, a composition includes SAM-6 Receptor (SAM-6/R) or SAM-6/R glycoprotein and a pharmaceutically acceptable carrier or excipient. In another embodiment, a composition includes an antibody that specifically binds to SAM-6 Receptor (SAM-6/R) or SAM-6/R glycoprotein and a pharmaceutically acceptable carrier or excipient.

Described herein are methods for treating a disorder in a subject in need of treatment. In one embodiment, a method includes administering SAM-6 Receptor (SAM-6/R) or SAM-6/R glycoprotein or an antibody that specifically binds to SAM-6 Receptor (SAM-6/R) or SAM-6/R glycoprotein in an amount effective to treat a cellular hyperproliferative disorder in a subject (*e.g*., a stage I, II, III, IV or V, metastatic or non-metastatic, solid or liquid neoplasia, tumor or cancer). In various aspects, a cellular hyperproliferative disorder affects or is present at least in part in brain, head or neck, breast, esophagus, mouth, nasopharynx, nose or sinuses, stomach, duodenum, ileum, jejunum, lung, liver, pancreas, kidney, adrenal gland, thyroid, bladder, colon, rectum, prostate, uterus, cervix, ovary, bone marrow, lymph, blood, bone, testes, skin or muscle, or hematopoetic system. In additional aspects, a cellular hyperproliferative disorder includes a neoplasia, tumor or cancer that affects or is at least in part present in breast, lung, thyroid, head and neck, nasopharynx, nose or sinuses, brain, spine, adrenal gland, thyroid, lymph, gastrointestinal tract, mouth, esophagus, stomach, duodenum, ileum, jejunum, small intestine, colon, rectum, genito-urinary tract, uterus, ovary, cervix, bladder, testicle, penis, prostate, kidney, pancreas, adrenal gland, liver, bone, bone marrow, lymph, blood, muscle, skin or is hematopoetic. In particular aspects, a neoplasia, tumor or cancer is a sarcoma, carcinoma, adenocarcinoma, melanoma, myeloma, blastoma, glioma, lymphoma leukemia. In additional particular aspects, a neoplasia, tumor or cancer is a lung adenocarcinoma, lung carcinoma, diffuse or interstitial gastric carcinoma, colon adenocarcinoma, prostate adenocarcinoma, esophagus carcinoma, breast carcinoma, pancreas adenocarcinoma, ovarian adenocarcinoma, or uterine adenocarcinoma. In further particular aspects, a neoplasia, tumor or cancer is progressively worsening or is in remission. In still additional aspects, treatment results in alleviating or ameliorating one or more adverse physical symptoms associated with a cellular hyperproliferative disorder, or a neoplasia, tumor or cancer, or reduces or decreases neoplasia, tumor or cancer volume, inhibits or prevents an increase in neoplasia, tumor or cancer volume, inhibits neoplasia, tumor or cancer progression or worsening, stimulates neoplasia, tumor or cancer cell lysis or apoptosis, or inhibits, reduces or decreases neoplasia, tumor or cancer proliferation or metastasis, or prolongs or extends lifespan of the subject, or improves the quality of life of the subject.

Methods include administration to the subject locally, regionally, or systemically. Exemplary subjects (*e.g*., mammals such as humans) include candidates for, and those undergoing, or having undergone an anti-cell proliferative or anti-cellular hyperproliferative disorder (*e.g*., anti-neoplastic, anti-tumor, anti-cancer or anti-metastasis) or immune-enhancing treatment or therapy.

The invention yet also provides combined methods for treating a disorder in a subject in need of treatment. In one embodiment, the SAM-6/R glycoprotein or an antibody that specifically binds to SAM-6 Receptor (SAM-6/R) or SAM-6/R glycoprotein for use in an anti-cell proliferative or immune-enhancing treatment or therapy to a subject (e.g., prior to, substantially contemporaneously with or following each other) is claimed. In various aspects, an anti-cell proliferative or immune-enhancing treatment or therapy includes surgical resection, radiotherapy, radiation therapy, chemotherapy, immunotherapy, hyperthermia, an alkylating agent, anti-metabolite, plant extract, plant alkaloid, nitrosourea, hormone, nucleoside or nucleotide analogue, a lymphocyte, plasma cell, macrophage, dendritic cell, NK cell or B-cell, an antibody, a cell growth factor, a cell survival factor, a cell differentiative factor, a cytokine or a chemokine.

The invention still additionally provides treating a disorder or disease associated with or caused by undesirable or excessive LDL or oxLDL levels. In one embodiment, a method includes administering to a subject an antibody that specifically binds to SAM-6 Receptor (SAM-6/R) or SAM-6/R glycoprotein in an amount effective to treat the disorder or disease associated with or caused by undesirable or excessive LDL or oxLDL levels in the subject. In various aspects, a disorder or disease associated with or caused by undesirable or excessive LDL or oxLDL levels is hyperlipidemia, hypercholesterolemia, arteriosclerosis, cardiovascular disease, coronary heart disease (CHD), stroke, glomerulonecrosis, high blood pressure or diabetes.

Further described are methods for detecting or screening for SAM-6 Receptor (SAM-6/R) or SAM-6/R glycoprotein. In one embodiment, a method includes contacting a biological material or sample with an antibody that specifically binds to SAM-6 Receptor (SAM-6/R) or SAM-6/R glycoprotein under conditions allowing binding of the antibody to the SAM-6 Receptor (SAM-6/R) or SAM-6/R glycoprotein, and assaying for binding of the antibody to SAM-6 Receptor (SAM-6/R) or SAM-6/R glycoprotein. The binding of the antibody to SAM-6 Receptor (SAM-6/R) or SAM-6/R glycoprotein detects the presence of SAM-6 Receptor (SAM-6/R) or SAM-6/R glycoprotein. In one aspect, SAM-6 Receptor (SAM-6/R) or SAM-6/R glycoprotein is present on a cell or tissue. In another aspect, the biological material or sample is obtained from a mammalian subject.

Described are also methods for diagnosing a subject having or at increased risk of having a cellular hyperproliferative disorder (e.g., neoplasia, tumor or cancer, or metastasis). In one embodiment, a method includes providing a biological material or sample from a subject, contacting the biological material or sample with an antibody that specifically binds to SAM-6 Receptor (SAM-6/R) or SAM-6/R glycoprotein under conditions allowing binding of the antibody to SAM-6 Receptor (SAM-6/R) or SAM-6/R glycoprotein, and assaying for binding of the antibody to SAM-6 Receptor (SAM-6/R) or SAM-6/R glycoprotein. The binding of the antibody to the polypeptide diagnoses the subject as having or at increased risk of having a neoplasia, tumor or cancer, or metastasis. In one aspect, the biological material or sample is obtained from a human. In additional aspects, the biological material or sample comprises a biopsy, such as a lung, pancreas, stomach, breast, esophageal, ovarian or uterine biopsy.

The invention additionally provides methods for producing an antibody that specifically binds to SAM-6 Receptor (SAM-6/R) or SAM-6/R glycoprotein. In one embodiment, a method includes administering SAM-6 Receptor (SAM-6/R) or SAM-6/R glycoprotein, or a fragment thereof, to an animal, screening the animal for expression of an antibody that binds to SAM-6 Receptor (SAM-6/R) or SAM-6/R glycoprotein, or the fragment thereof, selecting an animal that produces an antibody that binds to SAM-6 Receptor (SAM-6/R) or SAM-6/R glycoprotein, or the fragment thereof, and isolating the antibody from the selected animal. In another embodiment, a method includes administering SAM-6 Receptor (SAM-6/R) or SAM-6/R glycoprotein, or a fragment thereof to an animal capable of expressing a human immunoglobulin, isolating spleen cells from an animal that produces antibody that binds to SAM-6 Receptor (SAM-6/R) or SAM-6/R glycoprotein, or the fragment thereof, fusing the spleen cells with a myeloma cell to produce a hybridoma, and screening the hybridoma for expression of a human antibody that binds to the polypeptide or the fragment thereof. In particular aspects, the fragment of SAM-6 Receptor (SAM-6/R) or SAM-6/R glycoprotein includes a portion of the polypeptide sequence with an N- or O-linked carbohydrate moiety.

### Description of Drawings

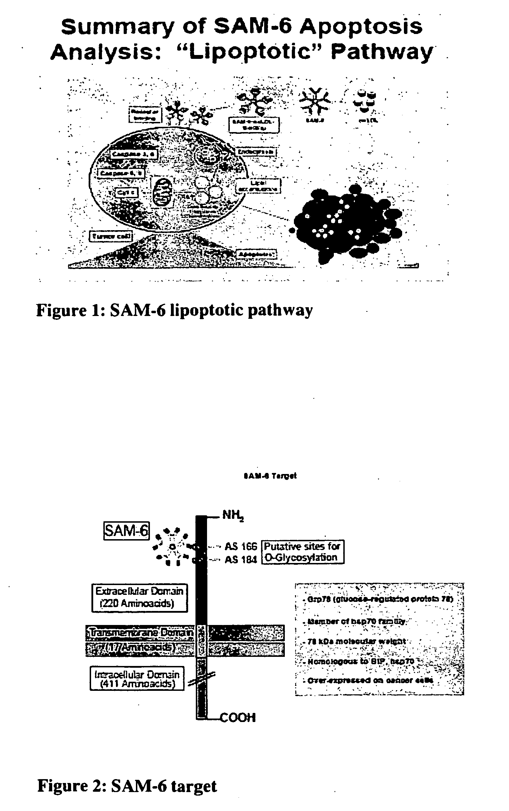

**Figure 1****:** SAM-6 lipoptotic pathway.
**Figure 2****:** SAM-6 target.
**Figure 3****:** Representative western blot of SAM-6 and unrelated IgM control on membrane extracts.
**Figure 4****:** Chromatogram after size exclusion chromatography.
**Figure 5****:** Western blot analysis of SAM-6 positive fractions after size exclusion chromatography.
**Figure 6****:** Coomassie staining of SDS-Page gel after size exclusion chromatography.
**Figure 7****:** Chromatogram after ion exchange chromatography.
**Figure 8****:** Western blot analysis of SAM-6 positive fractions after ion exchange chromatography.
**Figure 9****:** Coomassie Blue Staining after ion exchange chromatography.
**Figure 10****:** Peptide mass map of isolated 80kDa protein obtained by MALDI mass spectrometry analysis.
**Figure 11****:** Alignment of experimentally determined peptide sequences assigned to human Grp78 and the protein sequence of human Grp78/BiP [NP_005338].
**Figure 12****:** FACS analysis of SAM-6 binding on Grp78-siRNA-transfected BXPC-3 cells.
**Figure 13****:** Analysis of SAM-6 binding on Grp78-siRNA-transfected BXPC-3 cells. Percentages of cells positive for SAM-6surface antigens and controls (Grp78 and CD55) are shown.
**Figure 14****:** Cell Death ELISA with Grp78-siRNA-transfected BXPC-3 cells.
**Figure 15****:** Western Blot Analysis: Binding of SAM-6 antibody on membrane extracts of pancreas carcinoma cell line BXPC-3.
**Figure 16****:** SAM-6 binding on pancreas cancer cells after treatment with glycosidases.
**Figure 17****:** Immunoflourescence of SAM-6 endocytosis.
**Figure 18****:** Sudan III staining of antibody-induced pancreas cancer cells (BXPC-3).
**Figure 19****:** Analysis of SAM-6 induced apoptosis by measurement of Cytochrome C release.
**Figure 20****:** Analysis of SAM-6 induced apoptosis by measurement of activation of caspases -8, -9, -3 and -6.
**Figure 21****:** SAM-6 Dose dependent inhibition of stomach carcinoma tumour xenograft growth.

### Detailed Description

The invention is based, at least in part, on a glycoprotein, referred to as SAM-6 Receptor (SAM-6/R) or SAM-6/R glycoprotein. SAM-6/R glycoprotein has an apparent molecular weight in a range of about 80-82 kilodaltons (KDa) as determined by denaturing gel electrophoresis. A non-limiting exemplary feature of SAM-6/R glycoprotein is linkage of at least one nitrogen (N)- or oxygen (O)-linked carbohydrate moiety that is distinct from Grp78. Another non-limiting exemplary feature of SAM-6/R glycoprotein is that an antibody denoted SAM-6 specifically binds to SAM-6/R glycoprotein.

In accordance with the invention, there are provided isolated or purified glycoprotein denoted as SAM-6 Receptor (SAM-6/R) or SAM-6/R glycoprotein, having an apparent molecular weight in a range of about 80-82 kilodaltons (KDa) as determined by denaturing gel electrophoresis. In one embodiment, a SAM-6/R glycoprotein has at least one nitrogen (N)- or oxygen (O)-linked carbohydrate moiety distinct from Grp78. In another embodiment, an antibody denoted SAM-6 specifically binds to a SAM-6/R glycoprotein.

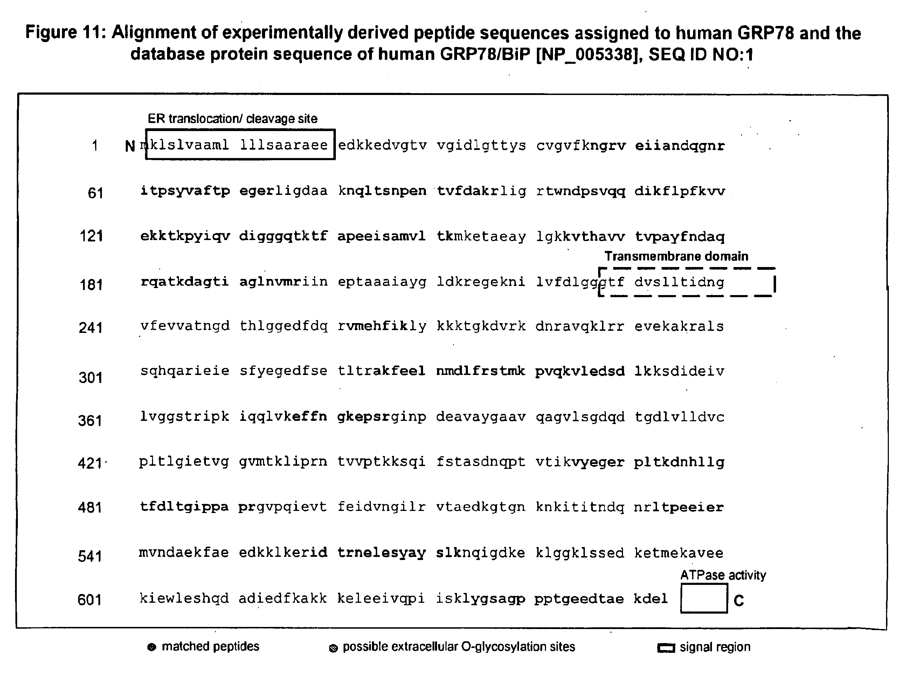

Sequence analysis of SAM-6/R glycoprotein revealed sequence identity with glucose-regulatory (or regulated) protein 78 (Grp78), also known and referred to as BiP, Hspa5, Heat shock 70 KDa protein 5 and Hsce70. Human Grp78/BiP sequence is as set forth in Figure 11 (SEQ ID NO:1). Sequences of SAM-6/R glycoprotein that appear identical to Grp78/BiP sequence are indicated in bold typeface. Thus, another non-limiting exemplary feature of SAM-6/R glycoprotein is at least partial sequence homology/identity with Grp78.

In accordance with the invention, there are provided isolated or purified glycoproteins denoted as SAM-6 Receptor (SAM-6/R) or SAM-6/R glycoprotein, having an apparent molecular weight in a range of about 80-82 kilodaltons (KDa) as determined by denaturing gel electrophoresis. In one embodiment, SAM-6/R glycoprotein has polypeptide sequence homology to Grp78 as set forth in SEQ ID NO:1.

In various non-limiting aspects, a SAM-6/R glycoprotein comprises a sequence of about 655 amino acids; has a transmembrane domain of about 17 amino acids; has an extracellular domain of about 220 amino acids; or has an intracellular domain of about 411 amino acids. In other non-limiting aspects, the SAM-6/R glycoprotein has a carbohydrate moiety linked to an amino acid, for example, an asparagine, serine or threonine residue of (*e.g.*, as in SEQ ID NO:1). Potential O-glycosylation sites of SAM-6/R glycoprotein are indicated by the underlined threonine (T) residues in SEQ ID NO: 1.

As used herein the term "glycoprotein" refers to a protein, polypeptide or peptide that has at least one sugar moiety covalently linked to an amino acid comprising the protein. A "carbohydrate moiety" refers to two or more sugar residues, e.g., mono-, di-, tri-saccharides, etc. The terms oligosaccharide and polysaccharide are synonymous with the term carbohydrate.

Sugars, carbohydrates, oligosaccharides and polysaccharides are typically linked to amino acid residues by a glycosidic bond. For eukaryotes, a series of sugar additions and removals occur post-translationally to form the carbohydrate moiety of the glycoprotein. Exemplary sugars include one or more of galactose, acetylgalactose, mannose, fucose, glucose, acetylglucose, sialic acid, N-acetylgalactosamine, N-acetylglucosamine and neuramic acids. A SAM-6/R glycoprotein optionally has have one or more of such particular sugars attached via an N- or O-linkage to a serine, threonine or asparagine residue, for example.

Contact of SAM-6/R glycoprotein with a glycosidase enzyme can reduce the apparent molecular weight of SAM-6/R glycoprotein due to removal of one or more sugar residues comprising the carbohydrate moiety. In one embodiment, contact of SAM-6/R glycoprotein with a glycosidase enzyme reduces the apparent molecular weight by about 1-10 kilodaltons (KDa), for example, from about 82 KDa to 72 KDa. In another embodiment, contact of SAM-6/R glycoprotein with an O-glycosidase reduces the apparent molecular weight of SAM-6/R glycoprotein. Of course, the skilled artisan will recognize that the amount and type, if any, of carbohydrate that can be removed from SAM-6/R glycoprotein will depend upon selection of a particular glycosidase enzyme, which will in turn affect the reduction, if any, in the apparent molecular weight of SAM-6/R glycoprotein.

Glycosidases capable of removing one or more sugars of a carbohydrate moiety, or the entire carbohydrate structure, include O-glycosidases, which typically cleave sugars that comprise carbohydrate moieties linked to the oxygen (O) of serine or threonine residues, and N-glycosidases, which typically cleave sugars that comprise carbohydrate moieties linked to the nitrogen (N) of asparagine residues. Particular examples of such glycosidases are O-glycosidase, N-glycosidase F, endoglycosidase H (endo H), neuraminidase and fucosidases. O-glycosidase cleaves serine- or threonine-linked oligosaccharide. N-glycosidase F cleaves asparagine bound N-glycans when the oligosaccharide has a minimum length of the chitobiose core unit. Endo H is a glycosidase that cleaves within the chitobiose core of high mannose and some hybrid oligosaccharides from N-linked glycoproteins. Neuraminidase removes terminal acylneuraminic residues. Fucosidases remove fucose, for example, from lactose and complex carbohydrates. Such glycosidases typically have at least some specificity in terms of the sugar linkages cleaved and whether the carbohydrate moieties are O- or N-linked and can therefore be used to characterize the composition and structure of the SAM-6/R glycoprotein carbohydrate moiety (ies).

A panel of carbohydrates was screened for binding of SAM-6 antibody. In particular, (GlcNAc)2, Man3, sTn, GM4, Lac-di-Nac, β-D-galactose-3-sulfate, H type 3, Neu5Ac6Gal, GlcNacβ3Gal, α-N-acetylneuraminic acid, 3'-SL, Atri, Tk, 3'SLN, sLe^{a}, Btri, GA1 Pk, Gb3, sLe^{x}, Adi, A type 2, Tαβ, 6'-SL, Bdi, B type 2, Galβ3Gal, Tββ, H type 2, Gala1-3'Lac, Le^{a} 3'-O-su-Le^{a}, 6'-O-su-LacNAc, GlcNAcβ1-2'TF, Le^{b}, 3'-O-su-Le^{x}, Hdi, Galα4GlcNAc, Le^{d} (H type 1), 3'-su-LacNAc, 3'-O-su-TF, β-N-acetylneuraminic acid, Le^{c}, 3'-su-Le^{c}, GlcNAcβ1-3'LacNAc, maltose, Le^{x}, melibiose, di-GalNAcβ, α-D-glucose, Le^{y}, Galα1-3'LacNAc, 3'-SiaTF, β-D-glucose, Lac, GlcNAcα1-3'TF, core 3, α-D-galactose, LacNAc, (Sia)₂, core 6, β-D-galactose, TF, (Sia)₃, core 4, α-D-mannose, Fucα3GlcNAc, GlcNAcβ1-3'TF, 3,6-SiaTF, α-D-mannose-6-phosphate, Fucα4GlcNAc Le, Gal2βGal, 6-SiaTF, α-L-fucose, Fs-2, 6-O-su-LacNAc, YDS, β-N-acetyl-D-glucosamine, core 5, core 2, 9-OS, α-N-acetyl-D-glalactosamine (Tn), Tαα, H type 4, 7-OS, β-N-acetyl-D-glalactosamine, 3'-SiaLe^{c}, LNT, 3,6-SiaTn, β-N-acetyl-D-glucosamine-6-sulfate, Gala2Gal, LNnT and 3-SiaTn were all screened for binding to SAM-6 antibody. SAM-6 antibody did not detectably bind to any of these carbohydrates. Consequently, a SAM-6 antibody of the invention includes antibodies that bind to SAM-6/R glycoprotein but not to one or more of the aforementioned carbohydrates.

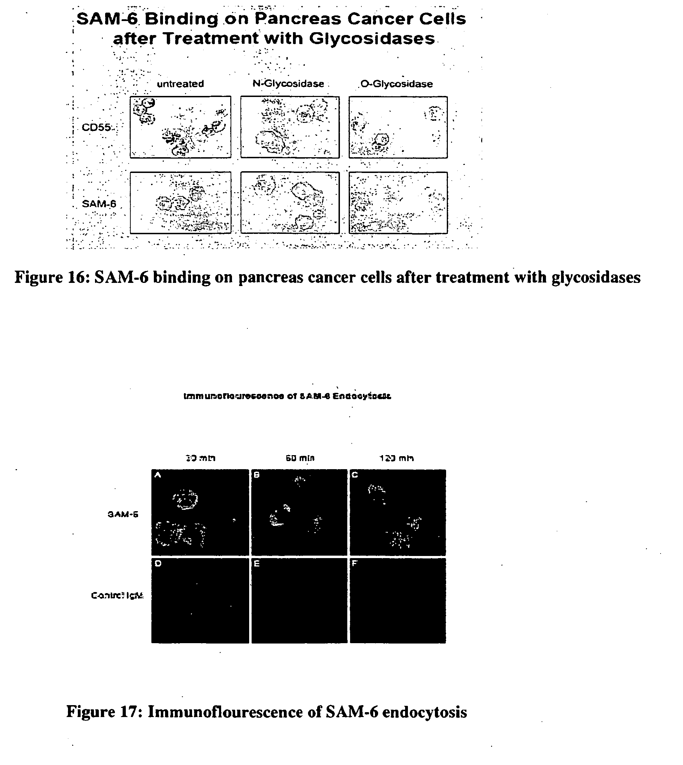

In embodiments in which antibody denoted SAM-6 specifically binds to SAM-6/R glycoprotein, the SAM-6 antibody binds to at least a portion of the SAM-6/R glycoprotein comprising a carbohydrate moiety (e.g., an N- or O-linked carbohydrate moiety). Treatment of SAM-6/R glycoprotein with an O-glycosidase enzyme reduced binding of SAM-6 antibody to the glycoprotein, presumably due to removal of one, several or all sugar(s) comprising a SAM-6 antibody binding epitope (Figure 16). Treatment of SAM-6/R glycoprotein with an N-glycosidase F enzyme did not destroy binding of SAM-6 antibody to the glycoprotein (Figure 16). Thus, binding of SAM-6 antibody to SAM-6/R glycoprotein appears to require or be mediated by an O-linked carbohydrate moiety, and not an N-linked carbohydrate moiety. Accordingly, SAM-6 binding to SAM-6/R may require or be mediated by, at least in part, one or more sugars comprising an O-linked carbohydrate moiety of a SAM-6/R glycoprotein, whereas SAM-6 binding to SAM-6/R does not appear to require an N-linked carbohydrate moiety of a SAM-6/R glycoprotein.

The term "SAM-6" or "SAM-6 antibody" refers to a human IgM antibody with a light chain variable region sequence (SEQ ID NO:13) and a heavy chain variable region sequence (SEQ ID NO:15). "SAM-6" or "SAM-6 antibody" specifically binds to SAM-6/R glycoprotein. SAM-6 appears to bind to a region of SAM-6/R glycoprotein that includes an O-linked carbohydrate moiety or binds to the O-linked carbohydrate moiety itself, as indicated by reduced SAM-6 binding to SAM-6/R glycoprotein observed after treatment of SAM-6/R glycoprotein with an O-glycosidase. SAM-6 does not appear to bind to a region of SAM-6/R glycoprotein that includes an N-linked carbohydrate moiety, as indicated by maintained SAM-6 binding to SAM-6/R glycoprotein after treatment of SAM-6/R glycoprotein with N-glycosidase F. SAM-6 has also been shown to bind to low density lipoprotein (LDL or oxLDL)..

Amino acid and nucleic acid sequences of SAM-6 light and heavy chain variable regions, with the complementary determining regions (CDR1-CDR3) indicated, are as follows.

Amino acid sequence of the variable region of the light chain (V_{L}) of antibody SAM-6 (SEQ ID NO:13): Ser Tyr Val Leu Thr Gln Pro Pro Ser Val 
Ser Val Ser Pro Gly Gln Thr Ala SerIle Thr Cys
Ser Gly Asp Lys Leu Gly Asp Lys Tyr Ala Cys
Trp Tyr Gln Gln Lys Pro Gly Gln Ser Pro Val
Leu Val Ile Tyr Gln Asp Ser Lys Arg Pro Ser
Gly Ile Pro Glu Arg Phe Ser Gly Ser Asn Ser
Gly Asn Thr Ala Thr Leu Thr Ile Ser Gly Thr
Gln Ala Met Asp Glu Ala Asp Tyr Tyr Cys Gln
Ala Trp Asp Ser Ser Ile Val Val
 Nucleotide sequence of the variable region of the light chain (V_{L}) of antibody SAM-6 (SEQ ID NO: 14): 
tcc tat gtg ctg act cag cca ccc tca gtg tcc gtg tcc cca gga 45
Ser Tyr Val Leu Thr Gln Pro Pro Ser Val Ser Val Ser Pro Gly
1               5                   10                  15

CDR1
cag aca gcc agc atc acc tgc tct gga gat aaa ttg ggg gat aaa 90
Gln Thr Ala Ser Ile Thr Cys Ser Gly Asp Lys Leu Gly Asp Lys
20 25 30

tat gct tgc tgg tat cag cag aag cca ggc cag tcc cct gtg ctg 135
Tyr Ala Cys Trp Tyr Gln Gln Lys Pro Gly Gln Ser Pro Val Leu
35 40 45

CDR2
gtc atc tat caa gat agc aag cgg ccc tca ggg atc cct gag cga 180
Val Ile Tyr Gln Asp Ser Lys Arg Pro Ser Gly Ile Pro Glu Arg
50 55 60

ttc tct ggc tcc aac tct ggg aac aca gcc act ctg acc atc agc 225
Phe Ser Gly Ser Asn Ser Gly Asn Thr Ala Thr Leu Thr Ile Ser
65 70 75

ggg acc cag gct atg gat gag gct gac tat tac tgt cag gcg tgg 270
Gly Thr Gln Ala Met Asp Glu Ala Asp Tyr Tyr Cys Gln Ala Trp
80 85 90
CDR3
gac agc agc att gtg gta 288
Asp Ser Ser Ile Val Val
95
 Amino Acid sequence of the variable region of the heavy chain (V_{H}) of antibody SAM-6 (SEQ ID NO: 15): 
Gln Val Gln Leu Val Glu Ser Gly Gly Gly
Val Val Gln Pro Gly Arg Ser Leu Arg Leu Ser
Cys Ala Ala Ser Gly Phe Thr Phe Ser Ser Tyr
Ala Met His Trp Val Arg Glu Ala Pro Gly Lys
Gly Leu Glu Trp Val Ala Val Ile Ser Tyr Asp
Gly Ser Asn Lys Tyr Tyr Ala Asp Ser Val Lys
Gly Arg Phe Thr Ile Ser Arg Asp Asn Ser Lys
Asn Thr Leu Tyr Leu Gln Met Asn Ser Leu Arg
Ala Glu Asp Thr Ala Val Tyr Tyr Cys Ala Arg
Asp Arg Leu Ala Val Ala Gly Lys Thr Phe Asp
Tyr
 Nucleotide sequence of the variable region of the heavy chain (V_{H}) of antibody SAM-6 (SEQ ID NO:16):

cag gtg cag ctg gtg gag tct ggg gga ggc gtg gtc cag cct ggg 45
Gln Val Gln Leu Val Glu Ser Gly Gly Gly Val Val Gln Pro Gly
1               5                   10                  15

agg tcc ctg aga ctc tcc tgt gca gcc tct gga ttc acc ttc agt 90
Arg Ser Leu Arg Leu Ser Cys Ala Ala Ser Gly Phe Thr Phe Ser
                20                  25                  30

CDR1
agc tat gct atg cac tgg gtc cgc cag gct cca ggc aag ggg ctg 135
Ser Tyr Ala Met His Trp Val Arg Glu Ala Pro Gly Lys Gly Leu
35 40 45

CDR2
gag tgg gtg gca gtt ata tca tat gat gga agc aat aaa tac tac 180
Glu Trp Val Ala Val Ile Ser Tyr Asp Gly Ser Asn Lys Tyr Tyr
50 55 60

gca gac tcc gtg aag ggc cga ttc acc atc tcc aga gac aat tcc 225
Ala Asp Ser Val Lys Gly Arg Phe Thr Ile Ser Arg Asp Asn Ser
65 70 75

aag aac acg ctg tat ctg caa atg aac agc ctg aga gct gag gac 270
Lys Asn Thr Leu Tyr Leu Gln Met Asn Ser Leu Arg Ala Glu Asp
80 85 90

CDR3
acg gct gtg tat tac tgt gcg aga gat cgg tta gca gtg gct ggt 315
Thr Ala Val Tyr Tyr Cys Ala Arg Asp Arg Leu Ala Val Ala Gly

95 100 105
CDR3
aaa act ttt gac tac
Lys Thr Phe Asp Tyr
110

In certain embodiments, SAM-6/R glycoprotein is characterized as being expressed on a hyperproliferative cell, such as a neoplastic, cancer or tumor cell. Non-limiting examples of neoplastic, cancer and tumor cells in which SAM-6/R glycoprotein has been detected include, for example, esophagus, stomach, breast, lung, colon, pancreas, prostate, uterus and ovary. In more particular aspects, SAM-6/R glycoprotein is characterized as being expressed on tumor cell lines denoted as BXPC-3 (ATCC Deposit No. CRL-1687; P.O. Box 1549 Manassas, VA, 20108, USA) and 23132/87 (DSMZ Deposit No. ACC 201; Deutsche Sammlung von Mikroorganismen und Zellkulturen GmbH (German Collection of Microorganisms and Cell Cultures), Inhoffenstrase 7 B 38124 Braunschweig, Germany).

In accordance with the invention, there are provided isolated or purified glycoprotein denoted as SAM-6 Receptor (SAM-6/R) or SAM-6/R glycoprotein, having an apparent molecular weight in a range of about 80-82 kilodaltons (KDa) as determined by denaturing gel electrophoresis 80%, 90%, 95% or more identity with SEQ ID NO:1, or any numerical value or range. In one embodiment, SAM-6/R glycoprotein has a polypeptide sequence identical to all or a part of Grp78 sequence as set forth in SEQ ID NO:1. In particular aspects, SAM-6/R glycoprotein has a polypeptide sequence identical to all or a part of one or more of the following amino acid sequences (SEQ ID NOs:2-12): NGRVEIIANDQGNRITPSYVAFTPEGER; NQLTSNPENTVFDAKR; TWNDPSVQQDIKFLPFKVVEKKTKPYIQVDIGGGQTKTFAPEEISAMVLTK; KVTHAVVTVPAYFNDAQRQATKDAGTIAGLNVMR; VMEHFIK; AKFEELNMDLFRSTMKPVQKVLEDSDLK; EFFNGKEPSR; VYEGERPLTKDNHLLGTFDLTGIPPAPR; LTPEEIER; IDTRNELESYAYSLK; or LYGSAGPPPTGEEDTAEKDEL. In another embodiment, SAM-6/R glycoprotein has a carbohydrate moiety linked thereto that is distinct from a Grp78 carbohydrate moiety. In particular aspects, the carbohydrate moiety is an N-linked or O-linked moiety.

The terms "identical" or "identity," mean that two or more referenced entities are the same. Thus, where two protein sequences are identical, they have the same amino acid sequence, at least within the referenced region or portion. Where two nucleic acid sequences are identical, they have the same polynucleotide sequence, at least within the referenced region or portion. An "area of identity" refers to a portion of two or more referenced entities that are the same. Thus, where two protein or nucleic acid sequences are identical over one or more sequence regions they share identity within that region.

The terms "homologous" or "homology" mean that two or more referenced entities share at least partial identity over a given region or portion. "Substantial homology" means that a molecule is structurally or functionally conserved such that it has or is predicted to have at least partial structure or function of one or more of the structures or functions (*e.g*., a biological function) of the reference molecule, or relevant/corresponding region or portion of the reference molecule to which it shares homology. Exemplary homology are polypeptides having amino acid sequences with 50%, 60%, 70%, 75%, 80%, 85%, 90%, 95%, 96%, 97%, 98%, or more sequence identity to a reference polypeptide. A polypeptide with substantial homology has or is predicted to have at least partial activity as the reference polypeptide. For example, in a particular embodiment, a SAM-6/R glycoprotein having one or more modifications (e.g., substitutions, deletions or additions of carbohydrate moiety or amino acid) that retains at least partial binding to SAM-6 is considered to have substantial homology to SAM-6/R glycoprotein.

The terms "protein," "polypeptide" and "peptide" are used interchangeably herein to refer to two or more amino acids, or "residues," covalently linked by an amide bond or equivalent. Polypeptides can be various lengths and the amino acids may be linked by non-natural and non-amide chemical bonds including, for example, those formed with glutaraldehyde, N-hydroxysuccinimide esters, bifunctional maleimides, or N, N'-dicyclohexylcarbodiimide (DCC). Non-amide bonds include, for example, ketomethylene, aminomethylene, olefin, ether, thioether and the like (see, *e.g*., Spatola in Chemistry and Biochemistry of Amino Acids, Peptides and Proteins, Vol. 7, pp 267-357 (1983), "Peptide and Backbone Modifications," Marcel Decker, NY).

The term "isolated" used as a modifier of a composition means that the composition is made by the hand of man or is separated from one or more other components in their naturally occurring *in vivo* environment. Generally, compositions so separated are substantially free of one or more materials with which they normally associate with in nature, for example, one or more protein, nucleic acid, lipid, carbohydrate, cell membrane. Thus, an isolated composition is substantially separated from other biological components in the cell of the organism in which the composition naturally occurs, or from the artificial medium in which it is produced (*e.g*., synthetically or through cell culture). For example, an isolated polypeptide is substantially separated from other polypeptides and nucleic acid and does not include a library of polypeptides or polynucleotides present among millions of polypeptide or nucleic acid sequences, such as a polypeptide, genomic or cDNA library, for example. An isolated nucleic acid is substantially separated from other polypeptides and nucleic acid and does not include a library of polypeptides or polynucleotides present among millions of polypeptide or nucleic acid sequences, such as a polypeptide, genomic or cDNA library, for example. The term "isolated" does not exclude alternative physical forms of the composition, for example, an isolated protein could include protein multimers, post-translational modifications (*e.g*., glycosylation, phosphorylation) or derivatized forms.

The term "purified" used as a modifier of a composition refers to a composition free of most or all of the materials with which it typically associates with in nature. Thus, a protein separated from cells is considered to be substantially purified when separated from cellular components by standard methods while a chemically synthesized nucleic acid sequence is considered to be substantially purified when separated from its chemical precursors. Purified therefore does not require absolute purity. Furthermore, a "purified" composition can be combined with one or more other molecules. Thus, the term "purified" does not exclude combinations of compositions.

"Purified" proteins and nucleic acid include proteins and nucleic acids produced by standard purification methods. The term also includes proteins and nucleic acids produced by recombinant expression in a host cell as well as chemical synthesis. "Purified" can also refer to a composition in which the level of contaminants is below a level that is acceptable to a regulatory agency for administration to a human or non-human animal, for example, the Food and Drug administration (FDA).

Substantial purity can be at least about 60% or more of the molecule by mass. Purity can also be about 70% or 80% or more, and can be greater, for example, 90% or more. Purity can be less, for example, in a pharmaceutical carrier the amount of a molecule by weight % can be less than 60% but the relative proportion of the molecule compared to other components with which it is normally associated with will be greater. Purity can be determined by any appropriate method, including, for example, UV spectroscopy, chromatography (*e.g*., HPLC, gas phase), gel electrophoresis (*e.g*., silver or coomassie staining) and sequence analysis (peptide and nucleic acid).

SAM-6/R glycoprotein is expressed on malignant and non-malignant, neoplastic, tumor and cancer cells. For example, SAM-6/R glycoprotein is expressed on malignant and non-malignant gastric tissue, lung squamous cell carcinoma, lung adenocarcinoma, melanomas and nasal cancer cells.

SAM-6/R glycoprotein is also expressed on tumors at various stages and grades. For example, SAM-6/R glycoprotein was detected on all of stages IA, IB, IIA, IIB, IIIA, IIIB and IV, and grades G1, G2 and G3, of lung squamous cell carcinoma and adenocarcinoma.

SAM-6/R glycoprotein is additionally expressed on tumor metastasis. For example, SAM-6/R glycoprotein was detected on lung squamous cell carcinoma and adenocarcinoma metastasis to lymph node and brain; SAM-6/R glycoprotein was detected on breast cancer (invasive ductal) metastasis to lymph node; SAM-6/R glycoprotein was detected on colon adenocarcinoma metastasis to liver and lymph node; SAM-6/R glycoprotein was detected on stomach adenocarcinoma (intestinal and diffuse) metastasis to lymph node; SAM-6/R glycoprotein was detected on pancreas adenocarcinmoa metastasis to lymph node; SAM-6/R glycoprotein was detected on head and neck squamous cell carcinoma metastasis to lymph node; and SAM-6/R glycoprotein was detected on melanoma metastasis to rectum, esophagus, skin, parotid gland, colon, adrenal gland and nasal epithelium.

SAM-6/R glycoprotein is further expressed on tumor cell lines. For example, SAM-6/R glycoprotein is expressed on tumor cell line denoted as BXPC-3 (ATCC Deposit No. CRL-1687; P.O. Box 1549 Manassas, VA, 20108, USA) 23132/87 (DSMZ Deposit No. ACC 201; Deutsche Sammlung von Mikroorganismen und Zellkulturen GmbH (German Collection of Microorganisms and Cell Cultures), Inhoffenstrase 7 B 38124 Braunschweig, Germany) melanoma cell lines CRL1424 and HTB-69, and nasal cancer cells (RPMI2650). Isolated or purified SAM-6/R glycoprotein can be obtained from these tumors, metastasis, cell lines and other cells (primary isolates or passaged or immortalized cell lines) using the purification methods disclosed herein or known in the art.

In accordance with the invention, there are provided antibodies that specifically bind to SAM-6/R glycoprotein and immunogenic subsequences of SAM-6/R glycoprotein, as well as cells that express SAM-6/R glycoprotein. Such SAM-6/R glycoprotein antibodies include antibodies that specifically bind to SAM-6/R glycoprotein, antibodies that specifically bind to an epitope that includes a carbohydrate moiety linked to SAM-6/R glycoprotein and antibodies that specifically bind to a carbohydrate moiety linked to SAM-6/R glycoprotein.

SAM-6/R glycoprotein is typically not expressed on non-cancer cells. For example, SAM-6/R glycoprotein was not detected on fresh frozen (FDA standard) normal human tissue from adrenal gland, cerebellum, cerebrum, pituitary, breast, colon, esophagus, heart, kidney, liver, lung, skeletal muscle, mesothileal, peripheral nerve, sciatic nerve, trigeminal nervew, ovary, pancreas, placenta, prostate, salivary gland, skin, small intestine, spleen, stomach, testis, thymus, thyroid, tonsil, uterus, cervix, or bone marrow. SAM-6/R glycoprotein was also not detected on lymphocytes, granulocytes, human fibroblasts and normal nasal cells (HNEPC). Thus, antibodies that specifically bind to SAM-6/R glycoprotein are not expected to bind one or more of the foregoing normal human tissue types, cells derived or that are considered representative of such normal human tissue types, nor lymphocytes, granulocytes, human fibroblasts or normal nasal cells (HNEPC).

Antibodies that specifically bind to SAM-6/R glycoprotein include isolated antibodies, purified antibodies and antibody subsequences. In one embodiment, an antibody that binds to the SAM-6/R glycoprotein extracellular domain is provided. In another embodiment, an antibody that specifically binds to an epitope comprising at least one N- or O-linked carbohydrate moiety of SAM-6/R glycoprotein is provided. In a further embodiment, an antibody that binds to a carbohydrate moiety linked to SAM-6/R glycoprotein that is distinct from Grp78 is provided. In an additional embodiment, an antibody is provided that specifically binds to SAM-6/R glycoprotein, but exhibits reduced binding after SAM-6/R glycoprotein is contacted with a glycosidase. In one aspect, O-glycosidase treatment of SAM-6/R glycoprotein reduces or destroys binding of the antibody to SAM-6/R. In another aspect, N-glycosidase F treatment of SAM-6/R glycoprotein does not reduce or destroy binding of the antibody to SAM-6/R. In yet another embodiment, an antibody binds to LDL or oxLDL. In a still further embodiment, an antibody is provided that does not bind to an epitope comprising an N- or O-linked carbohydrate moiety of SAM-6/R glycoprotein.

Invention antibodies that specifically bind to SAM-6/R glycoprotein are distinct from SAM-6 antibody. For example, an invention antibody does not have heavy and light chain variable region sequences identical to heavy and light chain variable region sequences of SAM-6 antibody set forth herein. Invention antibodies also include antibodies that do not have either a heavy or a light chain variable sequence identical to heavy or light chain variable sequences of SAM-6 antibody. Invention antibodies further include antibodies that do not have 1-3 or 3-5, 5-7, or 7-10 amino acid substitutions of the heavy or a light chain variable sequences of SAM-6. Invention antibodies additionally include antibodies that do not have 90%-95%, 96%, 97%, 98%, 99%, or more identity (e.g., 100%) to the heavy or light chain variable region sequence of SAM-6 antibody (or one or more CDRs within such heavy or light chain variable region sequences), or any numerical value or range within or encompassing such percent values.

In a particular embodiment, an antibody that specifically binds to SAM-6/R glycoprotein that is distinct from SAM-6 antibody as defined herein has a heavy chain sequence with 100% identity to a heavy chain variable region sequence or the third CDR (CDR3; ARDRLAVAGRPFDY; SEQ ID NO:17) within a heavy chain variable region amino acid sequence (SEQ ID NO: 18) set forth as:

<-------------------------------------------- FR1 - IMGT
G G G V V Q P
... ... ... ... ... ... ... ggg gga ... ggc gtg gtc cag cct

-----------------------------------------------> ___________
G R S L R L S C A A S G F T F
ggg agg tcc ctg aga ctc tcc tgt gca gcc tct gga ttc acc ttc

__ CDR1 - IMGT ______________ <-------------------------
S S Y A M H W V R Q A
agt agc tat gct ... ... ... ... atg cac tgg gtc cgc cag gct

FR2 - IMGT ---------------------------> ______________ CDR2
P G K G L E W V A V I S Y D G
cca ggc aag ggg ctg gag tgg gtg gca gtt ata tca tat gat gga

- IMGT __________ <-------------------------------------
S N K Y Y A D S V K G R
agc aat aaa ... ... tac tac gca gac tcc gtg aag ... ggc cga

------------------------------- FR3 - IMGT ------------
F T I S R D N S K N T L Y L Q
ttc acc atc tcc aga gac aat tcc aag aac acg ctg tat ctg caa

-----------------------------------------------------> ___
M N S L R A E D T A V Y Y C A
atg aac agc ctg aga gct gag gac acg gct gtg tat tac tgt gcg

_________________ CDR3 - IMGT __________________
R D R L A V A G R P F D Y W G
aga gat cgg tta gca gtg gct ggt aga cct ttt gac tac tgg ggc

Q G T L V T V S S G
cag gga acc ctg gtc acc gtc tcc tca ggg

Antibodies of the invention include antibodies having one or more activities or functions of SAM-6 antibody. In one embodiment, an antibody specifically binds to a cell expressing a SAM-6/R glycoprotein. In another embodiment, an antibody exhibits greater binding to a neoplastic, tumor or cancer, or metastasis cell, than a corresponding non-neoplastic, non-tumor or non-cancer or non-metastasis cell. In a further embodiment, binding of a SAM-6/R glycoprotein antibody to a cell expressing SAM-6/R glycoprotein inhibits, decreases or reduces cell growth or proliferation, or stimulates or induces death, lysis or apoptosis of the cell.

Non-limiting cells that express SAM-6/R glycoprotein to which the antibodies bind include malignant and non-malignant gastric tissue cell, lung squamous cell carcinoma, lung adenocarcinoma cell, of any stage (e.g., stages IA, IB, IIA, IIB, IIIA, IIIB or IV) or grade (e.g., grades G1, G2 or G3), melanomas and nasal cancer cells. Additional non-limiting cells that express SAM-6/R glycoprotein to which the antibodies bind include tumor metastasis, such as lung squamous cell carcinoma and adenocarcinmoa metastasis to lymph node and brain; breast cancer (invasive ductal) metastasis to lymph node; colon adenocarcinoma metastasis to liver and lymph node; SAM-6/R glycoprotein was detected on stomach adenocarcinoma (intestinal and diffuse) metastasis to lymph node; pancreas adenocarcinmoa metastasis to lymph node; head and neck squamous cell carcinoma metastasis to lymph node; and melanoma metastasis to rectum, esophagus, skin, parotid gland, colon, adrenal gland and nasal epithelium.

Further non-limiting cells that express SAM-6/R glycoprotein to which the antibodies bind include tumor cell lines, such as BXPC-3 (ATCC Deposit No. CRL-1687; P.O. Box 1549 Manassas, VA, 20108, USA), 23132/87 (DSMZ Deposit No. ACC 201; Deutsche Sammlung von Mikroorganismen und Zellkulturen GmbH (German Collection of Microorganisms and Cell Cultures), Inhoffenstrase 7 B 38124 Braunschweig, Germany), melanoma cells CRL1424 and HTB-69, and nasal cancer cells RPMI2650.

Thus, in additional embodiments, binding of SAM-6/R glycoprotein antibody to a neoplastic, tumor or cancer, or metastasis cell expressing SAM-6/R glycoprotein inhibits, decreases or reduces cell growth or proliferation, or stimulates or induces cell death, lysis or apoptosis. In still another embodiment, binding of SAM-6/R glycoprotein antibody to BXPC-3, 23132/87, CRL1424, HTB-69, or RPMI2650 cells inhibits, decreases or reduces BXPC-3, 23132/87, CRL1424, HTB-69, or RPMI265 cell growth or proliferation, or stimulates or induces BXPC-3, 23132/87, CRL1424, HTB-69, or RPMI265 cell death, lysis or apoptosis. In particular aspects, cell growth or proliferation is inhibited, decreased or reduced at least 20%, 30%, 40%, 50%, 60%, 75%, or more relative to a control (untreated) cell, or any numerical value or range within or encompassing such percent values. In further particular aspects, cell death, lysis or apoptosis is at least 20%, 30%, 40%, 50%, 60%, 75%, or more relative to a control (untreated) cell, or any numerical value or range within or encompassing such percent values. In yet a further embodiment, binding of the antibody to cells expressing the glycoprotein causes activation of a caspase (e.g., caspase-3, caspase-8 or caspase-9).

Antibodies of the invention include polyclonal and monoclonal antibodies. The term "monoclonal," when used in reference to an antibody refers to an antibody that is based upon, obtained from or derived from a single clone, including any eukaryotic, prokaryotic, or phage clone. A "monoclonal" antibody is therefore defined herein structurally, and not the method by which it is produced.

Antibodies of the invention can belong to any antibody class, IgM, IgG, IgE, IgA, IgD, or subclass. Exemplary subclasses for IgG are IgG₁, IgG₂, IgG₃ and IgG₄.

Antibodies of the invention can have kappa or lambda light chain sequences, either full length as in naturally occurring antibodies, mixtures thereof (i.e., fusions of kappa and lambda chain sequences), and subsequences/fragments thereof. Naturally occurring antibody molecules contain two kappa or two lambda light chains. The primary difference between kappa and lambda light chains is in the sequences of the constant region.

In accordance with the invention, there are provided antibodies that specifically bind to SAM-6/R glycoprotein, and that partially or fully compete for binding of SAM-6 to SAM-6/R glycoprotein. In one embodiment, an antibody that inhibits binding of SAM-6 to SAM-6/R glycoprotein is provided. In various aspects, the antibody competitively inhibits binding by at least 30%, 40%, 50%, 60%, 70%, 80%, 90% or more of the binding of SAM-6 to the SAM-6/R glycoprotein, or any numerical value or range within or encompassing such percent values. In another embodiment, an antibody that prevents or blocks binding of SAM-6 to SAM-6/R glycoprotein is provided.

Invention antibodies that compete for binding of the SAM-6 antibody to SAM-6/R glycoprotein can have the binding specificity of SAM-6 antibody. Thus, a SAM-6/R glycoprotein antibody that competes with SAM-6 antibody for binding to SAM-6/R glycoprotein can specifically bind to an epitope that includes a carbohydrate moiety linked to SAM-6/R glycoprotein, or to a carbohydrate moiety linked to SAM-6/R glycoprotein to which SAM-6 antibody binds. A SAM-6/R glycoprotein antibody may also be able to compete with SAM-6 antibody for binding to LDL or oxLDL. Accordingly, antibodies are provided that specifically bind to SAM-6/R glycoprotein, and that partially or fully compete for binding of SAM-6 antibody to SAM-6/R glycoprotein or LDL or oxLDL, including antibodies that bind to the same epitope, a part of the same epitope or a carbohydrate moiety as SAM-6 antibody.

SAM-6/R glycoprotein antibodies that compete for binding of the SAM-6 antibody to SAM-6/R glycoprotein of the antibody can be screened and identified using a conventional competition binding assays. Screened antibodies are selected based upon an ability to compete for binding of the SAM-6 antibody to SAM-6/R glycoprotein or LDL or oxLDL. The ability of an antibody to compete for binding of SAM-6 to SAM-6/R glycoprotein, or to inhibit, prevent or block binding of SAM-6 to SAM-6/R glycoprotein or LDL or oxLDL, can be determined by various assays know in the art, including enzyme linked immunosorbent assay (ELISA).

Invention antibodies include antibodies having binding affinity of SAM-6 antibody. The binding affinity of such antibodies may differ from SAM-6 antibody (i.e., have greater or less affinity for SAM-6/R glycoprotein, LDL or oxLDL) and therefore, the antibodies can vary in their ability to compete for binding to SAM-6 glycoprotein, LDL or oxLDL. Invention antibodies thus include antibodies that have greater or less affinity for SAM-6/R glycoprotein, LDL or oxLDL than SAM-6 antibody. For example, SAM-6/R glycoprotein antibody of the invention may have an affinity greater or less than 2-5, 5-10, 10-100, 100-1000 or 1000-10,000-fold affinity, or any numerical value or range within or encompassing such values, as the reference antibody (e.g., SAM-6 antibody). Specific non-limiting SAM-6/R glycoprotein antibodies have a binding affinity for SAM-6/R glycoprotein, LDL or oxLDL within about 1-5,000 fold of the binding affinity of SAM-6 for SAM-6/R glycoprotein, LDL or oxLDL. Additional specific non-limiting antibodies have a binding affinity for SAM-6/R glycoprotein, LDL or oxLDL is within about K_{d} 10⁻² M to about K_{d} 10⁻¹⁵ M, or within about K_{d} 10⁻⁶ M to about K_{d} 10⁻¹² M, of SAM-6 for SAM-6/R glycoprotein, LDL or oxLDL, or any numerical value or range within or encompassing such values. In more particular embodiments, a binding affinity for SAM-6/R glycoprotein, LDL or oxLDL with a dissociation constant (KD) less than 5x10⁻² M, 10⁻² M, 5x10⁻³ M, 10⁻³ M 5x10⁻⁴ M, 10⁻⁴ M 5x10⁻⁵ M, 10⁻⁵ M 5x10⁻⁶ M, 10⁻⁶ M 5x10⁻⁷ M, 10⁻⁷ M 5x10⁻⁸ M, 10⁻⁸ M 5x10⁻⁹ M, 10⁻⁹ M 5x10⁻¹⁰ M, 10⁻¹⁰ M 5x10⁻¹⁰ M, 10⁻¹¹ M 5x10⁻¹² M, 10⁻¹² M 5x10⁻¹³ M, 10⁻¹³ M 5x10⁻¹⁴ M, 10⁻¹⁴ M 5x10⁻¹⁵ M, and 10⁻¹⁵ M, or any numerical value or range within or encompassing such values.

Binding affinity can be determined by association (Kₐ) and dissociation (K_{d}) rate. Equilibrium affinity constant, KD, is the ratio of Kₐ/K_{d}. Association (Kₐ) and dissociation (K_{d}) rates can be measured using surface plasmon resonance (SPR) (Rich and Myszka, Curr. Opin. Biotechnol. 11:54 (2000); Englebienne, Analyst. 123:1599 (1998)). Instrumentation and methods for real time detection and monitoring of binding rates are known and are commercially available (BiaCore 2000, Biacore AB, Upsala, Sweden; and Malmqvist, Biochem. Soc. Trans. 27:335 (1999)). KD values can be defined as the antibody concentration required to saturate one half (50%) of the binding sites on SAM-6/R glycoprotein.

Methods of producing polyclonal and monoclonal antibodies are known in the art. For example, SAM-6/R glycoprotein or an immunogenic fragment thereof, optionally conjugated to a carrier such as keyhole limpet hemocyanin (KLH) or ovalbumin (*e.g*., BSA), or mixed with an adjuvant such as Freund's complete or incomplete adjuvant, and used to immunize an animal. Using conventional hybridoma technology, splenocytes from immunized animals that respond to SAM-6/R glycoprotein can be isolated and fused with myeloma cells. Monoclonal antibodies produced by the hybridomas can be screened for reactivity with SAM-6/R glycoprotein or an immunogenic fragment thereof.

Animals that may be immunized include mice, rats, rabbits, goats, sheep, cows or steer, guinea pigs or primates. Initial and any optional subsequent immunization may be through intravenous, intraperitoneal, intramuscular, or subcutaneous routes. Subsequent immunizations may be at the same or at different concentrations of SAM-6/R glycoprotein preparation, and may be at regular or irregular intervals.

Animals include those genetically modified to include human IgG gene loci, which can therefore be used to produce human antibodies. Transgenic animals with one or more human immunoglobulin genes that do not express endogenous immunoglobulins are described, for example in, U.S. Patent No. 5,939,598. Additional methods for producing human polyclonal antibodies and human monoclonal antibodies are described (see, *e.g*., Kuroiwa et al., Nat. Biotechnol. 20:889 (2002); WO 98/24893; WO 92/01047; WO 96/34096; WO 96/33735; U.S. Patent Nos. 5,413,923; 5,625,126; 5,633,425; 5,569,825; 5,661,016; 5,545,806; 5,814,318; 5,885,793; 5,916,771; and 5,939,598). An overview of the technology for producing human antibodies is described in Lonberg and Huszar (Int. Rev. Immunol. 13:65 (1995)).

SAM-6/R glycoprotein antibodies can also be generated using other techniques including hybridoma, recombinant, and phage display technologies, or a combination thereof (see U.S. Patent Nos. 4,902,614, 4,543,439, and 4,411,993; see, also Monoclonal Antibodies, Hybridomas: A New Dimension in Biological Analyses, Plenum Press, Kennett, McKearn, and Bechtol (eds.), 1980, and Harlow et al., Antibodies: A Laboratory Manual, Cold Spring Harbor Laboratory Press, 2nd ed. 1988).

SAM-6/R glycoprotein antibodies can be humanized using a variety of techniques known in the art including, for example, CDR-grafting (EP 239,400; W091/09967; U.S. Patent Nos. 5,225,539; 5,530,101; and 5,585,089), veneering or resurfacing (EP 592,106; EP 519,596; Padlan, Molecular Immunol. 28:489 (1991); Studnicka et al., Protein Engineering 7:805 (1994); Roguska. et al., Proc. Nat'l. Acad. Sci. USA 91:969 (1994)), and chain shuffling (U.S. Patent No. 5,565,332). Human consensus sequences (Padlan, Mol. Immunol. 31:169 (1994); and Padlan, Mol. Immunol. 28:489 (1991)) have previously used to produce humanized antibodies (Carter et al., Proc. Natl. Acad. Sci. USA 89:4285 (1992); and Presta et al., J. Immunol. 151:2623 (1993)).

Methods for producing chimeric antibodies are known in the art (*e.g.,* Morrison, Science 229:1202 (1985); Oi et al., BioTechniques 4:214 (1986); Gillies et al., J. Immunol. Methods 125:191 (1989); and U.S. Patent Nos. 5,807,715; 4,816,567; and 4,816,397). Chimeric antibodies in which a variable domain from an antibody of one species is substituted for the variable domain of another species are described, for example, in Munro, Nature 312:597 (1984); Neuberger et al., Nature 312:604 (1984); Sharon et al., Nature 309:364 (1984); Morrison et al., Proc. Nat'l. Acad. Sci. USA 81:6851 (1984); Boulianne et al., Nature 312:643 (1984); Capon et al., Nature 337:525 (1989); and Traunecker et al., Nature 339:68 (1989).

Suitable techniques that additionally may be employed in antibody methods include SAM-6/R glycoprotein-based affinity purification, non-denaturing gel purification, HPLC or RP-HPLC, size exclusion, purification on protein A column, or any combination of these techniques. The antibody isotype can be determined using an ELISA assay, for example, a human Ig can be identified using mouse Ig-absorbed anti-human Ig.

SAM-6/R glycoprotein suitable for generating antibodies can be produced by any of a variety of standard protein purification or recombinant expression techniques known in the art. For example, SAM-6/R glycoprotein can be obtained from cells, such as BXPC-3 cells (ATCC Deposit No. CRL-1687; P.O. Box 1549 Manassas, VA, 20108, USA) or 23132/87 cells (DSMZ Deposit No. ACC 201; Deutsche Sammlung von Mikroorganismen und Zellkulturen GmbH (German Collection of Microorganisms and Cell Cultures), Inhoffenstrase 7 B 38124 Braunschweig, Germany).

Forms of SAM-6/R glycoprotein suitable for generating an immune response include peptide subsequences of full length SAM-6/R glycoprotein, such as an immunogenic fragment with at least one N- or O-linked carbohydrate moiety, or an immunogenic fragment that binds to SAM-6 antibody. Additional forms of SAM-6/R glycoprotein include SAM-6/R glycoprotein containing preparations or cell extracts or fractions, partially purified SAM-6/R glycoprotein as well as whole cells that express SAM-6/R glycoprotein or preparations of such SAM-6/R glycoprotein expressing cells.

Further described are methods of producing antibodies that specifically bind to SAM-6/R glycoprotein. In one embodiment, a method includes administering a polypeptide having a molecular weight in a range of about 80-82 kilodaltons (kDa) as determined by denaturing gel electrophoresis, having at least one O-linked carbohydrate moiety, and has at least partial sequence homology with Grp78, or a fragment thereof, to an animal, screening the animal for expression of an antibody that binds to the polypeptide, selecting an animal that produces an antibody that binds to the polypeptide, and isolating the antibody from the selected animal. In another embodiment, a method includes administering a polypeptide having an apparent molecular weight in a range of about 80-82 kilodaltons (kDa) as determined by denaturing gel electrophoresis, having at least one N- or O-linked carbohydrate moiety, and having at least partial sequence homology with Grp78, or a fragment thereof, to an animal, screening the animal for expression of an antibody that binds to the polypeptide, selecting an animal that produces an antibody that binds to the polypeptide, and isolating the antibody from the selected animal. In a further embodiment, a method includes administering a polypeptide having an apparent molecular weight in a range of about 80-82 kilodaltons (kDa) as determined by denaturing gel electrophoresis, having at least one N- or O-linked carbohydrate moiety, and having at least partial sequence homology with Grp78, or a fragment thereof to an animal capable of expressing a human immunoglobulin; isolating spleen cells from an animal that produces antibody that binds to the polypeptide or the fragment thereof, fusing the spleen cells with a myeloma cell to produce a hybridoma, and screening the hybridoma for expression of an antibody that binds to the polypeptide having an apparent molecular weight in a range of about 80-82 kilodaltons (kDa) as determined by denaturing gel electrophoresis, having at least one N- or O-linked carbohydrate moiety, and having at least partial sequence homology with Grp78, or the fragment thereof. In various aspects, the polypeptide fragment includes a portion of the polypeptide with an N- or O-linked carbohydrate moiety.

Described herein is producing antibodies distinct from SAM-6 antibody that have one or more functions or activities of SAM-6 antibody. Exemplary functions or activities include, for example, binding to SAM-6/R glycoprotein (e.g., extracellular domain); binding to an epitope comprising a SAM-6/R glycoprotein carbohydrate moiety or an immunogenic fragment of SAM-6/R glycoprotein; competing for binding of SAM-6 antibody to SAM-6/R glycoprotein; binding to LDL or oxLDL; competing for binding of SAM-6 antibody to LDL or oxLDL; having binding affinity of SAM-6 antibody (*e.g*., greater or less affinity for SAM-6/R glycoprotein), binding to a cell expressing SAM-6/R glycoprotein; binding to a cell expressing SAM-6/R glycoprotein and decreasing or reducing cell growth or proliferation, or stimulating or inducing death, lysis or apoptosis of the cell (*e.g*., a neoplastic, tumor or cancer, or metastasis cell); binding to BXPC-3 or 23132/87 cells and inhibiting BXPC-3 or 23132/87 cell growth or proliferation, or stimulating or inducing BXPC-3 or 23132/87 cell death, lysis or apoptosis; causing activation of a caspase (*e.g*., caspase-3, caspase-8 or caspase-9). Exemplary functions or activities also include SAM-6/R glycoprotein binding sensitivity or insensitivity to glycosidases, for example, O-glycosidase enzyme treatment of SAM-6/R glycoprotein reducing or destroying binding of the antibody to SAM-6/R glycoprotein, and N-glycosidase F enzyme treatment of SAM-6/R glycoprotein not reducing or destroying binding of the antibody to SAM-6/R glycoprotein.

Described herein are modified forms of proteins, antibodies, nucleic acids, and other compositions, provided that the modified form retains, at least a part of, a function or activity of the unmodified or reference protein, nucleic acid, or antibody. For example, a modified SAM-6/R glycoprotein (*e.g.,* a subsequence or fragment) can be used as an immunogen to produce antibodies that specifically bind to SAM-6/R glycoprotein. A modified SAM-6/R glycoprotein antibody (*e.g*., a subsequence or fragment) can be used in the invention treatment, diagnostic, screening and detection methods.

As used herein, the term "modify" and grammatical variations thereof, means that the composition deviates from a reference composition. Such modified proteins, nucleic acids and other compositions may have greater or less activity than or a distinct function from a reference unmodified protein, nucleic acid, or composition.

Modifications include SAM-6/R glycoprotein amino acid and carbohydrate moiety substitutions, additions and deletions, which can be referred to as "variants." Specific non-limiting examples of amino acid modifications include SAM-6/R glycoprotein subsequences and fragments. Exemplary SAM-6/R glycoprotein subsequences and fragments include a portion of the SAM-6/R glycoprotein comprising an N- or O-linked carbohydrate moiety, the carbohydrate moiety optionally distinct from a carbohydrate moiety of Grp78. Exemplary SAM-6/R glycoprotein subsequences and fragments also include an immunogenic portion of SAM-6/R glycoprotein, for example, a portion of SAM-6/R glycoprotein that includes one or more N- or O-linked carbohydrate moiety(ies). Exemplary SAM-6/R glycoprotein subsequences and fragments further include a portion of SAM-6/R glycoprotein that binds to SAM-6 antibody. Specific non-limiting examples of carbohydrate moiety modifications include SAM-6/R glycoprotein having one or more sugar residues deleted (an O-linked moiety or a sugar thereof) that reduces or destroys binding to SAM-6 antibody or one or more sugar residues deleted (N-linked moiety or a sugar thereof) that does not reduce or destroy binding to SAM-6 antibody.

As used herein, the term "subsequence" or "fragment" means a portion of the full length molecule. A subsequence of a SAM-6/R glycoprotein has one or more less amino acids than a full length SAM-6/R glycoprotein (e.g. one or more internal or terminal amino acid deletions from either amino or carboxy-termini). A subsequence of a SAM-6/R glycoprotein antibody has one or more less amino acids than a full length SAM-6/R glycoprotein antibody. A nucleic acid subsequence has at least one less nucleotide than a full length comparison nucleic acid sequence. Subsequences therefore can be any length up to the full length native molecule.

Exemplary SAM-6/R glycoprotein antibody subsequences and fragments of the invention include Fab, Fab' and F(ab')₂, Fd, single-chain Fvs (scFv), single-chain antibodies, disulfide-linked Fvs (sdFv) and V_{L} and V_{H} domain fragments. Such subsequences and fragments can have the binding affinity as the full length antibody, the binding specificity as the full length antibody, or one or more activities or functions of as a full length antibody, *e.g*., a function or activity of SAM-6 antibody. The terms "functional subsequence" and "functional fragment" when referring to an antibody refers to a portion of an antibody that retains at least a part of one or more functions or activities as an intact reference antibody, *e.g*., a function or activity of SAM-6 antibody. For example, an antibody subsequence that binds to SAM-6 glycoprotein, an immunogenic fragment of SAM-6 glycoprotein, LDL or oxLDL is considered a functional subsequence.

Antibody subsequences and fragments can be combined. For example, a V_{L} or V_{H} subsequences can be joined by a linker sequence thereby forming a V_{L}-V_{H} chimera. A combination of single-chain Fvs (scFv) subsequences can be joined by a linker sequence thereby forming a scFv - scFv chimera. Antibody subsequences and fragments include single-chain antibodies or variable region(s) alone or in combination with all or a portion of other subsequences.

Antibody subsequences and fragments can be prepared by proteolytic hydrolysis of the antibody, for example, by pepsin or papain digestion of whole antibodies. Antibody subsequences and fragments produced by enzymatic cleavage with pepsin provide a 5S fragment denoted F(ab')₂. This fragment can be further cleaved using a thiol reducing agent to produce 3.5S Fab' monovalent fragments. Alternatively, an enzymatic cleavage using pepsin produces two monovalent Fab' fragments and the Fc fragment directly (see, *e.g*., U.S. Patent Nos. 4,036,945 and 4,331,647; and Edelman et al., Methods Enymol. 1:422 (1967)). Other methods of cleaving antibodies, such as separation of heavy chains to form monovalent light-heavy chain fragments, further cleavage of fragments, or other enzymatic or chemical may also be used.

Proteins and antibodies, as well as subsequences and fragments thereof, can be produced by genetic methodology. Such techniques include expression of all or a part of the gene encoding the protein or antibody into a host cell such as Cos cells or *E. coli.* The recombinant host cells synthesize full length or a subsequence, for example, an scFv (see, *e.g*., Whitlow et al., In: Methods: A Companion to Methods in Enzymology 2:97 (1991), Bird et al., Science 242:423 (1988); and U.S. Patent No. 4,946,778). Single-chain Fvs and antibodies can be produced as described in U.S. Patent Nos. 4,946,778 and 5,258,498; Huston et al., Methods Enzymol. 203:46 (1991); Shu et al., Proc. Natl. Acad. Sci. USA 90:7995 (1993); and Skerra et al., Science 240:1038 (1988).

Modified proteins also include one or more D-amino acids substituted for L-amino acids (and mixtures thereof), structural and functional analogues, for example, peptidomimetics having synthetic or non-natural amino acids or amino acid analogues and derivatized forms. Modifications include cyclic structures such as an end-to-end amide bond between the amino and carboxy-terminus of the molecule or intra- or inter-molecular disulfide bond.

Modified proteins further include amino acid substitutions. In particular embodiments, a modified protein has one or a few conservative or non-conservative substitutions. Such proteins that include amino acid substitutions can be encoded by a nucleic acid. Consequently, nucleic acid sequences encoding proteins that include amino acid substitutions are also provided.

A "conservative substitution" is the replacement of one amino acid by a biologically, chemically or structurally similar residue. Biologically similar means that the substitution does not destroy a biological activity, *e.g*., SAM-6 binding activity. Structurally similar means that the amino acids have side chains with similar length, such as alanine, glycine and serine, or a similar size. Chemical similarity means that the residues have the same charge or are both hydrophilic or hydrophobic. Particular examples include the substitution of one hydrophobic residue, such as isoleucine, valine, leucine or methionine for another, or the substitution of one polar residue for another, such as the substitution of arginine for lysine, glutamic for aspartic acids, or glutamine for asparagine, serine for threonine, and the like.

Modified forms include derivatized sequences, for example, amino acids in which free amino groups form amine hydrochlorides, p-toluene sulfonyl groups, carbobenzoxy groups; the free carboxy groups from salts, methyl and ethyl esters; free hydroxl groups that form O-acyl or O-alkyl derivatives, as well as naturally occurring amino acid derivatives, for example, 4-hydroxyproline, for proline, 5-hydroxylysine for lysine, homoserine for serine, ornithine for lysine, etc. Modifications can be produced using methods known in the art (*e.g*., PCR based site-directed, deletion and insertion mutagenesis, chemical modification and mutagenesis, cross-linking, etc.).

Modified forms of protein (*e.g*., antibody), nucleic acid, and other compositions include additions and insertions. For example, an addition can be the covalent or non-covalent attachment of any type of molecule to a protein (*e.g*., antibody), nucleic acid or other composition. Typically additions and insertions confer a distinct function or activity.

Additions and insertions include fusion (chimeric) polypeptide or nucleic acid sequences, which is a sequence having one or more molecules not normally present in a reference native (wild type) sequence covalently attached to the sequence. A particular example is an amino acid sequence of another protein (*e.g*., antibody) to produce a multifunctional protein (*e.g*., multispecific antibody).

In accordance with the invention, there are provided proteins, antibodies, nucleic acids, and other compositions that include a heterologous domain. Heterologous domains can be an amino acid addition or insertion, but are not restricted to amino acid residues. Thus, a heterologous domain can consist of any of a variety of different types of small or large functional moieties. Such moieties include nucleic acid, peptide, carbohydrate, lipid or small organic compounds, such as a drug (*e.g*., a cell proliferative agent), metals (gold, silver), etc.

Particular non-limiting examples of heterologous domains include, for example, tags, detectable labels and cytotoxic agents. Specific examples of tags and detectable labels include enzymes (horseradish peroxidase, urease, catalase, alkaline phosphatase, beta-galactosidase, chloramphenicol transferase); enzyme substrates; ligands (*e.g*., biotin); receptors (avidin); radionuclides (*e.g*., C¹⁴, S³⁵, P³², P³³, H³, I¹²⁵, I¹³¹, gallium-67 and 68, scantium-47, indium-111, radium-223); T7-, His-, myc-, HA-and FLAG-tags; electron-dense reagents; energy transfer molecules; paramagnetic labels; fluorophores (fluorescein, rhodamine, phycoerthrin); chromophores; chemi-luminescent (imidazole, luciferase); and bio-luminescent agents. Specific examples of cytotoxic agents include diptheria, toxin, cholera toxin and ricin.

Additional examples of heterologous domains include, for example, anti-cell proliferative agents (*e.g*., anti-neoplastic, anti-tumor or anti-cancer, or anti-metastasis agents). Specific non-limiting examples of anti-cell proliferative agents are disclosed herein and known in the art.

Linker sequences may be inserted between the protein (*e.g*., antibody), nucleic acid, or other composition and the addition or insertion (*e.g*., heterologous domain) so that the two entities maintain, at least in part, a distinct function or activity. Linker sequences may have one or more properties that include a flexible structure, an inability to form an ordered secondary structure or a hydrophobic or charged character which could promote or interact with either domain. Amino acids typically found in flexible protein regions include Gly, Asn and Ser. Other near neutral amino acids, such as Thr and Ala, may also be used in the linker sequence. The length of the linker sequence may vary (see, *e.g*., U.S. Patent No. 6,087,329). Linkers further include chemical cross-linking and conjugating agents, such as sulfo-succinimidyl derivatives (sulfo-SMCC, sulfo-SMPB), disuccinimidyl suberate (DSS), disuccinimidyl glutarate (DSG) and disuccinimidyl tartrate (DST).

Further examples of additions include glycosylation, fatty acids, lipids, acetylation, phosphorylation, amidation, formylation, ubiquitinatation, and derivatization by protecting/blocking groups and any of numerous chemical modifications. Other permutations and possibilities will be readily apparent to those of ordinary skill in the art, and are considered to be within the scope of the invention.

Such modified sequences can be made using recombinant DNA technology via cell expression or in vitro translation. Polypeptide and nucleic acid sequences can also be produced by chemical synthesis using methods known in the art, for example, an automated peptide synthesis apparatus (see, *e.g*., Applied Biosystems, Foster City, CA).

In accordance with the invention, there are provided isolated or purified nucleic acids encoding glycoproteins denoted as SAM-6 Receptor (SAM-6/R) or SAM-6/R glycoprotein, having an apparent molecular weight in a range of about 80-82 kilodaltons (KDa) as determined by denaturing gel electrophoresis. In one embodiment, a nucleic acid sequence encodes a SAM-6/R glycoprotein having polypeptide sequence homology to Grp78 as set forth in SEQ ID NO:1. In another embodiment, a nucleic acid sequence encodes a SAM-6/R glycoprotein capable of having linked thereto at least one nitrogen (N)- or oxygen (O)-linked carbohydrate moiety distinct from Grp78. In a further embodiment, a nucleic acid sequence encodes a SAM-6/R glycoprotein capable of having linked thereto at least one nitrogen (N)- or oxygen (O)-linked carbohydrate moiety that is an epitope or is a part of an epitope to which SAM-6 antibody specifically binds. Nucleic acids according to the invention therefore include sequences encoding 1) SAM-6/R glycoprotein that has polypeptide sequence identity to Grp78 as set forth in SEQ ID NO:1; 2) SAM-6/R glycoprotein sequences (*e.g*., capable of having linked thereto at least one nitrogen (N)- or oxygen (O)-linked carbohydrate moiety) that SAM-6 is capable of specifically binding; and 3) SAM-6/R glycoprotein subsequences and fragments (*e.g*., SEQ ID NOs:2-12).

In accordance with the invention, there are also provided isolated or purified nucleic acids encoding subsequences and fragments of SAM-6/R glycoprotein. In one embodiment a nucleic acid sequence encodes a SAM-6/R glycoprotein sequence capable of having linked thereto at least one nitrogen (N)- or oxygen (O)-linked carbohydrate moiety, the carbohydrate moiety optionally distinct from a carbohydrate moiety of Grp78. In particular aspects, the nucleic acid sequence has a length from about 10-20, 20-30, 30-50, 50-100, 100-150, 150-200, 200-250, 250-300, 300-400, 400-500, 500-1000, 1000-2000, nucleotides, or any numerical value or range within or encompassing such lengths, and optionally encodes a SAM-6/R glycoprotein capable of having linked thereto at least one nitrogen (N)- or oxygen (O)-linked carbohydrate moiety distinct from a carbohydrate moiety of Grp78.

The terms "nucleic acid" and "polynucleotide" and the like refer to at least two or more ribo- or deoxy-ribonucleic acid base pairs (nucleotides) that are linked through a phosphoester bond or equivalent. Nucleic acids include polynucleotides and polynucleosides. Nucleic acids include single, double or triplex, circular or linear, molecules. Exemplary nucleic acids include but are not limited to: RNA, DNA, cDNA, genomic nucleic acid, naturally occurring and non naturally occurring nucleic acid, e.g., synthetic nucleic acid.

Nucleic acids can be of various lengths. Nucleic acid lengths typically range from about 20 nucleotides to 20 Kb, or any numerical value or range within or encompassing such lengths, 10 nucleotides to 10Kb, 1 to 5 Kb or less, 1000 to about 500 nucleotides or less in length. Nucleic acids can also be shorter, for example, 100 to about 500 nucleotides, or from about 12 to 25, 25 to 50, 50 to 100, 100 to 250, or about 250 to 500 nucleotides in length, or any numerical value or range or value within or encompassing such lengths. Shorter polynucleotides are commonly referred to as "oligonucleotides" or "probes" of single- or double-stranded DNA. However, there is no upper limit to the length of such oligonucleotides.

Polynucleotides include L- or D-forms and mixtures thereof, which additionally may be modified to be resistant to degradation when administered to a subject. Particular examples include 5' and 3' linkages resistant to endonucleases and exonucleases present in various tissues or fluids of a subject.

In another embodiment, the invention provides nucleic acids that hybridize to a nucleic acid that encodes all or a subsequence or fragment of SAM-6/R glycoprotein sequence, that is at least 75-90% complementary or homologous to the nucleic acid sequence that encodes all or a subsequence or fragment of SAM-6/R glycoprotein sequence. In one embodiment, the nucleic acid sequence has a length from about 10-20, 20-30, 30-50, 50-100, 100-150, 150-200, 200-250, 250-300, 300-400, 400-500, 500-1000, 1000-2000, nucleotides, or any numerical value or range within or encompassing such lengths. In particular aspects, the nucleic acid sequence' hybridizes to a nucleic acid sequence that encodes SAM-6/R glycoprotein capable of having linked thereto at least one nitrogen (N)- or oxygen (O)-linked carbohydrate moiety distinct from Grp78.

The term "hybridize" and grammatical variations thereof refer to the binding between nucleic acid sequences. Hybridizing sequences will generally have more than about 50% homology (e.g., 50%, 60%, 70%, 80%, 90%, or more identity) to a nucleic acid that encodes an amino acid sequence of a reference (*e.g*., SAM-6/R glycoprotein) sequence or a sequence complementary to a nucleic acid that encodes an amino acid sequence of a reference (*e.g*., SAM-6/R glycoprotein) sequence. Hybridizing sequences that are 100% or fully complementary to a reference sequence, for example, to a nucleic acid that encodes an amino acid sequence of a reference (*e.g*., SAM-6/R glycoprotein) sequence, exhibit 100% base pairing with no mismatches. The hybridization region between hybridizing sequences typically is at least about 12-15 nucleotides, 15-20 nucleotides, 20-30 nucleotides, 30-50 nucleotides, 50-100 nucleotides, 100 to 200 nucleotides or more, or any numerical value or range within or encompassing such lengths.

In accordance with the invention, there are further provided antisense polynucleotides, small interfering RNA, and ribozyme nucleic acid that specifically hybridize to the nucleic acid sequence encoding SAM-6/R glycoprotein, or a portion thereof, or a sequence complementary to a nucleic acid that encodes SAM-6/R glycoprotein, or a portion thereof. Antisense polynucleotides can have a length from about 10-20, 20-30, 30-50, 50-100, 100-150, 150-200, 200-250, 250-300, 300-400, 400-500, 500-1000, 1000-2000 nucleotides, or any numerical value or range within or encompassing such lengths. In one embodiment, a nucleic acid sequence comprises an antisense polynucleotide that specifically hybridizes to the nucleic acid sequence encoding a SAM-6/R glycoprotein capable of having linked thereto an N- or O-linked carbohydrate moiety distinct from a carbohydrate moiety of Grp78. In particular aspects, an antisense is at least 90% complementary or homologous to a nucleic acid sequence encoding SAM-6/R glycoprotein, or a nucleic acid sequence encoding SAM-6/R glycoprotein capable of having linked thereto at least one nitrogen (N)- or oxygen (O)-linked carbohydrate moiety distinct from a carbohydrate moiety of Grp78, or a sequence complementary to a nucleic acid encoding SAM-6/R glycoprotein, or a nucleic acid sequence encoding SAM-6/R glycoprotein capable of having linked thereto at least one nitrogen (N)- or oxygen (O)-linked carbohydrate moiety distinct from a carbohydrate moiety of Grp78

As used herein, the term "antisense" refers to a polynucleotide or peptide nucleic acid capable of binding to a specific DNA or RNA sequence. Antisense includes single, double, triple or greater stranded RNA and DNA polynucleotides and peptide nucleic acids (PNAs) that bind RNA transcript or DNA. Particular examples include RNA and DNA antisense that binds to sense RNA. For example, a single stranded nucleic acid can target a protein transcript that participates in metabolism, catabolism, removal or degradation of glycogen from a cell (*e.g*., mRNA). Antisense molecules are typically 95-100% complementary to the sense strand but can be "partially" complementary, in which only some of the nucleotides bind to the sense molecule (less than 100% complementary, *e.g*., 95%, 90%, 80%, 70% and sometimes less), or any numerical value or range within or encompassing such percent values.

Triplex forming antisense can bind to double strand DNA thereby inhibiting transcription of the gene. Oligonucleotides derived from the transcription initiation site of the gene, *e.g*., between positions -10 and +10 from the start site, are one particular example.

Short interfering RNA (referred to as siRNA or RNAi) for inhibiting gene expression is known in the art (see, *e.g*., Kennerdell et al., Cell 95:1017 (1998); Fire et al., Nature, 391:806 (1998); WO 02/44321; WO 01/68836; WO 00/44895, WO 99/32619, WO 01/75164, WO 01/92513, WO 01/29058, WO 01/89304, WO 02/16620; and WO 02/29858). RNAi silencing can be induced by a nucleic acid encoding an RNA that forms a "hairpin" structure or by expressing RNA from each end of an encoding nucleic acid, making two RNA molecules that hybridize.

Ribozymes, which are enzymatic RNA molecules that catalyze the specific cleavage of RNA can be used to inhibit expression of the encoded protein. Ribozymes form sequence-specific hybrids with complementary target RNA, which is then cleaved. Specific examples include engineered hammerhead motif ribozyme molecules that can specifically and efficiently catalyze endonucleolytic cleavage of sequences encoding a protein that participates in metabolism, catabolism, removal or degradation of glycogen, for example.

Antisense, ribozymes, RNAi and triplex forming nucleic acid are referred to collectively herein as "inhibitory nucleic acid" or "inhibitory polynucleotides." Such inhibitory nucleic acid or polynucleotides can inhibit or prevent expression of SAM-6/R glycoprotein.

Inhibitory polynucleotides do not require expression control elements in order to function *in vivo*. Inhibitory polynucleotides can be absorbed by the cell or enter the cell via passive diffusion. Inhibitory polynucleotides can optionally be introduced into a cell using a vector. Inhibitory polynucleotides may be encoded by a nucleic acid so that it is transcribed. Furthermore, a nucleic acid encoding an inhibitory polynucleotide may be operatively linked to an expression control element for sustained or increased expression of the encoded antisense in cells or *in vivo.* Inhibitory nucleic acid can be designed based upon protein and nucleic acid sequences disclosed herein or available in the database.

Nucleic acid sequences further include nucleotide and nucleoside substitutions, additions and deletions, as well as derivatized forms and fusion/chimeric sequences (*e.g*., encoding recombinant polypeptide). For example, due to the degeneracy of the genetic code, nucleic acids include sequences and subsequences degenerate with respect to nucleic acids that encode SAM-6/R glycoprotein and subsequences or fragments (*e.g*., a SAM-6/R glycoprotein fragment capable of having linked thereto at least one nitrogen (N)- or oxygen (O)-linked carbohydrate moiety distinct from Grp78) or variants thereof. Other examples are nucleic acids complementary to a sequence that encodes an amino acid sequence of a SAM-6/R glycoprotein and subsequences or fragments thereof.

Nucleic acid deletions (subsequences and fragments) can have from about 10 to 25, 25 to 50 or 50 to 100 nucleotides. Such nucleic acids are useful for expressing polypeptide subsequences, for genetic manipulation (as primers and templates for PCR amplification), and as probes to detect the presence or an amount of a sequence encoding a protein (*e.g*., via hybridization), in a cell, culture medium, biological sample (*e.g*., tissue, organ, blood or serum), or in a subject.

Nucleic acids can be produced using various standard cloning and chemical synthesis techniques. Techniques include, but are not limited to nucleic acid amplification, *e.g*., polymerase chain reaction (PCR), with genomic DNA or cDNA targets using primers (*e.g*., a degenerate primer mixture) capable of annealing to antibody encoding sequence. Nucleic acids can also be produced by chemical synthesis (*e.g*., solid phase phosphoramidite synthesis) or transcription from a gene. The sequences produced can then be translated in vitro, or cloned into a plasmid and propagated and then expressed in a cell *(e.g.,* a host cell such as yeast or bacteria, a eukaryote such as an animal or mammalian cell or in a plant).

In accordance with the invention, there are further provided vectors that comprise nucleic acid sequences of the invention. In one embodiment, a vector includes a nucleic acid sequence encoding SAM-6/R glycoprotein. In another embodiment, a vector includes a nucleic acid sequence encoding SAM-6/R glycoprotein subsequence or fragment capable of having linked thereto at least one nitrogen (N)- or oxygen (O)-linked carbohydrate moiety that SAM-6 antibody is capable of specifically binding.

Vectors include viral, prokaryotic (bacterial) and eukaryotic (plant, fungal, mammalian) vectors. Vectors can be used for expression of nucleic acids in vitro or *in vivo.* Such vectors, referred to as "expression vectors," are useful for introducing nucleic acids, including nucleic acids that encode a SAM-6/R glycoprotein, subsequences and fragments thereof, nucleic acids that encode inhibitory nucleic acid, and expressing the encoded protein or inhibitory nucleic acid (*e.g*., in solution or in solid phase), in cells or in a subject *in vivo.*

Vectors can also be used for manipulation of nucleic acids. For genetic manipulation "cloning vectors" can be employed, and to transcribe or translate the inserted nucleic acid.

A vector generally contains an origin of replication for propagation in a cell in vitro or *in vivo.* Control elements, including expression control elements, present within a vector, can be included to facilitate transcription and translation, as appropriate.

Vectors can include a selection marker. A "selection marker" is a gene that allows for the selection of cells containing the gene. "Positive selection" refers to a process in which cells that contain the selection marker survive upon exposure to the positive selection. Drug resistance is one example of a positive selection marker-cells containing the marker will survive in culture medium containing the selection drug, and cells lacking the marker will die. Selection markers include drug resistance genes such as *neo*, which confers resistance to G418; *hygr*, which confers resistance to hygromycin; and *puro*, which confers resistance to puromycin. Other positive selection marker genes include genes that allow identification or screening of cells containing the marker. These genes include genes for fluorescent proteins (GFP and GFP-like chromophores, luciferase), the lacZ gene, the alkaline phosphatase gene, and surface markers such as CD8, among others. "Negative selection" refers to a process in which cells containing a negative selection marker are killed upon exposure to an appropriate negative selection agent. For example, cells which contain the herpes simplex virus-thymidine kinase (*HSV-tk*) gene (Wigler et al., Cell 11:223 (1977)) are sensitive to the drug gancyclovir (GANC). Similarly, the *gpt* gene renders cells sensitive to 6-thioxanthine.

Viral vectors include those based upon retroviral (lentivirus for infecting dividing as well as non-dividing cells), foamy viruses (U.S. Patent Nos. 5,624,820, 5,693,508, 5,665,577, 6,013,516 and 5,674,703; WO92/05266 and WO92/14829), adenovirus (U.S. Patent Nos. 5,700,470, 5,731,172 and 5,928,944), adeno-associated virus (AAV) (U.S. Patent No. 5,604,090), herpes simplex virus vectors (U.S. Patent No. 5,501,979), cytomegalovirus (CMV) based vectors (U.S. Patent No. 5,561,063), reovirus, rotavirus genomes, simian virus 40 (SV40) or papilloma virus (Cone et al., Proc. Natl. Acad. Sci. USA 81:6349 (1984); Eukaryotic Viral Vectors, Cold Spring Harbor Laboratory, Gluzman ed., 1982; Sarver et al., Mol. Cell. Biol. 1:486 (1981); U.S. Patent No. 5,719,054). Adenovirus efficiently infects slowly replicating and/or terminally differentiated cells and can be used to target slowly replicating and/or terminally differentiated cells. Additional viral vectors useful for expression include parvovirus, Norwalk virus, coronaviruses, paramyxo- and rhabdoviruses, togavirus (*e.g*., sindbis virus and semliki forest virus) and vesicular stomatitis virus (VSV).

Vectors including a nucleic acid can be expressed when the nucleic acid is operably linked to an expression control element. As used herein, the term "operably linked" refers to a physical or a functional relationship between the elements referred to that permit them to operate in their intended fashion. Thus, an expression control element "operable linked" to a nucleic acid means that the control element modulates nucleic acid transcription and as appropriate, translation of the transcript.

The term "expression control element" refers to nucleic acid that influences expression of an operably linked nucleic acid. Promoters and enhancers are particular non-limiting examples of expression control elements. A "promoter sequence" is a DNA regulatory region capable of initiating transcription of a downstream (3' direction) sequence. The promoter sequence includes nucleotides that facilitate transcription initiation. Enhancers also regulate gene expression, but can function at a distance from the transcription start site of the gene to which it is operably linked. Enhancers function at either 5' or 3' ends of the gene, as well as within the gene (*e.g*., in introns or coding sequences). Additional expression control elements include leader sequences and fusion partner sequences, internal ribosome binding sites (IRES) elements for the creation of multigene, or polycistronic, messages, splicing signal for introns, maintenance of the correct reading frame of the gene to permit in-frame translation of mRNA, polyadenylation signal to provide proper polyadenylation of the transcript of interest, and stop codons.

Expression control elements include "constitutive" elements in which transcription of an operably linked nucleic acid occurs without the presence of a signal or stimuli. Expression control elements that confer expression in response to a signal or stimuli, which either increase or decrease expression of operably linked nucleic acid, are "regulatable." A regulatable element that increases expression of operably linked nucleic acid in response to a signal or stimuli is referred to as an "inducible element." A regulatable element that decreases expression of the operably linked nucleic acid in response to a signal or stimuli is referred to as a "repressible element" (*i.e*., the signal decreases expression; when the signal is removed or absent, expression is increased).

Expression control elements include elements active in a particular tissue or cell type, referred to as "tissue-specific expression control elements." Tissue-specific expression control elements are typically more active in specific cell or tissue types because they are recognized by transcriptional activator proteins, or other transcription regulators active in the specific cell or tissue type, as compared to other cell or tissue types.

Tissue-specific expression control elements include promoters and enhancers active in hyperproliferative cells, such as cell proliferative disorders including neoplasias, tumors and cancers, and metastasis. Particular non-limiting examples of such promoters are hexokinase II, COX-2, alpha-fetoprotein, carcinoembryonic antigen, DE3/MUC1, prostate specific antigen, C-erB2/neu, telomerase reverse transcriptase and hypoxia-responsive promoter.

For bacterial expression, constitutive promoters include T7, as well as inducible promoters such as pL of bacteriophage λ, plac, ptrp, ptac (ptrp-lac hybrid promoter). In insect cell systems, constitutive or inducible promoters (*e.g*., ecdysone) may be used. In yeast, constitutive promoters include, for example, ADH or LEU2 and inducible promoters such as GAL (see, *e.g.*, Ausubel et al., In: Current Protocols in Molecular Biology, Vol. 2, Ch. 13, ed., Greene Publish. Assoc. & Wiley Interscience, 1988; Grant et al., In: Methods in Enzymology, 153:516-544 (1987), eds. Wu & Grossman, 1987, Acad. Press, N.Y.; Glover, DNA Cloning, Vol. II, Ch. 3, IRL Press, Wash., D.C., 1986; Bitter, In: Methods in Enzymology, 152:673-684 (1987), eds. Berger & Kimmel, Acad. Press, N.Y.; and, Strathern et al., The Molecular Biology of the Yeast Saccharomyces eds. Cold Spring Harbor Press, Vols. I and II (1982)).

For mammalian expression, constitutive promoters of viral or other origins may be used. For example, SV40, or viral long terminal repeats (LTRs) and the like, or inducible promoters derived from the genome of mammalian cells (*e.g*., metallothionein IIA promoter; heat shock promoter, steroid/thyroid hormone/retinoic acid response elements) or from mammalian viruses (*e.g*., the adenovirus late promoter; mouse mammary tumor virus LTR) are used.

In accordance with the invention, there are provided host cells transformed or transfected with a nucleic acid or vector of the invention. Host cells include but are not limited to prokaryotic and eukaryotic cells such as bacteria, fungi (yeast), plant, insect, and animal (*e.g*., mammalian, including primate and human) cells. For example, bacteria transformed with recombinant bacteriophage nucleic acid, plasmid nucleic acid or cosmid nucleic acid expression vectors; yeast transformed with recombinant yeast expression vectors; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid); insect cell systems infected with recombinant virus expression vectors (*e.g*., baculovirus); and animal cell systems infected with recombinant virus expression vectors (*e.g*., retroviruses, adenovirus, vaccinia virus), or transformed animal cell systems engineered for stable expression.

The cells may be a primary cell isolate, cell culture (*e.g*., passaged, established or immortalized cell line), or part of a plurality of cells, or a tissue or organ *ex vivo* or in a subject (*in vivo*). In particular embodiments, a cell is a hyperproliferative cell, a cell comprising a cellular hyperproliferative disorder, an immortalized cell, neoplastic cell, tumor cell or cancer cell, or metastasis cell.

The term "transformed" or "transfected" when use in reference to a cell (*e.g*., a host cell) or organism, means a genetic change in a cell following incorporation of an exogenous molecule, for example, a protein or nucleic acid (*e.g.*, a transgene) into the cell. Thus, a "transfected" or "transformed" cell is a cell into which, or a progeny thereof in which an exogenous molecule has been introduced by the hand of man, for example, by recombinant DNA techniques.

The nucleic acid or protein can be stably or transiently transfected or transformed (expressed) in the cell and progeny thereof. The cell(s) can be propagated and the introduced protein expressed, or nucleic acid transcribed. A progeny of a transfected or transformed cell may not be identical to the parent cell, since there may be mutations that occur during replication.

Typically, cell transfection or transformation employs a "vector," which refers to a plasmid, virus, such as a viral vector, or other vehicle known in the art that can be manipulated by insertion or incorporation of a nucleic acid.

A viral particle or vesicle can be designed to be targeted to particular cell types (*e.g*., hyperproliferating cells) by inclusion of a protein on the surface that binds to a target cell ligand or receptor. Alternatively, a cell type-specific promoter and/or enhancer can be included in the vector in order to express the nucleic acid in target cells. Thus, the viral particle or vesicle itself, viral vector, or a protein on the viral surface can be made to target cells for transfection or transformation in vitro, *ex vivo* or *in vivo.*

Introduction of compositions (*e.g*., protein and nucleic acid) into target cells (*e.g.,* host cells) can also be carried out by methods known in the art such as osmotic shock (*e.g.*, calcium phosphate), electroporation, microinjection, cell fusion, etc. Introduction of nucleic acid and polypeptide *in vitro*, *ex vivo* and *in vivo* can also be accomplished using other techniques. For example, a polymeric substance, such as polyesters, polyamine acids, hydrogel, polyvinyl pyrrolidone, ethylene-vinylacetate, methylcellulose, carboxymethylcellulose, protamine sulfate, or lactide/glycolide copolymers, polylactide/glycolide copolymers, or ethylenevinylacetate copolymers. A nucleic acid can be entrapped in microcapsules prepared by coacervation techniques or by interfacial polymerization, for example, by the use of hydroxymethylcellulose or gelatin-microcapsules, or poly (methylmethacrolate) microcapsules, respectively, or in a colloid system. Colloidal dispersion systems include macromolecule complexes, nano-capsules, microspheres, beads, and lipid-based systems, including oil-in-water emulsions, micelles, mixed micelles, and liposomes.

Liposomes for introducing various compositions into cells are known in the art and include, for example, phosphatidylcholine, phosphatidylserine, lipofectin and DOTAP (*e.g.,* U.S. Patent Nos. 4,844,904, 5,000,959, 4,863,740, and 4,975,282; and GIBCO-BRL, Gaithersburg, Md). Piperazine based amphilic cationic lipids useful for gene therapy also are known (see, *e.g*., U.S. Patent No. 5,861,397). Cationic lipid systems also are known (see, *e.g*., U.S. Patent No. 5,459,127). Polymeric substances, microcapsules and colloidal dispersion systems such as liposomes are collectively referred to herein as "vesicles." Accordingly, viral and non-viral vector means of delivery into cells, tissue or organs, *in vitro*, *in vivo* and *ex vivo* are included.

The invention includes *in vivo* methods. For example, a cell such as a hyperproliferative cell or cellular hyperproliferative disorder that expresses SAM-6/R glycoprotein can be present in a subject, such as a mammal (*e.g*., a human subject). Cells comprising the cell proliferative or cellular hyperproliferative disorder may therefore be treated by administering, for example, an antibody, or subsequence or fragment thereof, that specifically binds to SAM-6/R glycoprotein or an inhibitory nucleic acid of SAM-6/R glycoprotein. Cells comprising the cell proliferative or cellular hyperproliferative disorder may also be treated by administering, for example, a SAM-6/R glycoprotein or a subsequence thereof, which can elicit an immune response against SAM-6/R glycoprotein thereby functioning as a vaccine. In addition, disorders and diseases associated with or caused by undesirable or excessive LDL or oxLDL levels can be treated in accordance with the invention by administering, for example, an antibody, or subsequence or fragment thereof that specifically binds to SAM-6/R glycoprotein, which reduces LDL or oxLDL.

In accordance with the invention, there are provided methods of treating cell proliferation or a cell proliferative or cellular hyperproliferative disorder in a subject. In one embodiment, a method includes administering to a subject an antibody that specifically binds to SAM-6/R glycoprotein antibody in an amount effective to treat the cell proliferation or a cell proliferative or cellular hyperproliferative disorder in the subject. In another embodiment, a method includes administering to a subject a SAM-6/R glycoprotein in an amount effective to treat the cell proliferation or a cell proliferative or cellular hyperproliferative disorder in the subject.

As used herein, the terms "cell proliferative disorder" and "cellular hyperproliferative disorder" and grammatical variations thereof, when used in reference to a cell, tissue or organ, refers to any undesirable, excessive or abnormal cell, tissue or organ growth, proliferation, differentiation or survival. A hyperproliferative cell denotes a cell whose growth, proliferation, or survival is greater than desired, such as a reference normal cell, *e.g*., a cell that is of the same tissue or organ but is not a hyperproliferative cell, or a cell that fails to differentiate normally. Cell proliferative and hyperproliferative disorders include diseases and physiological conditions, both benign hyperplastic conditions characterized by undesirable, excessive or abnormal cell numbers, cell growth, cell proliferation, cell survival or differentiation in a subject. Specific examples of such disorders include metastatic and non-metastatic neoplasia, tumors and cancers (malignancies).

In various embodiments, a method includes administering to a subject an antibody or subsequence thereof that specifically binds to SAM-6/R glycoprotein in an amount effective to treat the cell proliferative or cellular hyperproliferative disorder in the subject. In particular aspects, the disorder is a neoplasia, tumor or metastatic or non-metastatic cancer (malignancy). In additional aspects, the disorder affects or is present in part at least in breast, lung, thyroid, head and neck, nasopharynx, nose or sinuses, brain, spine, adrenal gland, thyroid, lymph, gastrointestinal (mouth, esophagus, stomach, duodenum, ileum, jejunum (small intestine), colon, rectum), genito-urinary tract (uterus, ovary, cervix, bladder, testicle, penis, prostate), kidney, pancreas, adrenal gland, liver, bone, bone marrow, lymph, blood, muscle, skin, or the hematopoetic system.

The terms "neoplasia" and "tumor" are used interchangeably herein and refer to a cell or population of cells of any cell, tissue or organ origin, whose growth, proliferation or survival is greater than growth, proliferation or survival of a normal counterpart cell. A "cancer" is a malignant neoplasia or tumor, which typically invades other regions, tissues or organs and has the potential to metastasize to other sites via blood or lymph transport.

Neoplasias, tumors and cancers include a sarcoma, carcinoma, adenocarcinoma, melanoma, myeloma, blastoma, glioma, lymphoma or leukemia. Exemplary cancers include, for example, carcinoma, sarcoma, adenocarcinoma, melanoma, neural (blastoma, glioma), mesothelioma and reticuloendothelial, lymphatic or haematopoietic neoplastic disorders (*e.g*., myeloma, lymphoma or leukemia). In particular aspects, a neoplasia, tumor or cancer includes a lung adenocarcinoma, lung carcinoma, diffuse or interstitial gastric carcinoma, colon adenocarcinoma, prostate adenocarcinoma, esophagus carcinoma, breast carcinoma, pancreas adenocarcinoma, ovarian adenocarcinoma, or uterine adenocarcinoma.

Neoplasia, tumors and cancers include benign, malignant, metastatic and non-metastatic types, and include any stage (I, II, III, IV or V) or grade (G1, G2, G3, etc.) of neoplasia, tumor, or cancer, or a neoplasia, tumor, cancer or metastasis that is progressing, worsening, stabilized or in remission.

Neoplasias, tumors and cancers can arise from a multitude of primary tumor types, including but not limited to breast, lung, thyroid, head and neck, nasopharynx, nose or sinuses, brain, spine, adrenal gland, thyroid, lymph, gastrointestinal (mouth, esophagus, stomach, duodenum, ileum, jejunum (small intestine), colon, rectum), genito-urinary tract (uterus, ovary, cervix, bladder, testicle, penis, prostate), kidney, pancreas, adrenal gland, liver, bone, bone marrow, lymph, blood, muscle, skin, and the hematopoetic system, and may metastasize to secondary sites.

A "solid neoplasia, tumor or cancer" refers to neoplasia, tumor or cancer (*e.g.*, metastasis) that typically aggregates together and forms a mass. Specific examples include visceral tumors such as melanomas, breast, pancreatic, uterine and ovarian cancers, testicular cancer, including seminomas, gastric or colon cancer, hepatomas, adrenal, renal and bladder carcinomas, lung, head and neck cancers and brain tumors/cancers.

Carcinomas refer to malignancies of epithelial or endocrine tissue, and include respiratory system carcinomas, gastrointestinal system carcinomas, genitourinary system carcinomas, testicular carcinomas, breast carcinomas, prostatic carcinomas, endocrine system carcinomas, and melanomas. The term also includes carcinosarcomas, *e.g*., which include malignant tumors composed of carcinomatous and sarcomatous tissues. Adenocarcinoma includes a carcinoma of a glandular tissue, or in which the tumor forms a gland like structure. Melanoma refers to malignant tumors of melanocytes and other cells derived from pigment cell origin that may arise in the skin, the eye (including retina), or other regions of the body. Additional carcinomas can form from the uterine/cervix, lung, head/neck, colon, pancreas, testes, adrenal gland, kidney, esophagus, stomach, liver and ovary.

Sarcomas refer to malignant tumors of mesenchymal cell origin. Exemplary sarcomas include for example, lymphosarcoma, liposarcoma, osteosarcoma, chondrosarcoma, leiomyosarcoma, rhabdomyosarcoma and fibrosarcoma.

Neural neoplasias include glioma, glioblastoma, meningioma, neuroblastoma, retinoblastoma, astrocytoma, oligodendrocytoma

Specific non-limiting examples of neoplasias, tumors and cancers amenable to treatment include malignant and non-malignant neoplasias, tumors and cancers, and metastasis. In particular, melanomas, gastric tissue, lung squamous cell carcinoma, lung adenocarcinoma cell and nasal cancer cells, of any stage (e.g., stages IA, IB, IIA, IIB, IIIA, IIIB or IV) or grade (e.g., grades G1, G2 or G3). Specific non-limiting examples of metastasis include lung squamous cell carcinoma and adenocarcinoma metastasis to lymph node and brain; breast cancer (invasive ductal) metastasis to lymph node; colon adenocarcinoma metastasis to liver and lymph node; SAM-6/R glycoprotein was detected on stomach adenocarcinoma (intestinal and diffuse) metastasis to lymph node; pancreas adenocarcinmoa metastasis to lymph node; head and neck squamous cell carcinoma metastasis to lymph node; and melanoma metastasis to rectum, esophagus, skin, parotid gland, colon, adrenal gland and nasal epithelium.

A "liquid neoplasia, tumor or cancer" refers to a neoplasia, tumor or cancer of the reticuloendothelial or hematopoetic system, such as a lymphoma, myeloma, or leukemia, or a neoplasia that is diffuse in nature. Particular examples of leukemias include acute and chronic lymphoblastic, myeolblastic and multiple myeloma. Typically, such diseases arise from poorly differentiated acute leukemias, *e.g*., erythroblastic leukemia and acute megakaryoblastic leukemia. Specific myeloid disorders include, but are not limited to, acute promyeloid leukemia (APML), acute myelogenous leukemia (AML) and chronic myelogenous leukemia (CML); lymphoid malignancies include, but are not limited to, acute lymphoblastic leukemia (ALL), which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Specific malignant lymphomas include, non-Hodgkin lymphoma and variants, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

In accordance with the invention, there are provided methods of reducing LDL or oxLDL, as well as methods of treating disorders and diseases associated with or caused by undesirable or excessive LDL or oxLDL levels. In one embodiment, a method includes administering to a subject an antibody that specifically binds to SAM-6/R glycoprotein in an amount effective to treat the disorder or disease associated with or caused by undesirable or excessive LDL or oxLDL levels (*e.g*., plasma levels) in the subject.

Non-limiting exemplary disorders and diseases associated with undesirable or excessive LDL or oxLDL levels include hyperlipidemia, hypercholesterolemia, arteriosclerosis, cardiovascular disease, coronary heart disease (CHD), stroke, glomerulonecrosis, high blood pressure and diabetes. Thus, in additional embodiments, a method includes administering to a subject an antibody that specifically binds to SAM-6/R glycoprotein in an amount effective to treat hyperlipidemia, hypercholesterolemia, arteriosclerosis, cardiovascular disease, coronary heart disease (CHD), stroke, glomerulonecrosis, high blood pressure or diabetes.

As used herein, the terms "treat," "treating," "treatment" and grammatical variations thereof mean subjecting an individual patient to a protocol, regimen, process or remedy, in which it is desired to obtain a physiologic response or outcome in that patient. Since every treated patient may not respond to a particular treatment protocol, regimen, process or remedy, treating does not require that the desired physiologic response or outcome be achieved in each and every patient or patient population. Accordingly, a given patient or patient population may fail to respond or respond inadequately to treatment.

Methods of the invention may be practiced by any mode of administration or by any route, systemic, regional and local administration. Exemplary administration routes include intravenous, intrarterial, intradermal, intramuscular, subcutaneous, intra-pleural, transdermal (topical), transmucosal, intra-cranial, intra-spinal, intra-ocular, rectal, oral (alimentary) and mucosal.

Methods of the invention include, among other things, methods that provide a detectable or measurable improvement in a condition of a given subject, such as alleviating or ameliorating one or more adverse (physical) symptoms or consequences associated with the presence of a cell proliferative or cellular hyperproliferative disorder, neoplasia, tumor or cancer, or metastasis, i.e., a therapeutic benefit or a beneficial effect.

A therapeutic benefit or beneficial effect is any objective or subjective, transient, temporary, or long-term improvement in the condition or pathology, or a reduction in onset, severity, duration or frequency of an adverse symptom associated with or caused by cell proliferation or a cellular hyperproliferative disorder such as a neoplasia, tumor or cancer, or metastasis. A satisfactory clinical endpoint of a treatment method in accordance with the invention is achieved, for example, when there is an incremental or a partial reduction in severity, duration or frequency of one or more associated pathologies, adverse symptoms or complications, or inhibition or reversal of one or more of the physiological, biochemical or cellular manifestations or characteristics of cell proliferation or a cellular hyperproliferative disorder such as a neoplasia, tumor or cancer, or metastasis. A therapeutic benefit or improvement therefore be a cure, such as destruction of target proliferating cells (*e.g*., neoplasia, tumor or cancer, or metastasis) or ablation of one or more, most or all pathologies, adverse symptoms or complications associated with or caused by cell proliferation or the cellular hyperproliferative disorder such as a neoplasia, tumor or cancer, or metastasis. However, a therapeutic benefit or improvement need not be a cure or complete destruction of all target proliferating cells (*e.g*., neoplasia, tumor or cancer, or metastasis) or ablation of all pathologies, adverse symptoms or complications associated with or caused by cell proliferation or the cellular hyperproliferative disorder such as a neoplasia, tumor or cancer, or metastasis. For example, partial destruction of a tumor or cancer cell mass, or a stabilization of the tumor or cancer mass, size or cell numbers by inhibiting progression or worsening of the tumor or cancer, can reduce mortality and prolong lifespan even if only for a few days, weeks or months, even though a portion or the bulk of the tumor or cancer mass, size or cells remain.

Specific non-limiting examples of therapeutic benefit include a reduction in neoplasia, tumor or cancer, or metastasis volume (size or cell mass) or numbers of cells, inhibiting or preventing an increase in neoplasia, tumor or cancer volume (*e.g*., stabilizing), slowing or inhibiting neoplasia, tumor or cancer progression, worsening or metastasis, stimulating, inducing or increasing neoplasia, tumor or cancer cell lysis or apoptosis or inhibiting neoplasia, tumor or cancer proliferation, growth or metastasis. An invention method may not take effect immediately. For example, treatment may be followed by an increase in the neoplasia, tumor or cancer cell numbers or mass, but over time eventual stabilization or reduction in tumor cell mass, size or numbers of cells in a given subject may subsequently occur after cell lysis or apoptosis of the neoplasia, tumor or cancer, or metastasis. Reduction of LDL or oxLDL levels may take several days, weeks or even months following treatment.

Additional benefits include reducing LDL or oxLDL, reducing or reversing narrowing of arteries or veins in a subject. Improvement in lipid profiles and increasing HDL levels are also nonlimiting examples of treatment benefits.

Additional adverse symptoms and complications associated with neoplasia, tumor, cancer and metastasis that can be inhibited, reduced, decreased, delayed or prevented include, for example, nausea, lack of appetite, lethargy, pain and discomfort. Thus, a partial or complete decrease or reduction in the severity, duration or frequency of an adverse symptom or complication associated with or caused by a cellular hyperproliferative disorder, an improvement in the subjects well being, such as increased energy, appetite, psychological well being, are all particular non-limiting examples of therapeutic benefit. A therapeutic benefit or improvement therefore can also include a subjective improvement in the quality of life of a treated subject.

In various embodiments, a method reduces or decreases neoplasia, tumor or cancer, or or metastasis volume, inhibits or prevents an increase in neoplasia, tumor or cancer volume, inhibits or delays neoplasia, tumor or cancer progression or worsening, stimulates neoplasia, tumor or cancer, or metastasis cell lysis or apoptosis, or inhibits, reduces, decreases or delays neoplasia, tumor or cancer proliferation or metastasis. In an additional embodiment, a method prolongs or extends lifespan of the subject. In a further embodiment, a method improves the quality of life of the subject.

Examination of a biopsied sample containing a neoplasia, tumor or cancer, or metastasis (*e.g*., blood or tissue sample), can establish neoplastic, tumor or cancer cell volume or cell numbers, and therefore whether a reduction or stabilization in mass or numbers of neoplastic, tumor or cancer cells or inhibition of neoplasia, tumor or cancer cell proliferation, growth or survival (apoptosis) has occurred. For a solid neoplasia, tumor or cancer, invasive and non-invasive imaging methods can ascertain neoplasia, tumor or cancer size or volume. Examination of blood or serum, for example, for populations, numbers and types of cells (*e.g*., hematopoetic cellular hyperproliferative disorders) can establish whether a reduction or stabilization in mass or numbers of neoplastic, tumor or cancer cells or inhibition of neoplastic, tumor or cancer proliferation, growth or survival (apoptosis) has occurred.

Invention compositions and methods can be combined with any other treatment or therapy that provides a desired effect. In particular, treatments and therapies that have been characterized as having an anti-cell proliferative activity or function are applicable. Exemplary treatments and therapies include anti-cell proliferative or immune enhancing agents or drugs. In the case of LDL or oxLDL levels, additional treatments and therapies include LDL or oxLDL lowering agents and drugs, such as statins. The treatments and therapies can be performed prior to, substantially contemporaneously with any other methods of the invention, for example, an anti-cell proliferative cellular hyperproliferative disorder (*e.g*., a neoplasia, tumor or cancer, or metastasis) or LDL reducing treatment or therapy.

The invention therefore provides combination methods in which the methods of the invention are used in a combination with any therapeutic regimen, treatment protocol or composition, such as an anti-cell proliferative or LDL reducing protocol, agent or drug set forth herein or known in the art. In one embodiment, a method includes administering SAM-6/R glycoprotein antibody or an inhibitory nucleic acid and an anti-cell proliferative or immune enhancing treatment, agent or drug. The anti-cell proliferative or immune enhancing treatment, agent or drug can be administered prior to, substantially contemporaneously with or following administration of SAM-6/R glycoprotein antibody or an inhibitory nucleic acid. In another embodiment, a method includes administering an LDL or oxLDL reducing treatment, agent or drug.

As used herein, an "anti-cell proliferative," "anti-neoplastic," "anti-tumor," or "anti-cancer" treatment, therapy, activity or effect means any therapy, treatment regimen, agent, drug, protocol or process that is useful in treating pathologies, adverse symptoms or complications associated with or caused by abnormal or undesirable cell proliferation (cell hyperproliferation), a cellular hyperproliferative disorder, neoplasia, tumor or cancer, or metastasis. Particular therapies, treatment regimens, agents, drugs, protocol or processes can inhibit, decrease, slow, reduce, delay, or prevent cell proliferation, cell growth, cellular hyperproliferation, neoplastic, tumor, or cancer (malignant) growth, proliferation, survival or metastasis. Such treatments, therapies, regimens, protocols, agents and drugs, can operate by disrupting, reducing, inhibiting or delaying cell cycle progression or cell proliferation or growth; increasing, stimulating or enhancing cell apoptosis, lysis or death; inhibiting nucleic acid or protein synthesis or metabolism; reducing, decreasing, inhibiting or delaying cell division; or decreasing, reducing or inhibiting cell survival, or production or utilization of a cell survival factor, growth factor or signaling pathway (extracellular or intracellular).

Examples of anti-cell proliferative treatments and therapies include chemotherapy, immunotherapy, radiotherapy (ionizing or chemical), local or regional thermal (hyperthermia) therapy and surgical resection.

Specific non-limiting classes of anti-cell proliferative agents and drugs include alkylating agents, anti-metabolites, plant extracts, plant alkaloids, nitrosoureas, hormones (steroids), nucleoside and nucleotide analogues. Specific non-limiting examples of microbial toxins include bacterial cholera toxin, pertussis toxin, anthrax toxin, diphtheria toxin, and plant toxin ricin. Specific examples of drugs include cyclophosphamide, azathioprine, cyclosporin A, melphalan, chlorambucil, mechlorethamine, busulphan, methotrexate, 6-mercaptopurine, thioguanine, 5-fluorouracil, 5-fluorouridine, cytosine arabinoside, AZT, 5-azacytidine (5-AZC) and 5-azacytidine related compounds, bleomycin, actinomycin D, mithramycin, mitomycin C, carmustine, calicheamicin, lomustine, semustine, streptozotocin, teniposide, etoposide, hydroxyurea, cisplatin, carboplatin, levamisole, mitotane, procarbazine, dacarbazine, taxol, vinblastine, vincristine, vindesine, doxorubicin, daunomycin and dibromomannitol. Specific non-limiting examples of hormones include prednisone, prednisolone, diethylstilbesterol, flutamide, leuprolide, and gonatrophin releasing hormone antagonists.

Radiotherapy includes internal or external delivery to a subject. For example, alpha, beta, gamma and X-rays can administered to the subject externally without the subject internalizing or otherwise physically contacting the radioisotope. Specific examples of X-ray dosages range from daily doses of 50 to 200 roentgens for prolonged periods of time (3 to 5/week), to single doses of 2000 to 6000 roentgens. Dosages vary widely, and depend on duration of exposure, the half-life of the isotope, the type of radiation emitted, the cell type and location treated and the progressive stage of the disease. Specific non-limiting examples of radionuclides include, for example, ⁴⁷Sc⁶⁷Cu, ⁷²Se, ⁸⁸Y, ⁹⁰Sr, ⁹⁰Y, ⁹⁷Ru, ⁹⁹Tc, ¹⁰⁵Rh, ¹¹¹In, ¹²⁵I, ¹³¹I, ¹⁴⁹Tb, ¹⁵³Sm, ¹⁸⁶Re, ¹⁸⁸Re, ¹⁹⁴Os, ²⁰³Pb, ²¹¹At, ²¹²Bi, ²¹³Bi, ²¹²Pb, ²²³Ra_{,} ²²⁵Ac, ²²⁷Ac, and ²²⁸Th.

Antibodies that bind to tumor cells are a particular example of an anti-cell proliferative treatment or therapy. Anti-tumor antibodies include, for example, M195 antibody which binds to leukemia cell CD33 antigen (U.S. Patent No. 6,599,505); monoclonal antibody DS6 which binds to ovarian carcinoma CA6 tumor-associated antigen (U.S. Patent No. 6,596,503); human IBD12 monoclonal antibody which binds to epithelial cell surface H antigen (U.S. Patent No. 4,814,275); and BR96 antibody which binds to Le^{x} carbohydrate epitope expressed by colon, breast, ovary, and lung carcinomas. Additional anti-tumor antibodies that can be employed include, for example, Herceptin (anti-Her-2 neu antibody), Rituxan®, Zevalin, Bevacizumab (Avastin), Bexxar, Campath®, Oncolym, 17-1A (Edrecolomab), 3F8 (anti-neuroblastoma antibody), MDX-CTLA4, IMC-C225 (Cetuximab) and Mylotarg.

As used here, the term "immune enhancing," when used in reference to a treatment, therapy, agent or drug means that the treatment, therapy, agent or drug provides an increase, stimulation, induction or promotion of an immune response, humoral or cell-mediated. Such therapies can enhance immune response generally, or enhance immune response to a specific target, *e.g*., a cell proliferative or cellular hyperproliferative disorder such as a neoplasia, tumor or cancer, or metastasis.

Specific non-limiting examples of immune enhancing agents include antibody, cell growth factors, cell survival factors, cell differentiative factors, cytokines and chemokines. Additional examples of immune enhancing agents and treatments include immune cells such as lymphocytes, plasma cells, macrophages, dendritic cells, NK cells and B-cells that either express antibody against the cell proliferative disorder or otherwise are likely to mount an immune response against the cell proliferative disorder. Cytokines that enhance or stimulate immunogenicity include IL-2, IL-1α, IL-1β, IL-3, IL-6, IL-7, granulocyte-macrophage-colony stimulating factor (GMCSF), IFN-γ, IL-12, TNF-α, and TNFβ, which are also non-limiting examples of immune enhancing agents. Chemokines including MIP-1α, MIP-1β, RANTES, SDF-1, MCP-1, MCP-2, MCP-3, MCP-4, eotaxin, eotaxin-2, I-309/TCA3, ATAC, HCC-1, HCC-2, HCC-3, PARC, TARC, LARC/MIP-3α, CKβ, CKβ6, CKβ7, CKβ8, CKβ9, CKβ11, CKβ12, C10, IL-8, ENA-78, GROα, GROβ, GCP-2, PBP/CTAPIIIβ-TG/NAP-2, Mig, PBSF/SDF-1, and lymphotactin are further non-limiting examples of immune enhancing agents.

Methods of the invention also include, among other things, methods that result in a reduced need or use of another treatment protocol or therapeutic regimen, process or remedy. For example, for a neoplasia, tumor or cancer, or metastasis, a method of the invention has a therapeutic benefit if in a given subject it results in a less frequent or reduced dose or elimination of an anti-cell proliferative (*e.g*., anti-neoplastic, anti-tumor or anti-cancer) or immune enhancing treatment or therapy, such as a chemotherapeutic drug, radiotherapy, immunotherapy, or surgery for neoplasia, tumor or cancer, or metastasis treatment or therapy.

In accordance with the invention, methods of reducing need or use of an anti-cell proliferative (*e.g*., anti-neoplastic, anti-tumor, anti-cancer or anti-metastasis) treatment or therapy are provided. In one embodiment, a method includes administering to a subject an antibody that binds to SAM-6/R glycoprotein in an amount effective to treat a cellular hyperproliferative disorder (*e.g*., a neoplasia, tumor or cancer, or metastasis), and to reduce or eliminate need for an anti-cell proliferative (anti-neoplasia, anti-tumor or anti-cancer, or anti-metastasis) or immune-enhancing therapy. The methods can be performed prior to, substantially contemporaneously with or following administration of an anti-neoplastic, tumor, cancer or metastasis, or immune-enhancing therapy.

The doses or "amount effective" or "amount sufficient" in a method of treatment or therapy in which it is desired to achieve a therapeutic benefit or improvement includes, for example, any objective or subjective alleviation or amelioration of one, several or all pathologies, adverse symptoms or complications associated with or caused by the target (*e.g*., cellular hyperproliferative disorder), to a measurable or detectable extent, although preventing, inhibiting or delaying a progression or worsening of the target (*e.g*., cellular hyperproliferative disorder) pathology, adverse symptom or complication, is a satisfactory outcome. Thus, in the case of a cellular hyperproliferative disorder, the amount will be sufficient to provide a therapeutic benefit to a given subject or to alleviate or ameliorate a pathology, adverse symptom or complication of the disorder in a given subject. The dose may be proportionally increased or reduced as indicated by the status of treatment or therapeutic target (*e.g*., cellular hyperproliferative disorder) or any side effect(s) of the treatment or therapy.

Exemplary non-limiting amounts (doses) are in a range of about 0.1 mg/kg to about 100 mg/kg, and any numerical value or range or value within such ranges. Greater or lesser amounts (doses) can be administered, for example, 0.01-500 mg/kg, and any numerical value or range or value within such ranges. Additional exemplary non-limiting amounts (doses) range from about 0.5-50 mg/kg, 1.0-25 mg/kg, 1.0-10 mg/kg, and any numerical value or range or value within such ranges.

Methods of the invention may be practiced one or more times (*e.g*., 1-10, 1-5 or 1-3 times) per day, week, month, or year. The skilled artisan will know when it is appropriate to delay or discontinue administration. An exemplary non-limiting dosage schedule is 1-7 times per week, for 1, 23, 4, 5, 6, 7, 8, 9, 10, 15, 20 or more weeks, and any numerical value or range or value within such ranges.

Of course, as is typical for any treatment or therapy, different subjects will exhibit different responses to treatment and some may not respond or respond inadequately to a particular treatment protocol, regimen or process. Amounts effective or sufficient will therefore depend at least in part upon the disorder treated (*e.g*., cell proliferation, benign hyperplasia or a neoplasia, tumor or cancer and the type or stage, *e.g*., the tumor or cancer grade and if it is advanced, late or early stage), the therapeutic effect desired, as well as the individual subject (*e.g*., the bioavailability within the subject, gender, age, etc.) and the subject's response to the treatment based upon genetic and epigenetic variability (*e.g*., pharmacogenomics).

Cell toxicity and viability (cell apoptosis, lysis, growth proliferation, etc.) can be measured in a variety of ways on the basis of colorimetric, luminescent, radiometric, or fluorometric assays known in the art. Colorimetric techniques for determining cell viability include, for example, Trypan Blue exclusion (see, for example, Examples 1 and 2). In brief, cells are stained with Trypan Blue and counted using a hemocytometer. Viable cells exclude the dye whereas dead and dying cells take up the blue dye and are easily distinguished under a light microscope. Neutral Red is adsorbed by viable cells and concentrates in cell lysosomes; viable cells can be determined with a light microscope by quantitating numbers of Neutral Red stained cells.

Fluorometric techniques for determining cell viability include, for example, propidium iodide, a fluorescent DNA intercalating agent. Propidium iodide is excluded from viable cells but stains the nucleus of dead cells. Flow cytometry of propidium iodide labeled cells can then be used to quantitate viable and dead cells. Release of lactate dehydrogenase (LDH) indicates structural damage and death of cells, and can be measured by a spectrophotometric enzyme assay. Bromodeoxyuridine (BrdU) is incorporated into newly synthesized DNA and can be detected with a fluorochrome-labeled antibody. The fluorescent dye Hoechst 33258 labels DNA and can be used to quantitate proliferation of cells (*e.g.*, flow cytometry). Quantitative incorporation of the fluorescent dye carboxyfluorescein diacetate succinimidyl ester (CFSE or CFDA-SE) can provide cell division analysis (*e.g*., flow cytometry). This technique can be used either in vitro or *in vivo.* 7-aminoactinomycin D (7-AAD) is a fluorescent intercalator that undergoes a spectral shift upon association with DNA, and can provide cell division analysis (*e.g*., flow cytometry).

Radiometric techniques for determining cell proliferation include, for example, [³H]-Thymidine, which is incorporated into newly synthesized DNA of living cells and frequently used to determine proliferation of cells. Chromium (⁵¹Cr)-release from dead cells can be quantitated by scintillation counting in order to quantitate cell viability.

Luminescent techniques for determining cell viability include, for example, the CellTiter-Glo luminescent cell viability assay (Promega Madison WI). This technique quantifies the amount of ATP present to determine the number of viable cells.

Commercially available kits for determining cell viability and cell proliferation include, for example, Cell Proliferation Biotrak ELISA (Amersham Biosciences Piscataway, NJ); the Guava ViaCount™ Assay, which provides rapid cell counts and viability determination based on differential uptake of fluorescent reagents (Guava Technologies, Hayward, CA); the CyQUANT® Cell Proliferation Assay Kit (Molecular Probes, Inc., Eugene, OR); and the CytoLux Assay Kit (PerkinElmer Life Sciences Inc., Boston, MA). The DELFIA® Assay Kits (PerkinElmer Life Sciences Inc., Boston, MA) can determine cell proliferation and viability using a time-resolved fluorometric method. The Quantos™ Cell Proliferation Assay is a fluorescence-based assay that measures the fluorescence of a DNA-dye complex from lysed cells (Stratagene, La Jolla, CA). The CellTiter-Glo cell viability assay is a luminescent assay for measuring cell viability (Promega, Madison WI).

The terms "subject" and "patient" are used interchangeably herein and refer to animals, typically mammals, such as humans, non-human primates (gorilla, chimpanzee, orangutan, macaque, gibbon), domestic animals (dog and cat), farm and ranch animals (horse, cow, goat, sheep, pig), laboratory and experimental animals (mouse, rat, rabbit, guinea pig). Subjects include disease model animals (*e.g*., such as mice, rats and non-human primates) for studying *in vivo* efficacy (*e.g*., a neoplasia, tumor or cancer, or metastasis animal model). Human subjects include children, for example, newborns, infants, toddlers and teens, between the ages of 1 and 5, 5 and 10 and 10 and 18 years, adults between the ages of 18 and 60 years, and the elderly, for example, between the ages of 60 and 65, 65 and 70 and 70 and 100 years.

Subjects include mammals (*e.g.*, humans) in need of treatment, that is, they have undesirable or aberrant cell proliferation (cell hyperproliferation) or a cellular hyperproliferative disorder. Subjects include those at risk of having a cellular hyperproliferative disorder (*e.g*., exhibit undesirable cell proliferation that is predisposed to become a cellular hyperproliferative disorder), a candidate subject for or a subject in need of an anti-cell proliferative or immune enhancing treatment or therapy due to a lab or clinical diagnosis warranting such treatment; subjects undergoing an anti-cell proliferative or immune enhancing therapy, and subjects having undergone an anti-cell proliferative or immune enhancing therapy and are at risk of relapse or recurrence.

At risk subjects include those with a family history, genetic predisposition, or who have suffered a previous affliction with a cell proliferative or cellular hyperproliferative disorder (*e.g*., a benign hyperplasia, neoplasia, tumor or cancer, or metastasis), and are at risk of relapse or recurrence. At risk subjects further include environmental exposure to carcinogens or mutagens, such as smokers, or those in an occupational (industrial, chemical, agricultural) setting. Such subjects at risk for developing a cell proliferative or cellular hyperproliferative disorder such as neoplasia, tumor or cancer can be identified with genetic screens for tumor associated genes, gene deletions or gene mutations. Subjects that lack *Brca1* are at risk for developing breast cancer, for example. Subjects at risk for developing colon cancer have deleted or mutated tumor suppressor genes, such as adenomatous polyposis coli (*APC*), for example. At risk subjects having particular genetic predisposition towards cell proliferative disorders are known (see, *e.g*., The Genetic Basis of Human Cancer 2nd ed. by Bert Vogelstein (Editor), Kenneth W. Kinzler (Editor) (2002) McGraw-Hill Professional; The Molecular Basis of Human Cancer. Edited by WB Coleman and GJ Tsongalis (2001) Humana Press; and The Molecular Basis of Cancer. Mendelsohn et al., WB Saunders (1995)).

At risk subjects can therefore be treated in order to inhibit or reduce the likelihood of developing a cell proliferative or cellular hyperproliferative disorder, or after having been cured of a cell proliferative disorder, suffering a relapse or recurrence of the same or a different cell proliferative or cellular hyperproliferative disorder. The result of such treatment can be to reduce the risk of developing a cell proliferative or cellular hyperproliferative disorder, or to prevent a cell proliferative or cellular hyperproliferative disorder, or a pathology, adverse symptom or complication thereof in the treated at risk subject.

Described herein are kits, including proteins (*e.g*., antibodies), nucleic acids, agents, drugs and pharmaceutical formulations, packaged into suitable packaging material, optionally in combination with instructions for using the kit components, *e.g*., instructions for performing a method of the invention. In one embodiment, a kit includes an antibody that binds to SAM-6/R glycoprotein and instructions for detecting SAM-6/R glycoprotein. In another embodiment, a kit includes an antibody that binds to SAM-6/R glycoprotein or an inhibitory nucleic acid and instructions for treating a condition treatable with an antibody or an inhibitory nucleic acid that binds to SAM-6/R glycoprotein or a SAM-6/R glycoprotein nucleic acid. In one aspect, the instructions are for treating undesirable cell proliferation or hyperproliferation, or a cellular hyperproliferative disorder. In another aspect, the instructions are for treating a neoplasia, tumor or cancer, or metastasis. In a further embodiment, a kit includes an antibody that binds to SAM-6/R glycoprotein, instructions for treating undesirable cell proliferation or hyperproliferation, or a cellular hyperproliferative disorder, and an anti-cell proliferative or immune enhancing treatment, agent or drug. In various aspects, a kit includes an anti-neoplastic, anti-cancer or anti-tumor agent. In still a further aspects, a kit includes an article of manufacture, for example, an article of manufacture for delivering the antibody or nucleic acid, anti-cell proliferative or immune enhancing treatment, agent or drug into a subject locally, regionally or systemically.

The term "packaging material" refers to a physical structure housing the components of the kit. The packaging material can maintain the components sterilely, and can be made of material commonly used for such purposes (*e.g*., paper, corrugated fiber, glass, plastic, foil, ampules, etc.). The label or packaging insert can include appropriate written instructions, for example, practicing a method of the invention, *e.g*., treating a cell proliferative or cellular hyperproliferative disorder, an assay for screening for, detecting or identifying SAM-6/R glycoprotein or a SAM-6/R glycoprotein nucleic acid, etc. Thus, in additional embodiments, a kit includes a label or packaging insert including instructions for practicing a method of the invention in solution, in vitro, *in vivo,* or *ex vivo.*

Instructions can therefore include instructions for practicing any of the methods of the invention described herein. For example, invention pharmaceutical compositions can be included in a container, pack, or dispenser together with instructions for administration to a subject to treat a cell proliferative or cellular hyperproliferative disorder, such as a neoplasia, tumor or cancer, or metastasis. Instructions may additionally include indications of a satisfactory clinical endpoint or any adverse symptoms or complications that may occur, storage information, expiration date, or any information required by regulatory agencies such as the Food and Drug Administration for use in a human subject.

The instructions may be on "printed matter," *e.g*., on paper or cardboard within the kit, on a label affixed to the kit or packaging material, or attached to a vial or tube containing a component of the kit. Instructions may comprise voice or video tape and additionally be included on a computer readable medium, such as a disk (floppy diskette or hard disk), optical CD such as CD- or DVD-ROM/RAM, magnetic tape, electrical storage media such as RAM and ROM and hybrids of these such as magnetic/optical storage media.

Invention kits can additionally include a buffering agent, a preservative, or a protein/nucleic acid stabilizing agent. The kit can also include control components for assaying for activity, *e.g*., a control sample or a standard. Each component of the kit can be enclosed within an individual container or in a mixture and all of the various containers can be within single or multiple packages.

The proteins (*e.g*., SAM-6/R glycoprotein), antibodies (*e.g*., SAM-6/R glycoprotein antibody) nucleic acids, and other compositions and methods of the invention can be included in or employ pharmaceutical formulations. Such pharmaceutical formulations are useful for treatment of, or administration or delivery to, a subject *in vivo* or *ex vivo.*

Pharmaceutical formulations include "pharmaceutically acceptable" and "physiologically acceptable" carriers, diluents or excipients. As used herein the terms "pharmaceutically acceptable" and "physiologically acceptable" include solvents (aqueous or non-aqueous), solutions, emulsions, dispersion media, coatings, isotonic and absorption promoting or delaying agents, compatible with pharmaceutical administration. Such formulations can be contained in a liquid; emulsion, suspension, syrup or elixir, or solid form; tablet (coated or uncoated), capsule (hard or soft), powder, granule, crystal, or microbead. Supplementary compounds (*e.g*., preservatives, antibacterial, antiviral and antifungal agents) can also be incorporated into the formulations.

Pharmaceutical formulations can be made to be compatible with a particular local, regional or systemic administration or delivery route. Thus, pharmaceutical formulations include carriers, diluents, or excipients suitable for administration by particular routes. Specific non-limiting examples of routes of administration for compositions of the invention are parenteral, *e.g.*, intravenous, intrarterial, intradermal, intramuscular, subcutaneous, intra-pleural, transdermal (topical), transmucosal, intra-cranial, intra-spinal, intra-ocular, rectal, oral (alimentary), mucosal administration, and any other formulation suitable for the treatment method or administration protocol.

Solutions or suspensions used for parenteral application can include: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide.

Pharmaceutical formulations for injection include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). The carrier can be a solvent or dispersion medium containing, for example, water, ethanol, polyol (for example, glycerol, propylene glycol, and liquid polyetheylene glycol, and the like), and suitable mixtures thereof. Fluidity can be maintained, for example, by the use of a coating such as lecithin, by the maintenance of the required particle size in the case of dispersion and by the use of surfactants. Antibacterial and antifungal agents include, for example, parabens, chlorobutanol, phenol, ascorbic acid and thimerosal. Isotonic agents, for example, sugars, polyalcohols such as mannitol, sorbitol, sodium chloride can be included in the composition. Including an agent which delays absorption, for example, aluminum monostearate or gelatin can prolong absorption of injectable compositions.

Sterile injectable formulations can be prepared by incorporating the active composition in the required amount in an appropriate solvent with one or a combination of above ingredients. Generally, dispersions are prepared by incorporating the active composition into a sterile vehicle containing a basic dispersion medium and any other ingredient. In the case of sterile powders for the preparation of sterile injectable solutions, methods of preparation include, for example, vacuum drying and freeze-drying which yields a powder of the active ingredient plus any additional desired ingredient from a previously prepared solution thereof.

For transmucosal or transdermal administration, penetrants appropriate to the barrier to be permeated are used in the formulation. Such penetrants are known in the art, and include, for example, for transmucosal administration, detergents, bile salts, and fusidic acid derivatives. Transmucosal administration can be accomplished through the use of nasal sprays, inhalation devices (e.g., aspirators) or suppositories. For transdermal administration, the active compounds are formulated into ointments, salves, gels, creams or patches.

The pharmaceutical formulations can be prepared with carriers that protect against rapid elimination from the body, such as a controlled release formulation or a time delay material such as glyceryl monostearate or glyceryl stearate. The formulations can also be delivered using articles of manufacture such as implants and microencapsulated delivery systems to achieve local, regional or systemic delivery or controlled or sustained release.

Biodegradable, biocompatible polymers can be used, such as ethylene vinyl acetate, polyanhydrides, polyglycolic acid, collagen, polyorthoesters, and polylactic acid. Methods for preparation of such formulations are known to those skilled in the art. The materials can also be obtained commercially from Alza Corporation (Palo Alto, CA). Liposomal suspensions (including liposomes targeted to cells or tissues using antibodies or viral coat proteins) can also be used as pharmaceutically acceptable carriers. These can be prepared according to known methods, for example, as described in U.S. Patent No. 4,522,811.

Additional pharmaceutical formulations appropriate for administration are known in the art (see, *e.g*., Gennaro (ed.), Remington: The Science and Practice of Pharmacy, 20th ed., Lippincott, Williams & Wilkins (2000); Ansel et al., Pharmaceutical Dosage Forms and Drug Delivery Systems, 7th ed., Lippincott Williams & Wilkins Publishers (1999); Kibbe (ed.), Handbook of Pharmaceutical Excipients American Pharmaceutical Association, 3rd ed. (2000); and Remington's Pharmaceutical Principles of Solid Dosage Forms, Technonic Publishing Co., Inc., Lancaster, Pa., (1993)).

The compositions used in accordance with the invention, including proteins (antibodies), nucleic acid (inhibitory), treatments, therapies, agents, drugs and pharmaceutical formulations can be packaged in dosage unit form for ease of administration and uniformity of dosage. "Dosage unit form" as used herein refers to physically discrete units suited as unitary dosages treatment; each unit contains a quantity of the composition in association with the carrier, excipient, diluent, or vehicle calculated to produce the desired treatment or therapeutic (*e.g*., beneficial) effect. The unit dosage forms will depend on a variety of factors including, but not necessarily limited to, the particular composition employed, the effect to be achieved, and the pharmacodynamics and pharmacogenomics of the subject to be treated.

Described herein are cell-free (*e.g*., in solution, in solid phase) and cell-based (*e.g*., *in vitro* or *in vivo*) methods of screening, detecting and identifying SAM-6/R glycoprotein. The methods can be performed in solution, *in vitro* using a biological material or sample, and *in vivo,* for example, using neoplastic, tumor or cancer, or metastasis cells, tissue or organ (*e.g*., a biopsy) from an animal.

There are described methods of identifying, detecting or screening for SAM-6/R glycoprotein, and methods for identifying, detecting or screening for a nucleic acid or portion thereof encoding a SAM-6/R glycoprotein sequence. In one embodiment, a method includes contacting a biological material or sample with an antibody that binds to SAM-6/R glycoprotein under conditions allowing binding of the antibody to SAM-6/R glycoprotein; and assaying for binding of the antibody to SAM-6/R glycoprotein. The binding of the antibody SAM-6/R glycoprotein detects the presence of SAM-6/R glycoprotein. In another embodiment, a method includes contacting a biological material or sample with a polynucleotide that hybridizes to a nucleic acid or portion thereof encoding a SAM-6/R glycoprotein sequence under conditions allowing binding of the polynucleotide to the nucleic acid; and assaying for binding of the antibody to SAM-6/R glycoprotein. The binding of the polynucleotide to the nucleic acid detects the presence of SAM-6/R glycoprotein. In one aspect, the SAM-6/R glycoprotein is present on a cell or tissue. In another aspect, the biological material or sample is obtained from a mammalian subject. In a further aspect, the antibody that binds to SAM-6/R glycoprotein is distinct from SAM-6 antibody.

Further described herein are cell-free (*e.g*., in solution, in solid phase) and cell-based (*e.g*., *in vitro* or *in vivo*) methods of diagnosing a subject having or at increased risk of having undesirable or aberrant cell proliferation or a cellular hyperproliferative disorder (*e.g*., neoplasia, tumor or cancer, or metastasis). The methods can be performed in solution, *in vitro* using a biological material or sample, for example, a biopsy of suspicious cells that may comprise or be indicative of neoplastic, tumor or cancer, or metastasis cells, tissue or organ. The methods can also be preformed *in vivo,* for example, in an animal.

Also described herein are provided methods of diagnosing a subject having or at increased risk of having undesirable or aberrant cell proliferation or a cellular hyperproliferative disorder (*e.g*., neoplasia, tumor or cancer, or metastasis). In one embodiment, a method includes providing a biological material or sample from a subject, contacting the biological material or sample with an antibody that binds to SAM-6/R glycoprotein under conditions allowing binding of the antibody to SAM-6/R glycoprotein; and assaying for binding of the antibody to SAM-6/R glycoprotein. The binding of the antibody to the SAM-6/R glycoprotein diagnoses the subject as having or at increased risk of having undesirable or aberrant cell proliferation or a cellular hyperproliferative disorder (*e.g*., neoplasia, tumor or cancer, or metastasis). In one aspect, the biological material or sample is obtained from a human. In another aspect, the biological material or sample comprises a biopsy (*e.g*., a biopsy of lung, pancreas, stomach, breast, esophagus, ovary or uterus).

Identifying, detecting, screening and diagnostic assays of the invention can be practiced by analysis of suspect hyperproliferating cells, for example, a cell of a cellular hyperproliferative disorder. Cells include hyperproliferating, immortalized, neoplastic, tumor and cancer cell lines and primary isolates derived from breast, lung, thyroid, head and neck, nasopharynx, nose or sinuses, brain, spine, adrenal gland, thyroid, lymph, gastrointestinal (mouth, esophagus, stomach, duodenum, ileum, jejunum (small intestine), colon, rectum), genito-urinary tract (uterus, ovary, cervix, bladder, testicle, penis, prostate), kidney, pancreas, adrenal gland, liver, bone, bone marrow, lymph, blood, muscle, skin, and the hematopoetic system, and metastasis or secondary sites.

The term "contacting," when used in reference to a composition such as a protein (*e.g*., antibody), material, sample, or treatment, means a direct or indirect interaction between the composition (*e.g*., protein such as an antibody) and the other referenced entity. A particular example of direct interaction is binding. A particular example of an indirect interaction is where the composition acts upon an intermediary molecule, which in turn acts upon the referenced entity. Thus, for example, contacting a cell (*e.g*., that comprises a cellular hyperproliferative disorder) with an antibody includes allowing the antibody to bind to the cell (*e.g*., through binding to SAM-6/R glycoprotein), or allowing the antibody to act upon an intermediary that in turn acts upon the cell.

The terms "assaying" and "measuring" and grammatical variations thereof are used interchangeably herein and refer to either qualitative or quantitative determinations, or both qualitative and quantitative determinations. When the terms are used in reference to binding, any means of assessing the relative amount, affinity or specificity of binding is contemplated, including the various methods set forth herein and known in the art. For example, antibody binding can be assayed or measured by an ELISA assay.

Unless otherwise defined, all technical and scientific terms used herein have the same meaning as commonly understood by one of ordinary skill in the art to which this invention relates. Although methods and materials similar or equivalent to those described herein can be used in the practice or testing of the invention, suitable methods and materials are described herein.

As used herein, singular forms "a", "and," and "the" include plural referents unless the context clearly indicates otherwise. Thus, for example, reference to "a glycoprotein" or "antibody" includes a plurality of glycoproteins or antibodies and reference to "a treatment or therapy" can include multiple or sequential treatments or therapies, and so forth.

As used herein, all numerical values or numerical ranges include whole integers within or encompassing such ranges and fractions of the values or the integers within or encompassing ranges unless the context clearly indicates otherwise. Thus, for example, reference to a range of 90-100%, includes 91%, 92%, 93%, 94%, 95%, 95%, 97%, etc., as well as 91.1%, 91.2%, 91.3%, 91.4%, 91.5%, etc., 92.1%, 92.2%, 92.3%, 92.4%, 92.5%, etc., and so forth. For example, reference to a range of 1-5,000 fold includes 1, 2, 3, 4, 5, 6, 7, 8, 9, 10, 11, 12, 13, 14, 15, 16, 17, 18, 19, 20, fold, etc., as well as 1.1, 1.2, 1.3, 1.4, 1.5, fold, etc., 2.1, 2.2, 2.3, 2.4, 2.5, fold, etc., and so forth.

The following is a table of carbohydrate structures and abbreviations for those structures.

**Table 1**

| **Carbohydrate structure** | **Abbreviation** |
|---|---|
| GalNAcβ1-4GlcNAcβ- | Lac-di-Nac |
| GlcNAcβ1-3Galβ- | GlcNacβ3Gal |
| GlcNAcβ1-6(GlcNAcβ1-3)Galβ1-4GlcNAcβ- | Tk |
| GalNAcβ1-4Galβ1-4Glcβ- | GA1 |
| GalNAcα1-3(Fucα1-2)Galβ1-4GlcNAcβ- | A type 2 |
| Galα1-3(Fucα1-2)GalB1-4GlcNAcβ- | B type 2 |
| Galα1-3Galβ1-4Glcβ- | Gala1-3'Lac |
| GlcNAcβ1-2Galβ1-3GalNAcα- | GlcNAcβ1-2'TF |
| Galα1-4GlcNAcβ- | Galα4GlcNAc |
| Neu5Acβ- | β-N-acetylneuraminic acid |
| Glcα1-4Glcβ- | maltose |
| Glcα- | α-D-glucose |
| Glcβ- | β-D-glucose |
| Galα- | α-D-galactose |
| Galβ- | β-D-galactose |
| α-D-Man- | α-D-mannose |
| 6-H₂PO₃Manα- | α-D-mannose-6-phosphate |
| α-L-Fuc- | α-L-fucose |
| β-D-GlcNAc- | β-N-acetyl-D-glucosamine |
| α-D-GalNAc- | α-N-acetyl-D-glalactosamine (Tn) |
| β-D-GalNAc- | β-N-acetyl-D-glalactosamine |
| Manα1-3(Manα1-6)Manα- | Man₃ |
| 3-O-su-Galβ- | β-D-galactose-3-sulfate |
| Neu5Acα- | α-N-acetylneuraminic acid |
| Neu5Acα2-3Galα1-4GlcNAcα- | 3'SLN |
| Galα1-4Galβ1-4Glcβ- | Pₖ, Gb₃ |
| Galα1-3GalNAcβ- | Tαβ |
| Galβ1-3Galβ- | Galβ3Gal |
| Galβ1-3(Fucα1-4)GlcNAcβ- | Le^{a} |
| Fucα1-2Galβ1-3(Fucα1-4)GlcNAcβ- | Le^{b} |
| Fucα1-2Galβ1-3GlcNAcβ- | Le^{d} (H type 1) |
| Galβ1-3GlcNAcβ- | Le^{c} |
| Galβ1-4(Fucα1-3)GlcNAcβ- | Le^{x} |
| Fucα1-2Galβ1-4(Fucα1-3)GlcNAcβ- | Le^{y} |
| Galβ1-4Glcβ- | Lac |
| Galβ1-4GlcNAcβ- | LacNAc |
| Galβ1-3GalNAcα- | TF |
| Fucα1-3GlcNAcβ- | Fucα3GlcNAc |
| Fucα1-4GlcNAcβ- | Fucα4GlcNAc, Le |
| GalNAcα1-3GalNAcβ- | Fs-2 |
| GalNAcα1-3GalNAcα- | core 5 |
| Galα1-3GalNAcα- | Tαα |
| Neu5Acα2-3Galβ1-3GlcNAcβ- | 3'-SiaLe^{c} |
| Galα1-2Galβ- | Gala2Gal |
| Galβ1-3GalNAcβ- | Tββ |
| GlcNAcβ1-4GlcNAcβ- | (GlcNAc)₂ |
| Neu5Acα2-6GalNAcα- | sTn |
| Fucα1-2Galβ1-3GalNAca- | H type 3 |
| Neu5Acα2-3Galα1-4Glcα- | 3'-SL |
| Neu5Aca2-3Galβ1-3(Fucα1-4)GlcNAcβ- | sLe^{a} |
| Neu5Aca2-3Galb1-4(Fuca1-3)GlcNAcb- | sLe^{x} |
| Neu5Acα2-6Galα1-4Glcα- | 6'-SL |
| 6-O-su-GlcNAcβ- | β-N-acetyl-D-glucosamine-6-sulfate |
| O-su-3Galβ1-3(Fucα1-4)GlcNAcβ- | 3'-O-su-Le^{a} |
| O-su-3Galβ1-4(Fucα1-3)GlcNAcβ- | 3'-O-su-Le^{x} |
| 3'-O-su-LacNAcβ- | 3'-su-LacNAc |
| 3'-O-su-Galβ1-3GlcNAcβ- | 3'-su-Le^{c} |
| Galα1-6Glcβ- | melibiose |
| Galα1-3Galβ1-4GlcNAcβ- | Galα1-3'LacNAc |
| GlcNAcα1-3Galβ1-3GalNAcα- | GlcNAcα1-3'TF |
| Neu5Acα2-8Neu5Acα2 | (Sia)₂ |
| Neu5Acα2-8Neu5Acα2-8Neu5Acα2 | (Sia)₃ |
| GlcNAcβ1-3Galβ1-3GalNAcα- | GlcNAcβ1-3'TF |
| Galβ1-2Galβ- | Gal2βGal |
| Galβ1-4(6-O-su)GlcNAcβ- | 6-O-su-LacNAc |
| Galβ1-3(GlcNAcβ1-6)GalNAcα- | core 2 |
| Fucα1-2Galβ1-3GalNAcβ- | H type 4 |
| Galβ1-3GlcNAcβ1-3Galβ1-4GlcNAcβ- | LNT |
| Galβ1-4GlcNAcβ1-3Galβ1-4GlcNAcβ- | LNnT |
| Neu5Acα2-3Galβ- | GM4 |
| Neu5Acα2-6Galβ- | Neu5Ac6Gal |
| GalNAcα1-3(Fucα1-2)Galβ- | Aₜᵣᵢ |
| Galα1-3(Fucα1-2)Galβ- | Bₜᵣᵢ |
| GalNAcα1-3Galβ- | A_{di} |
| Galα1-3Galβ- | B_{di} |
| Fuca1-2Galβ1-4GlcNAcβ- | H type 2 |
| 6'-su-LacNAcβ- | 6'-O-su-LacNAc |
| Fucα1-2Galβ- | H_{di} |
| 3'-O-su-Galβ1-3GalNAcα- | 3'-O-su-TF |
| GlcNAcβ1-3Galβ1-4GlcNAcβ | GlcNAcβ1-3'LacNAc |
| GalNAcβ1-3GalNAcβ- | di-GalNAcβ |
| Neu5Acα2-3Galβ1-3GalNAcα- | 3'-SiaTF |
| GlcNAcβ1-3GalNAcα- | core 3 |
| GlcNAcβ1-6GalNAcα- | core 6 |
| GlcNAcβ1-3(GlcNAcβ1-6)GalNAcα- | core 4 |
| Neu5Acα2-6(Neu5Acα2-3Galβ1-3)GalNAcα- | 3,6-SiaTF |
| Galβ1-3(NeuAcα2-6)GalNAcα- | 6-SiaTF |
| Neu5Acα2-6Galβ1-4GlcNAcβ1-2Manα1-6(Neu5Acα2-6Galβ1-4GlcNAcβ1-2Manα1-6)Manβ1-4GlcNAcβ1-4GlcNAcβ- | YDS |
| Galβ1-4GlcNAcβ1-2Manα1-6(Gaβ1-4GlcNAcβ1-2Manα1-6)Manβ1-4GlcNAcβ1-4GlcNAcβ- | 9-OS |
| GlcNAcβ1-2Manα1-6(GlcNAcβ1-2Manα1-6)Manβ1-4GlcNAcβ1-4GlcNAcβ- | 7-OS |
| Neu5Acα2-3(Neu5Acα2-6)GalNAcα- | 3,6-SiaTn |
| Neu5Acα2-3GalNAcα | 3-SiaTn |
| Neu5Acα2-6Galβ1-4GlcNAcβ- | 6'SLN |
| Gal | Galactose |
| Glc | Glucose |
| Man | Mannose |
| Neu5Ac | N-Acetylneuraminic acid |
| Fuc | Fucose |
| Lac | Lactose |
| GlcNAc | N-Acetylglucosamine |
| GalNac | N-Acetylgalactosamine |
| LacNAc | N-acetyllactosamine |

A number of embodiments of the invention have been described. Nevertheless, it will be understood that various modifications may be made without departing from the spirit and scope of the invention. Accordingly, the following examples are intended to illustrate but not limit the scope of invention described in the claims.

### Examples

### Example 1

This example describes various exemplary materials and methods.

### Cell Culture

Human pancreas carcinoma cell line BXPC-3 and human stomach adeno carcinoma cell line 23132/87 were cultured in RPMI 1640 (PAA, Vienna, Austria) supplemented with 10% heat inactivated FCS (PAA, Vienna, Austria), 2mM L-glutamine and penicillin/ streptomycin (both 1%, PAA). SAM-6 antibody producing hybridoma cells were grown in cell culture flasks (175cm²) in AIM/V serum-free medium (Invitrogen, Karlsruhe, Germany). All cells were incubated in a humidified, 7% CO₂ atmosphere at 37°C.

### SAM-6 antibody purification

For purification of SAM-6 antibody, cell culture supernatant was collected and purified via cation-exchange column (HiTrap SP FF column, Amersham Bioscience, Freiburg, Germany) using a fast liquid chromatography system (FPLC). After column equilibration with starting buffer (20mM phosphate buffer pH 5.9) cell culture supernatant (same pH) was applied to the column. Unbound proteins were eluted with starting buffer, while bound antibody was displaced by increasing salt concentration with 75% elution buffer (20mM phosphate buffer, 1M NaCl pH 8.0). Antibody containing fractions were diluted in 0.9% sodium chloride, sterile filtered and stored at-70°C until use. Purity of the antibody was determined by SDS gel electrophoresis and activity confirmed by immunhistochemistry and functional assays.

### Preparation of Tumor Cell Membrane Extract (for antigen purification)

For purification of SAM-6 antigen (SAM-6/R glycoprotein), membrane proteins were isolated from human stomach adeno carcinoma cell line 23132/87 using the ProteoExtract^{™} Native Membrane Protein Extraction Kit (M-PEK)(Calbiochem, Darmstadt, Germany). The whole procedure of the native 2-step extraction of integral and membrane-associated proteins was performed at 4°C or on ice. All required buffer solutions and reagents were provided in the M-PEK.
3-5 x 10⁶ tumor cells (frozen cell pellet) were washed twice with 2ml ice cold Wash buffer. After 10min centrifugation at 300 x g and 4°C, supernatant was removed without disturbing the pellet and discarded. The pellet was carefully resuspended in 2ml ice cold extraction buffer I containing 10µl protease inhibitor cocktail. After 10min incubation at 4°C under gentle agitation on a rotary shaker, insoluble material was sedimented by centrifugation at 16,000 x g and 4°C for 15min. For the purpose of control, an aliquot of the supernatant, enriched in soluble proteins, was transferred into a sample tube without disturbing the cell layer. The rest of the fraction was discarded. In a second extraction step, 1ml extraction buffer II containing 5µl protein inhibitor cocktail, was added to the pellet of step I, carefully mixed by using a pipette and incubated for 30min at 4°C under gentle agitation. Insoluble material was centrifuged for 15min at 16,000 x g and 4°C. The supernatant, now enriched in integral membranes and membrane associated proteins, was completely transferred into a new sample tube without disturbing the debris pellet. Aliquots were stored at-20°C after determination of the protein content by BCA method using bovine serum albumin as standard.

### Western Blot Analysis

Reducing SDS-Page gels (8%) and Western blotting of membrane proteins were performed using standard protocols. In short, blotted 0.2µm nitrocellulose membranes were blocked with PBS containing 0.05% (v/v) Tween-20 and 5% (w/v) low fat milk powder, followed by incubation for 1 h with 20µg/ml SAM-6 human IgM antibody or unrelated human control IgM (Chrompure IgM, Dianova, Hamburg, Germany). The secondary antibody (peroxidase-coupled rabbit anti-human IgM antibody 1:1,000; Dianova) was detected with the SuperSignal chemiluminescence kit from Pierce (Perbio Science Deutschland GmbH, Bonn, Germany).

### Purification of SAM-6 Receptor

Membrane fraction obtained from tumor cell line 23132/87 after extraction with M-PEK was used to purify the antigen of SAM-6 antibody. For the first of step of purification size exclusion chromatography was used. 10ml M-PEK extract (1mg/ml) was injected to a Superdex 200 column (XK16/60; Amersham Biosciences, Uppsala, Sweden) at a flow rate of 1ml/min using a super loop system and a fast protein liquid chromatography unit (FPLC) (Amersham Pharmacia Biotech, Freiburg, Germany). The column was previously equilibrated with buffer A (100mM Tris/HCl pH 7.5, 40mM NaCl, 2mM EDTA, 1% TritonX-100) and eluted with the same buffer at a flow rate of 2ml/min. Fractions of 2ml each were collected and fractions corresponding to the peak of SAM-6 receptor activity, were combined and applied to an equilibrated anion-exchange column (HiTrap^{™} Sepharose Q XL, 5ml; Amersham Biosciences, Uppsala, Sweden). Unbound components were eluted with buffer A, whereas bound protein were released by increasing salt concentration via linear step gradient using buffer B (100mM Tris/HCl pH 7.5, 1 M NaCl, 2mM EDTA, 1% TritonX-100).
Again a flow rate of 2m1/min was used and fractions of 2ml were collected and monitored at 280nm. After concentrating the eluate via acetone precipitation at -20°C over night, protein pellet was dissolved in 1x SDS buffer and examined by SDS-Page and Western Blot analysis for reaction with SAM-6 antibody. Positive bands were excised from Coomassie stained gel and sequenced.

### Protein Identification by Peptide Mass Mapping

Protein Sequencing was performed by TopLab (Martinsried, Germany). After one-dimensional SDS-Page with Coomassie staining a protein band having an estimated molecular mass of 80kDa was excised and after reduction and alkylation with iodoacetamide, the band was in-gel digested with trypsin. The pool of tryptic peptides was desalted via ZipTipC18 and analyzed by Matrix assisted Laser Desorption Ionization (MALDI) Mass Spectrometry (Voyager-DE STR; Applied Biosystems, CA, USA) followed by databank research (Profound and Mascot versus NCBI). Hits for the best matching protein candidates (probability of 1.00) were compared using the Basic Local Alignment Search Tool.

### Transfection studies with small interfering RNA of Grp78

Cell line BXPC-3 was used for SAM-6 receptor binding studies after transfection with small interfering RNA (siRNA). SiGENOME SMARTpool reagent against human HSPA5, pre-designed and validated siRNA for silencing the target molecule Grp78, was purchased from Dharmacon (Lafayette, CO, USA). Silencer^{™} Negative control, as control for non-specific effects on gene expression caused by siRNA introduction, was purchased from Ambion (Cambridge, UK).
As further controls served mock transfected (without siRNA) and untreated cells (grown in complete RPMI-1640, not transfected). For transfection of siRNA siLentFect^{™} Lipid (BioRad Labratories, CA, USA) was used. Additionally, to control transfection of siRNA into the cells, Silencer CY3 GAPDH siRNA (Ambion, Cambridge, UK) was transfected and verified using confocal microscopy.
The day before transfection 24-well plates were seeded with 1 x 10⁴ cells in 1ml complete growth medium per well (50% confluent the following day). Plates were incubated over night at 37°C and 7% CO₂ 30 min prior transfection, the medium was carefully replaced by 0.4ml fresh and complete medium per well. For each well 100µl transfection mixture was prepared and added to the cells. The mixture consisted of 49µl serum-free OptiMEM (Invitrogen, Karlsruhe, Germany), 1µl siLentFect^{™} Lipid combined with 50µl siRNA after incubation for 20 min at room temperature. Final concentration of SiGENOME SMARTpool was 100nM and 25nM of control siRNAs. Transfected cells were incubated over a period of 24, 48 and 72 h at 37°C and 7% CO₂ atmosphere. Knockdown of Grp78 after the indicated time was monitored by FACS analysis.

### Detection of Grp78 protein knockdown by FACS analysis

Tumor cell line BXPC-3 was transfected with siGENOME siRNA against human GRP78. Cells were harvested after 1 and 3 days by gentle detaching with Trypsin/ EDTA (PAA, Vienna, Austria). After 1 and 3 days protein levels of GRP78 were monitored by FACS analysis. Respectively 2 x 10⁵ cells were incubated on ice with SAM-6 antibody (100 µg/ml), anti-GRP78 antibody (100 µg/ml; clone ET-21, Sigma, Taufkirchen, Germany) or anti-CD55 antibody (1:50; clone 143-30, DPC Biermann, Bad Naunheim, Germany) for 30 minutes. Unrelated human IgM (Chrompure IgM, Dianova, Hamburg, Germany) and rabbit/ mouse IgG (Sigma, Taufkirchen, Germany) served as negative controls. Incubation with FITC-labeled secondary antibodies (rabbit antihuman IgM antibody, Dako, Hamburg, Germany; goat anti-rabbit IgG or goat anti-mouse IgG, both Acris, Hiddenhausen, Germany) for 15 minutes followed. Cells were analyzed by flow cytometry (FACScan; Becton Dickinson, San Jose, California) using WinMDI software.

### Apoptosis Assay with SAM-6 on Transfected Tumor Cells

The extent of antibody-induced apoptosis on BXPC-3 cells before and after transfection with GRP78 siRNA was detected by the Cell Death Detection ELISAPLUS (Roche, Mannheim, Germany) according to the manufacturer's protocol. 48 hours after transfection 1 x 104 cells were plated on 96-well plates and incubated in presence of 100 µg/ml SAM-6 antibody or unrelated IgM control (Chrompure human IgM, Dianova, Hamburg, Germany) for 4 h at 37 °C and 7% CO2 in a humidified atmosphere. To demonstrate normal growth, cells were supplemented with complete growth medium.

### Preparation of Tumor Cell Membrane Extracts (for glycosidase assay)

Cell line BXPC-3 was grown to 80% confluency on 100mm cell culture plates. Culture plates were washed twice with phosphate buffered saline pH 7.4 (PBS), adherent cells were harvested in PBS followed by centrifugation for 5 min at 1,500rpm. Cells were resuspended in ice cold hypotonic buffer (20mM HEPES, 3mM KCl, 3mM MgCl₂) and incubated for 15min, followed by 5 min ultrasonic vibration. Nuclei and cytoskeletons were pelleted by 10min of centrifugation at 10,000 x g. The supernatant was centrifuged for 30min at 100,000 x g in a SW28 rotor to yield the microsomal pellet, and finally carefully resuspended in modified lysis buffer (50mM Tris-HCl, pH 7.4; 150mM NaCl; 1mM EDTA; 1% Nonidet NP-40 and 0.25% sodium deoxycholate). Insoluble material was removed by centrifugation for 10min at 16,000 x g. The supernatant was transferred into a new sample tube without disturbing the debris pellet. Aliquots were stored at -20°C after determination of the protein content by BCA method using bovine serum albumin as standard. The whole procedure was performed at 4°C or on ice. Complete protease inhibitor tablets (Roche Biochemicals, Mannheim) were added to all solutions.

### Glycosidase Assay

Membrane extracts of BXPC-3 cells, prepared by differential centrifugation, were used for glycosylation studies. To cleave all types of N- and O-linked carbohydrate chains, the membrane extract was denaturated in buffer containing 1% sodiumdedecylsulfate and 1% ß-mercaptoethanol for 3 min at 95°C. The denaturated extract was diluted with reaction buffer (PBS pH 7.4, 1 % nonidet NP-40, 1% ß-mercaptoethanol) to the final protein concentration of 0.5 mg/ml. For deglycosylation of O- and N-linked carbohydrates, aliquots of 100µl were incubated either with 10U N-glycosidase F (Roche Applied Science, Mannheim, Germany) or 5mU O-glycosidase (Roche Applied Science, Mannheim, Germany) at 37°C over night. Untreated extract in reaction buffer served as control. The extent of deglycosylation was analyzed by SDS-Page and Western Blotting procedure.

### Glycosidase Assay on cytospins

4x10⁵ human pancreas carcinoma cells (BXPC-3) were washed and resuspended in 1ml Dulbecco's phosphate buffered saline pH 7.2 (Sigma, Taufkirchen, Germany). Incubation for 4 hours at 37°C with 5U/ml N-glycosidase or 20mU/ml O-glycosidase (both Roche Applied Science, Mannheim, Germany) followed. Untreated cells in phosphate buffer served as control. Corresponding aliquots of 100µl were washed and spun on coverslips at 500rpm for 2 min. Dried cytospins were fixed with acetone and stained immunohistochemically.

### Immunohistochemical staining of cytospins

Dryed cytospins were fixed with acetone (10 minutes) and blocked for 30min with low fat milk powder/ PBS (4%). After washing with Tris/NaCl, the coverslips were incubated with antibody SAM-6 (50µg/ml) or control antibodies for 30 minutes. As negative control served unrelated human IgM (Chrompure IgM, Dianova, Hamburg, Germany) in the same concentration and as positive control anti-CD55 antibody (1:30; clone 143-30, DPC Biermann, Bad Naunheim, Germany). After washing with Tris/NaCl incubation with secondary antibodies for 30minutes followed (peroxidase-labeled rabbit antihuman or rabbit antimouse conjugate 1:50). After final washing with Tris/NaCl and incubation in PBS for 10minutes, staining was performed with diaminobenzidine (0.05%)-hydrogen peroxide (0.02%) for 10min at room temperature. The reaction was stopped under running tap water and the coverslips counterstained with hematoxylin. After mounting with glycerol-gelatin, the extent of deglycosylation was visual analyzed using light microscopy.

### Labeling of SAM-6 antibody with fluorescein isothiocyanate

Endocytosis of SAM-6 antibody was determined on human pancreas carcinoma cell line BXPC-3. For immunofluorescence studies conjugation of monoclonal antibody SAM-6 and isotype control IgM (Chrompure IgM, Dianova, Hamburg, Germany) was performed with Fluoro Tag FITC Conjugation Kit (Sigma-Aldrich, Saint Louis, USA) according manufacturer's protocol.
Purified antibody SAM-6 or IgM isotype control were dissolved in sodium carbonate-bicarbonate buffer pH 9.0 at a concentration of 2.5mg/ml. Therefore, consisting buffer was exchanged with sodium carbonate-bicarbonate buffer pH 9.0 over a Sephadex^{™} G-25 column. The final concentration of antibodies was approx. 1.7 mg/ml (dilution factor 1.5). A solution of fluorescein isothiocyanate (FITC) in 2ml 0.1M carbonate-bicarbonate buffer (per FITC vial) was prepared just prior to addition to antibodies. 50µl of the FITC solution was drop-wise added to 0.2ml of each antibody solution and incubated for 2 h at room temperature in the dark with gentle stirring. Finally, a molar ratio of 20:1 of FITC (MW 389) to IgM (MW 900) was used in the reaction mixture, expected fluorescein-antibody conjugates with F/P ratio from 3 to 6. Cells were trypsinized and left on ice for 1h.
FITC labeled antibodies were separated from free FITC by gel filtration on a Sephadex^{™} G-25 column. After column equilibration with at least 30ml PBS the reaction mixture was applied to the top of the column gel bed. Column elution started with 10ml PBS, collecting 0.25ml fractions. During elution, the first of two appearing peaks contained the conjugate. Protein content was determined by BCA method using bovine serum albumin (Roth, Karlsruhe, Germany) as standard.

### SAM-6 Endocytosis

Endocytosis was determined for SAM-6 antibody on human pancreas carcinoma cell-line BXPC-3. Monoclonal antibodies SAM-6 (purified) and isotype control (ChromPure IgM, Dianova, Hamburg, Germany), were conjugated with Fluoro Tag FITC Conjuagtion Kit (Sigma-Aldrich, Saint Louis, USA) as described above. Conjugated antibodies at a final concentration of 40µg/ml were directly given to 1 x 10⁶ cells and incubated for 30, 60, 120 minutes at 37°C. Cells were harvested, rinsed and resuspended in phosphate buffered saline pH 7.4 (PBS). 100µl of each cell suspension was fixed on slides. Finally the slides were mounted with Fluorescent Mounting Medium (DakoCytomation, Carpinteria, USA) and analyzed by confocal microscopy.

### Lipid staining with Sudan III

For intracellular lipid staining pancreas carcinoma cells BXPC-3 were grown on glass slides. Adherent cells were incubated for 48h with 100µg/ml SAM-6 antibody, anti-Grp78 (cloneET-21, Sigma, Taufkirchen, Germany) or unrelated control (Chrompure IgM, Dianova, Hamburg, Germany). After two washing steps with PBS, cells were fixed for 5 minutes with 60% isopropanol. Before use, a 60% solution of a Sudan III stock (0.5% Sudan III in 100% isopropanol) was matured over night, filtered and added to the fixed cells. After 40minutes cells were washed with distilled water, differentiated in 60% isopropanol, washed again and counterstained for 6 minutes with hematoxylin. Finally cells were rinsed with water for 10minutes and mounted with glycerol gelatine. The extent of lipid inclusions was visualized using light microscopy.

### Cytochrome C Assay

For screening whether cytochrome c was released during apoptosis induced by SAM-6 the Cytochrome C ELISA Kit (Calbiochem, LaJolla, USA) was applied. In short, 1.5 x 10⁶ stomach carcinoma cells (23132/87) were incubated with 200 µg/ml purified SAM-6 or unrelated IgM antibody for one and four hours respectively. After trypsinization the cells were washed three times with cold PBS, resuspended in lysis buffer and incubated for one hour at RT with gentle mixing. After three washing steps with ice-cold PBS and centrifugation at 1,000 x g for 15 min, the supernatants were transferred into a fresh tube and diluted (1:10) with Calibrator Diluent RD5P (1x). Then a mixture (1:1) of each of the diluted samples with Calibrator Diluent RD5P (1x) was add into the micro-titer plate delivered in the kit. A incubation for two hours at RT and then a washing with 0.4 % washing buffer followed. Then the Cytochrome C Conjugate was put into each well. The incubation and washing steps were repeated in the way before. After the addition of Substrate Solution (1:1 mixture of Color Reagent A and B) to each well the plate was incubated for 30 min at RT. The measurement at 415 nm (reference wavelength 540 nm) was carried out after the addition of Stop Solution.

### Caspase Assay

For screening antibody-treated cells for Caspase-2, -3, -6, -8, and -9 activity the Apo Target^{™} Colorimetric Protease Assay Sampler Kit (Calbiochem, LaJolla, USA) was used following the suppliers manual. In short, 3x10⁶ stomach carcinoma cells (23132/87) were incubated with 200 µg/ml purified SAM-6 or unrelated IgM antibody for one and four hours respectively. After trypsinization, the cells were resuspended in cold lysis buffer. They were incubated for 10 min on ice and centrifuged for 1 min at 10,000 x g. To determine the amount of protein in the cell lysates Bradford assay was applied. Each cytosol extract was diluted to a protein concentration of 4 µg/ml. Then reaction buffer containing DTT and the various conjugated protease substrates were added to the samples in a 96-well micro-titer plate. A mixture (1:1) of lysis and reaction buffer served as a blank. After incubation for two hours at 37 °C and 7% CO₂ in a humidified CO₂ incubator the absorption and thus the extent of caspase activity was measured in an ELISA reader at 415 nm. For experiments with caspase-3-inhibitor the Caspase-3 Cellular Activity Assay Kit (Calbiochem, LaJolla, USA) was used following the suppliers manual, using similar conditions as described above.

### In vivo studies

To determine the effects of SAM-6 antibody on tumor cell growth *in vivo*, a nude mouse/human stomach carcinoma cell system was used. Briefly, 2 x 10⁶ stomach carcinoma cells (23132/87) were injected subcutaneously (s.c.) into 6-7 week old NMRI-nu/nu mice (Harlan Winkelmann GmbH, Borchen, Germany) followed by injections of antibody SAM-6 antibody when tumors reached visible size. 50, 250, 500 or 750 µg antibody was given respectively at days 9, 11, 14, 16 and 17 i.p. post carcinoma cell injection. Control mice were injected with 750 µg unrelated human IgM (Chrompure IgM, Dianova, Hamburg, Germany). Visible tumor growth was measured macroscopically during the experiment. The study was terminated when tumors had reached maximal tolerable size (day 18), whereupon the mice were sacrificed, tumor volume respectively tumor weight was determined, and organs and tissues inspected for the spread of tumors and other alterations.

### Example 2

This example includes a description of purification and analysis of SAM-6/R glycoprotein.

### Western Blot Analysis on Membrane Extracts

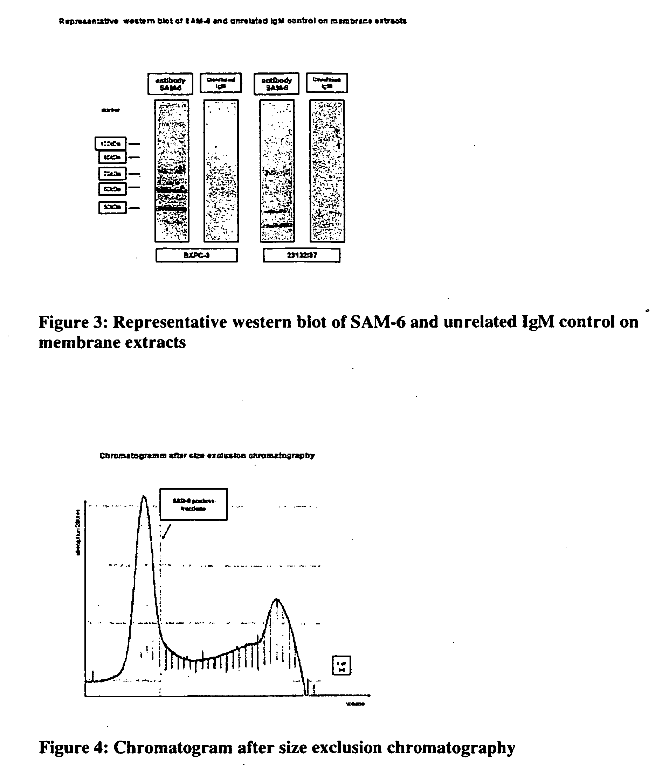

The isolation and characterization of SAM-6 antigen was performed on tumor cell line 23132/87 and BXPC-3. SAM-6 antibody bound to an antigen with a relative molecular mass of about 80kDa (Fig. 3). On both cell lines, western blot showed comparable results in the pattern of appearing bands. Also, there was no significant difference, whether we used differential centrifugation or the MPEK method to prepare the samples. Nevertheless, for purification of the antigen we employed the method of native Membrane Extraction Kit, because of the greater enrichment and fewer unspecific bands at the lower molecular weight. To find out unspecific binding of IgM antibodies, unrelated human IgM was used for control. Non specific binding could be observed at about 50kDa. Additional prominent protein bands at a molecular mass of about 60 and 100kDa shaped up as impurities from membrane fractionation during preparation.

### Purification and Identification of Human SAM-6 Receptor by Peptide Mass Mapping

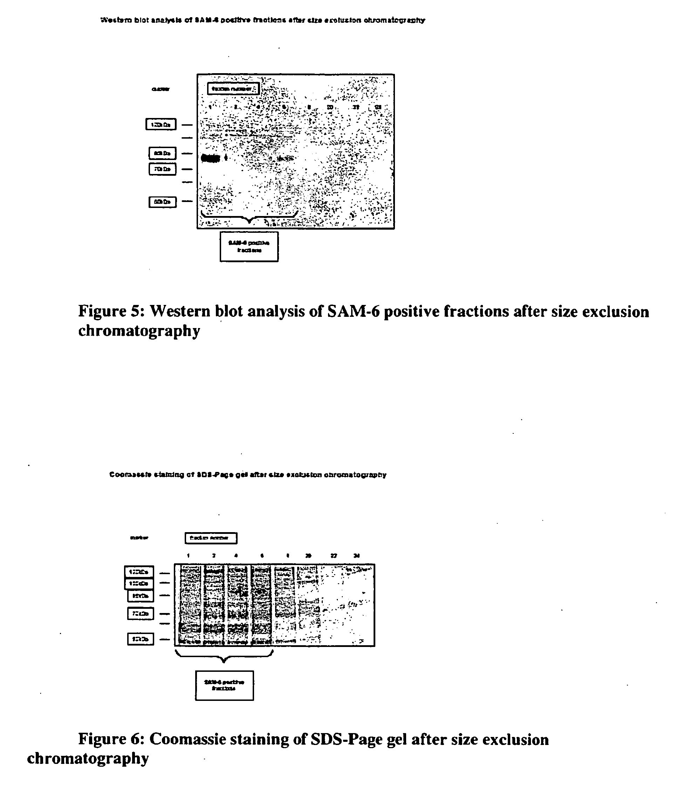

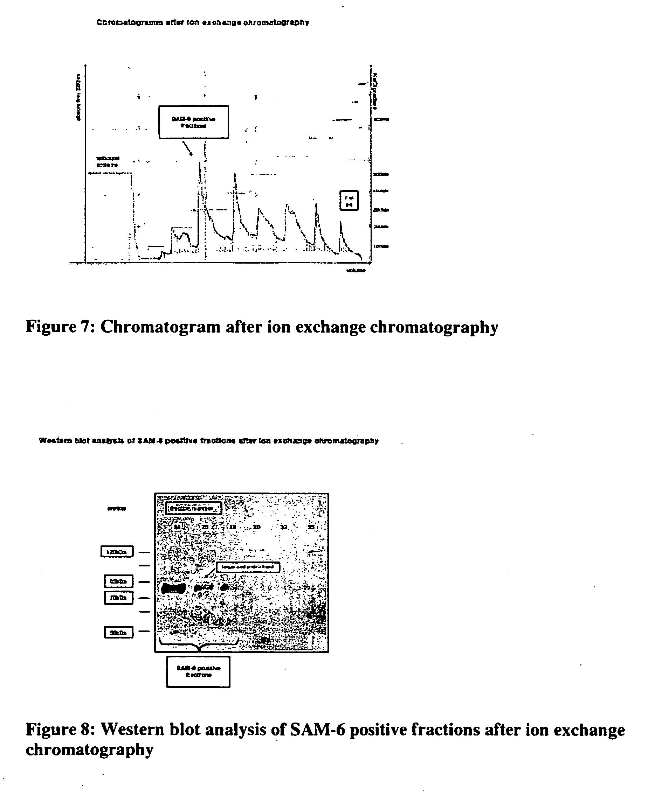

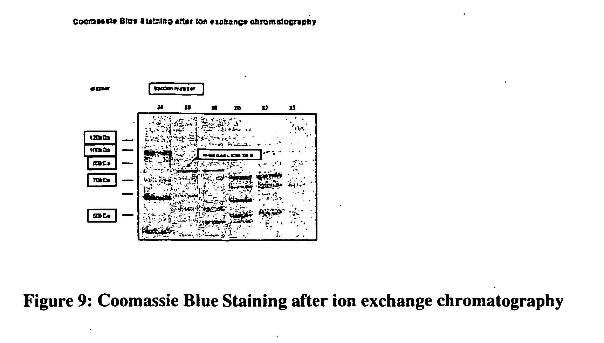

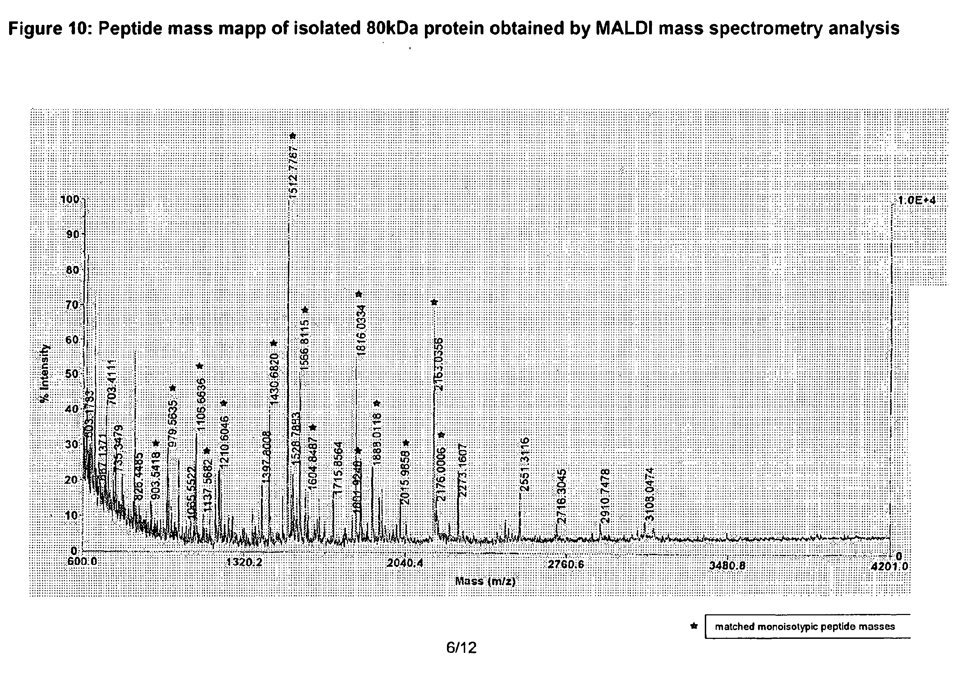

To identify the 80-kDa protein binding to SAM-6 antibody on western blot, two steps of column chromatography was used to purify and MALDI mass spectroscopy to identify the protein. Figure 4 shows a representative chromatogram after the first step (size exclusion chromatography). Fractions, containing the protein binding to SAM-6 antibody, were analyzed by SDS-PAGE, followed by western blot and Coomassie staining. Positive fractions are branded in Figures 5 and 6. The second step followed using anion exchange chromatography (chromatogram see Fig.7). Subsequent analysis by SDS-PAGE is shown in Figures 8 and 9. Arrows indicate the band representing the 80kDa protein, that finally was excised and sequenced. Protein sequencing was performed by TopLab (Martinsried, Germany).
A sequence database search with the matched set of tryptic peptide masses calculated human Grp78 (accession no. NP_005338.1) as the highest ranking candidate. A total of 21 tryptic peptide masses were assigned to Grp78 protein corresponding to an amino acid sequence coverage of minimum 35% (Figs. 10 and 11). The peptide mass error was less than 50ppm.
Because of the high number of assigned peptides and the high sequence coverage, Grp78 protein was identified from the database. Alignment of experimentally derived peptide sequences and the protein database sequence of human Grp78/ BiP is shown in Figure 11. Bold text are sequences that display the masses obtained by MALDI peptide-mass fingerprinting and are marked by stars in the peptide mass map (Fig. 10).

### Structure analysis

Structure analysis for potential trans-membrane region and glycosylation sites was performed using sequence analysis tools of the ExPASy (Expert Protein Analysis System) proteomics server of the Swiss Institute of Bioinformatics (www.expasy.org). Beside signal regions at amino-acids 1 to 17 and 650 to 654, Grp78 shows a possible transmembrane region from amino-acid 220 to 237 and potential extra-cellular O-glycosylation sites at amino-acid residues166 and 184. N-glycosylation sites could not be determined (Fig. 11).

### Example 3

This example includes a description of Grp78 expression inhibition studies with siRNA.

### Knockdown of Grp78

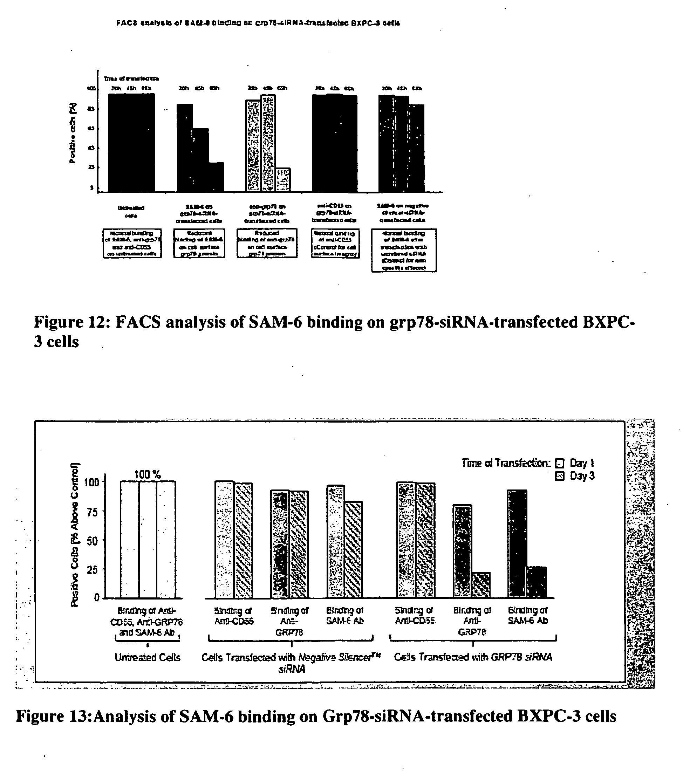

Studies were performed to investigate if silencing of Grp78 gene, coupled with knockdown of Grp78 reduced binding of SAM-6 antibody on BXPC-3 tumor cells. Transfection of tumor cell line BXPC-3 with Grp78 siRNA reduced protein expression and binding of SAM-6 antibody. On cells transfected with Negative silencer^{™} siRNA (unrelated siRNA) strong binding of SAM-6 and control antibodies is detectable during the whole incubation time, showing no significant effects of Grp78 expression. Cells transfected with GRP78 siRNA show reduced binding of anti-GRP78 (76% reduction) and SAM-6 (70% reduction). Silencing did not affect the expression of other cell surface membrane molecules. Binding of anti-CD55 after transfection was as strong as before (Figs. 12 and 13). Knockdown of surface located GRP78 reduces target protein expression and binding of SAM-6 antibody (Figs. 12 and 13).

### Apoptosis Assay

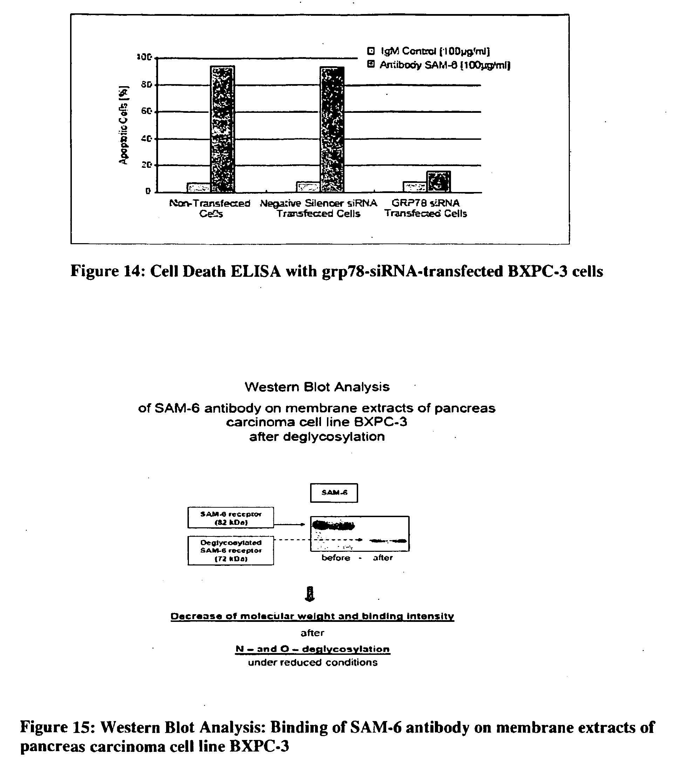

Apoptotic activity was measured by Cell Death Detection ELISA^{PLUS} (Roche) to demonstrate the extent of antibody-induced apoptosis of transfected tumor cells. Transfected cells were incubated with 100 µg/ml SAM-6 antibody for 4h. Unrelated human IgM in the same concentration was negative control. Tumor cells transfected with siRNA against human Grp78 show a clear reduction of the apoptotic activity induced by SAM-6 antibody compared to untreated cells and cells transfected with unrelated siRNA (Fig. 14). Calculation of apoptotic cell content was related to the content of cells grown in complete medium.

### Example 4

This example includes a description of studies on the effects of glycosidase treatment of SAM-6/R glycoprotein on SAM-6 antibody binding.

### Glycosidase Assay

To confirm binding of SAM-6 antibody on O-linked carbohydrates, the effect of deglycosylation of membrane extracts of tumor cell line BXPC-3 was studied. Membrane extracts were deglycosylated under reduced conditions with O- and N-glycosidase over night, afterwards separated via SDS-Page and analyzed by western blotting. After incubation with glycosidases the molecular weight of the prominent 80kDa protein clearly decreases and so does the binding activity of SAM-6 (Fig. 15). These results indicate that sugars are involved in binding of SAM-6 antibody to SAM-6/R glycoprotein.

### Glycosidase Assay on cytospins

To confirm SAM-6 antibody binding on an O-linked carbohydrate moiety on the tumor cell surface a Glycosidase Assay was done on cytospins. Human pancreas carcinoma cells (BXPC-3) were incubated with N-glycosidase or O-glycosidase. After preparation of cytospins, a staining was performed with SAM-6 antibody and anti-CD55.
Immunohistochemical staining with SAM-6 antibody shows a significant reduction of surface binding when treated with O-Glycosidase. Treatment with N-Glycosidase had no detectable effect on SAM-6 antibody binding (Fig. 16).
The cell surface molecule CD55 served as a control for membrane integrity and showed no changed binding after treatment with glycosidases.

### Example 5

This example includes a description of studies of SAM-6 antibody binding to SAM-6/R glycoprotein present on cells.

### SAM-6 Endocytosis

SAM-6 antibody binds to cell membrane SAM-6/R glycoprotein. This antibody/receptor binding initiates the accumulation of lipids and the apoptotic cascade. SAM-6 antibody also binds to oxLDL and carries this into cancer cells. Lipoproteins are normally internalized by a receptor-mediated endocytosis.
To study what happens after SAM-6 antibody binds to a cancer cell membrane, SAM-6 antibody and an isotype control were conjugated with FITC. Conjugated antibodies in the presence of LDL were directly given to human pancreas carcinoma cell-line BXPC-3 and incubated for 30, 60, 120 minutes. Cells were finally analyzed by confocal microscopy. After 30 min of incubation with SAM-6 antibody, binding to the cell surface could be observed (Fig.17A). After 60 min SAM-6 antibody is concentrated at the membrane, seen as a typical formation of "capping" (Fig. 17B). One hour later SAM-6 antibody is completely internalized into the cell (Fig. 17C). In comparison, the labeled control antibody did not show similar events (Fig. 17D, E, F). It can be assumed that oxLDL is carried into the cell together with the antibody.

### Lipid staining with Sudan III

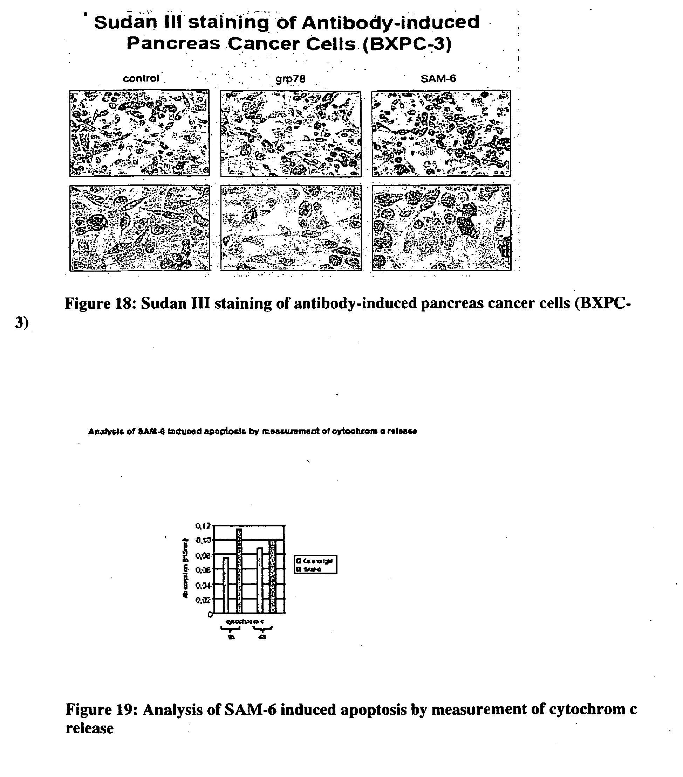

To examine SAM-6 antibody-induced intracellular lipid accumulation, we performed a staining with Sudan III. This dye is specific for the detection of neutral lipids and fatty acids. Fig. 18 shows the data obtained after 48h of incubation with SAM-6 antibody, anti-Grp78 or unrelated human control IgM on pancreas carcinoma cell-line BXPC-3. When treated with SAM-6 antibody, the tumor cells show clearly an antibody-induced accumulation of neutral lipid droplets (Fig. 18). In contrast, cells treated with anti-Grp78 and unrelated control antibody respectively show no significant accumulation of lipids. These data indicate that the intracellular accumulation of lipids is a direct effect mediated by SAM-6 antibody.

### SAM-6 apoptosis

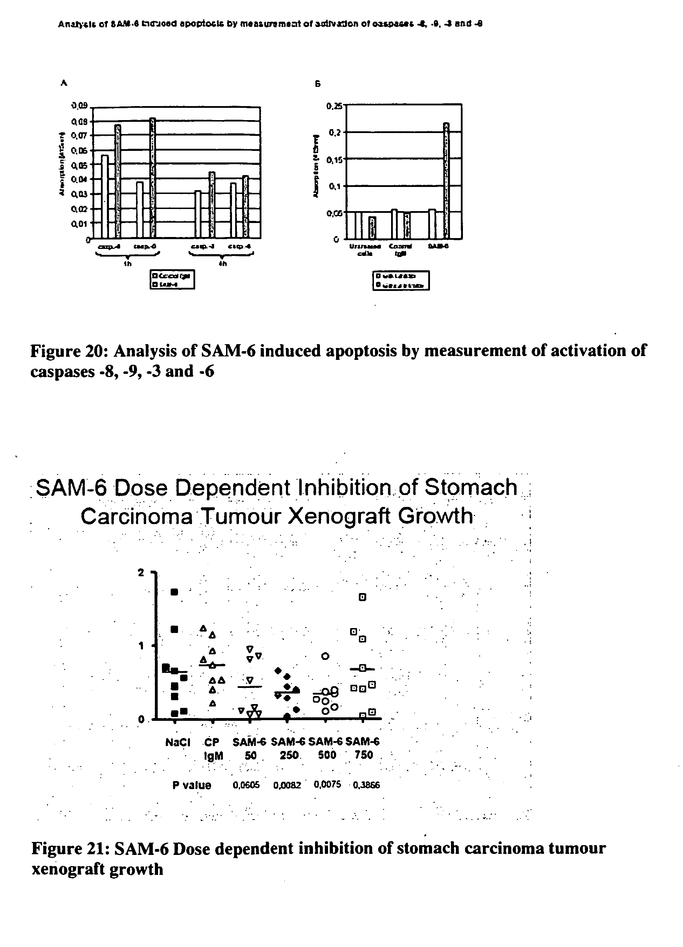

SAM-6 induced apoptosis may be a consequence of the disturbed lipid homeostasis. So far, however, nothing was known about the pathway between antibody-binding, lipid accumulation and the ultimate cell death nor whether caspases are activated. A better understanding of the signaling pathway activated by SAM-6 will make a further contribution to innovative therapies for the fight against cancer. Caspases were therefore studied for activation during the SAM-6 induced apoptotic process.
To examine whether Cytochrome C was set free in gastric cancer cells after incubation with SAM-6 antibody and control IgM respectively, the Cytochrome C ELISA Kit was applied. The sandwich enzyme immunoassay technique indicated that Cytochrome C was released at a higher level in cells treated with SAM-6 antibody than in the cells treated with the unrelated, human IgM after 1h. Additionally, the amount of the polypeptide in both samples differed more than after four hours of incubation. Also observable is a decrease in the SAM-6 sample after four hours (Fig. 19). This clearly indicates that in the SAM-6 induced pathway a perturbation of the mitochondria occurred leading to the break of the outer membrane and resulting in the Cytochrome C release. The Cytochrome C level in SAM-6 antibody treated cells came close to that of control after four hours of incubation signifying that the mitochondrial breakdown only occurred at an early stage of the pathway.
In order to determine whether caspases and which caspases are induced by SAM-6 antibody, the Apo Target^{™} Colorimetric Protease Assay Sampler Kit was used. After 1h incubation with the antibodies the activity of caspase-2, -3, -6, -8, and -9 was measured. The initiator caspases -8 and -9 but not -2 were activated in cells treated with SAM-6 antibody compared with those treated with the unrelated human IgM control already after 1h (Fig. 20A). In addition, after 4hr an activation of the effector caspases -3 and -6 could be detected.
To exclude artifacts, a study using a caspase-3 inhibitor was prepared additionally as an example. Figure 20B shows the suppressed caspase-3 activity when the inhibitor was added. In absence of the caspase-3 inhibitor a clear activation of caspase-3 could be observed in the SAM-6 treated tumor cells.

### Example 6

This example includes a description of *in vitro* studies of malignant melanoma.
To determine the effect of SAM-6 antibody on melanoma cells, sensitivity of malignant melanoma cells HTB-69 and CRL 1424 to SAM-6 antibody was determined. In brief, cells were trypsinized and diluted with RPMI containing 2%FCS to a concentration of 2x10⁵ cells/ml. A 50µl cell-suspension per well were plated onto a 96-well-plate. Antibody was diluted according to 100µg/ml with RPMI (leading to a final concentration of 1% FCS per well). IgM, RPMI and buffer were used as controls. Cells were incubated in an incubator at 37°C for 2, 5, 24 or 48 hours, supernatant was discarded, 30µl Trypsin added per well and incubated for a few minutes, and cells displaced from the bottom of the well observed with a microscope. The reaction was terminated with 6µl FCS per well, 324µl Guava ViaCount Reagent (Guava Technologies, Hayward, USA) was added per well and mixed gently and incubated for 5min. at room temperature, and the data analyzed with Guava PCA-96.

The data indicate that SAM-6 antibody was able to kill both melanoma cell types. Cell killing was greatest for HTB-69 cells incubated for 48 hours.

### Example 7

This example includes a description of *in vivo* studies.

To determine the effects of SAM-6 antibody on tumor cell growth *in vivo,* a nude mouse-human stomach carcinoma cell system was used. A concentration of 2 x 10⁶ cells derived from the human stomach carcinoma cell line 23132/87 were injected intraperitoneal (i.p.) into NMRI nu/nu mice. Nine days after the inoculation of tumor cells, different doses of SAM-6 antibody were injected i.p. Unrelated human control IgM as well as NaCl solution (0.9%) served as negative controls. The antibody was given again on days 11, 14, 16 and 17 post carcinoma cell implantation. Control mice were injected with 750 µg unrelated human IgM (Chrompure IgM, Dianova, Hamburg, Germany).
Throughout the study, tumor growth was controlled macroscopically. After 18 days the mice were sacrificed, tumor volumes were determined and Student's t test was used to compare tumor sizes between treatment and control groups. The tumors which developed during the course of the study showed a significant reduction in volume when treated with SAM-6 antibody (Fig. 21). Moreover, the reduction of tumor volume in mice treated with SAM-6 antibody is dose dependent. Already mice treated with 50µg SAM-6 antibody show a clearly reduced tumor volume compared with the control groups. Animals treated with 250µg respectively 500µg SAM-6 antibody had statistical significantly smaller tumor volumes (t-Test, p< 0,05 for both concentrations) when compared with the IgM control. However animals treated with 750µg SAM-6 antibody show no significant reduction in tumor volume.

## Claims

1. An isolated or purified glycoprotein denoted as SAM-6 Receptor (SAM-6/R), wherein said SAM-6/R glycoprotein has an apparent molecular weight in a range of about 80-82 kilodaltons (kDa) as determined by denaturing gel electrophoresis, a sequence comprising SEQ ID NO:1, has at least one nitrogen (N)- or oxygen (O)-linked carbohydrate moiety distinct from Grp78, wherein an antibody denoted SAM-6 specifically binds to the glycoprotein, and wherein treatment of the SAM-6/R glycoprotein with an O-glycosidase reduces binding of the SAM-6 antibody to the glycoprotein, wherein the SAM-6 antibody comprises:
(a) a heavy chain variable region sequence, wherein the heavy chain variable region sequence comprises the amino acid sequence shown in SEQ ID NO:15, and
(b) a light chain variable region sequence, wherein the light chain variable region sequence comprises the amino acid sequence shown in SEQ ID NO:13.

2. The isolated or purified glycoprotein of claim 1, wherein treatment of the SAM-6/R glycoprotein with an O-glycosidase reduces binding of an antibody denoted SAM-6 to the glycoprotein.

3. The glycoprotein of claim 1 or 2, wherein the carbohydrate moiety is linked to an asparagine, serine or threonine residue of SEQ ID NO:1.

4. The glycoprotein of claim 1 or 2, wherein the SAM-6 antibody as defined in claim 1 binds to a portion of the glycoprotein comprising the N- or O-linked carbohydrate moiety, or to the N- or O-linked carbohydrate moiety.

5. The glycoprotein of claim 1 or 2, wherein the glycoprotein is further characterized as being expressed on a pancreas carcinoma cell line or gastric carcinoma cell line denoted respectively as BXPC-3 (ATCC Deposit No. CRL-1687) or 23132/87 (DSMZ Deposit No. ACC 201).

6. A nucleic acid sequence capable of encoding the glycoprotein of claim 1 or 2, or a subsequence thereof capable of having linked thereto at least one nitrogen (N)- or oxygen (O)-linked carbohydrate moiety distinct from a carbohydrate moiety of Grp78.

7. A nucleic acid sequence that specifically hybridizes to the nucleic acid sequences of claim 6.

8. A host cell transformed with the nucleic acid that encodes the glycoprotein of claim 1 or 2.

9. An isolated or purified antibody or subsequence thereof comprising:
(a) a heavy chain variable region sequence, wherein the heavy chain variable region sequence comprises:
(i) an amino acid sequence which is at least 90% identical to the sequence shown in SEQ ID NO:8, and
(ii) a complementarity determining region (CDR) with 100% identity to the CDR3 (ARDRLAVAGRPFDY) shown in SEQ ID NO:17; and
(b) a light chain variable region sequence, wherein the light chain variable region sequence comprises an amino acid sequence which is at least 90% identical to the sequence shown in SEQ ID NO:13.

10. The isolated or purified antibody or subsequence thereof of claim 9, wherein the three CDRs of the heavy chain variable region comprise or consist of CDR1, SYAMH, CDR2, VISYDGSNKYYADSVKG, and CDR3, ARDRLAVAGRPFDY; and the three CDRs of the light chain variable region comprise or consist of CDR1, SGDKLGDKYAC, CDR2, QDSKRPS, and CDR3, QAWDSSIVV.

11. A pharmaceutical composition comprising the SAM-6/R glycoprotein of claims 1 or 2, or an antibody or subsequence thereof of claim 9 or 10, and a pharmaceutically acceptable carrier or excipient.

12. The antibody or subsequence thereof of any of claims 9 to 11 for use in the treatment of a cellular hyperproliferative disorder.

13. The antibody or subsequence thereof for use of claim 12, wherein the cellular hyperproliferative disorder affects or is present at least in part in brain, head or neck, breast, esophagus, mouth, nasopharynx, nose or sinuses, stomach, duodenum, ileum, jejunum, lung, liver, pancreas, kidney, adrenal gland, thyroid, bladder, colon, rectum, prostate, uterus, cervix, ovary, bone marrow, lymph, blood, bone, testes, skin or muscle, or hematopoetic system.

14. The antibody or subsequence thereof for use of claim 12, wherein the cellular hyperproliferative disorder comprises a neoplasia, tumor or cancer.

15. The antibody or subsequence thereof for use of claim 14, wherein the neoplasia, tumor or cancer comprises a sarcoma, carcinoma, adenocarcinoma, melanoma, myeloma, blastoma, glioma, lymphoma or leukemia.

16. The antibody or subsequence thereof for use of claim 15, wherein the myeloma is multiple myeloma.

## Patentansprüche

1. Als SAM-6-Rezeptor (SAM-6/R) bezeichnetes isoliertes oder aufgereinigtes Glykoprotein, wobei das SAM-6/R-Glykoprotein ein durch denaturierende Gelelektrophorese bestimmtes scheinbares Molekulargewicht im Bereich von etwa 80-82 Kilodalton (kDa) hat, eine SEQ. ID. NR: 1 umfassende Sequenz aufweist, mindestens eine von Grp78 verschiedene, über Stickstoff (N) oder Sauerstoff (O) gebundene Kohlenhydratgruppierung aufweist, wobei ein als SAM-6 bezeichneter Antikörper spezifisch an das Glykoprotein bindet, und wobei durch die Behandlung des SAM-6/R-Glykoproteins mit einer O-Glykosidase die Bindung des SAM-6-Antikörpers an das Glykoprotein vermindert wird, wobei der SAM-6/-Antikörper Folgendes umfasst:
(a) eine Sequenz der variablen Region der schweren Kette, wobei die Sequenz der variablen Region der schweren Kette die in SEQ. ID. NR: 15 gezeigte Aminosäuresequenz umfasst, und
(b) eine Sequenz der variablen Region der leichten Kette, wobei die Sequenz der variablen Region der leichten Kette die in SEQ. ID. NR: 13 gezeigte Aminosäuresequenz umfasst.

2. Isoliertes oder aufgereinigtes Glykoprotein nach Anspruch 1, wobei durch die Behandlung des SAM-6/R-Gykoproteins mit einer O-Glykosidase die Bindung eines als SAM-6 bezeichneten Antikörpers an das Glykoprotein vermindert wird.

3. Glykoprotein nach Anspruch 1 oder 2, wobei die Kohlenhydratgruppierung an einen Asparagin-, Serin- oder Threoninrest von SEQ. ID. NR: 1 gebunden ist.

4. Glykoprotein nach Anspruch 1 oder 2, wobei der wie in Anspruch 1 definierte SAM-6-Antikörper an einen Teil des Glykoproteins bindet, der die über Stickstoff oder Sauerstoff gebundene Kohlenhydratgruppierung umfasst, oder an die über Stickstoff oder Sauerstoff gebundene Kohlenhydratgruppierung.

5. Glykoprotein nach Anspruch 1 oder 2, wobei das Glykoprotein weiterhin **dadurch gekennzeichnet ist, dass** es auf einer Bauchspeicheldrüsenkarzinomzelllinie oder Magenkarzinomzelllinie, die als BXPC-3 (ATCC-Deposit-Nr. CRL-1687) bzw. 23132/87 (DSMZ-Deposit-Nr. ACC 201) bezeichnet wird, exprimiert wird.

6. Nukleinsäuresequenz, die dazu fähig ist, für das Glykoprotein nach Anspruch 1 oder 2 zu codieren, oder eine Untersequenz davon, an die wenigstens eine von einer Kohlenhydratgruppierung von Grp78 verschiedene, über Stickstoff (N) oder Sauerstoff (O) gebundene Kohlenhydratgruppierung gebunden werden kann.

7. Nukleinsäuresequenz, die spezifisch mit den Nukleinsäuresequenzen nach Anspruch 6 hybridisiert.

8. Wirtszelle, transformiert mit der für das Glykoprotein nach Anspruch 1 oder 2 codierenden Nukleinsäure.

9. Isolierter oder aufgereinigter Antikörper oder Untersequenz davon, umfassend:
(a) eine Sequenz der variablen Region der schweren Kette, wobei die Sequenz der variablen Region der schweren Kette Folgendes umfasst:
(i) eine Aminosäuresequenz mit mindestens 90% Identität zu der in SEQ. ID. NR: 18 gezeigten Sequenz, und
(ii) eine Antigenbindungsstelle (CDR) mit 100% Identität zu der in SEQ. ID. NR: 17 gezeigten CDR3 (ARDRLAVAGRPFDY), und
(b) eine Sequenz der variablen Region der leichten Kette, worin die Sequenz der variablen Region der leichten Kette eine Aminosäuresequenz mit mindestens 90% Identität zu der in SEQ. ID. NR: 13 gezeigten Sequenz umfasst.

10. Isolierter oder aufgereinigter Antikörper oder Untersequenz davon nach Anspruch 9, wobei die drei CDRs der variablen Region der schweren Kette CDR1, SYAMH, CDR2, VISYDGSNKYYADSVKG und CDR3, ARDRLAVAGRPFDY umfassen bzw. daraus bestehen und die drei CDRs der variablen Region der leichten Kette CDR1, SGDKLGDKYAC, CDR2, QDSKRPS und CDR3, QAWDSSIVV umfassen bzw. daraus bestehen.

11. Pharmazeutische Zusammensetzung, umfassend das SAM-6/R-Glykoprotein nach Anspruch 1 oder 2 oder einen Antikörper oder eine Untersequenz davon nach Anspruch 9 oder 10 und einen pharmazeutisch unbedenklichen Träger oder Exzipienten.

12. Antikörper oder Untersequenz davon nach einem der Ansprüche 9 bis 11 zur Verwendung bei der Behandlung einer zellulären hyperproliferativen Erkrankung.

13. Antikörper oder Untersequenz davon zur Verwendung nach Anspruch 12, wobei die zelluläre hyperproliferative Erkrankung mindestens teilweise das Gehirn, den Hals, bzw. Nacken, die Brust, die Speiseröhre, den Mund, den Nasopharynx, die Nase oder die Nasennebenhöhlen, den Magen, den Zwölffingerdarm, das Ileum, das Jejunum, die Lunge, die Leber, die Bauchspeicheldrüse, die Niere, die Nebenniere, die Schilddrüse, die Blase, den Dickdarm, das Rektum, die Prostata, die Gebärmutter, den Gebärmutterhals, den Eierstock, das Knochenmark, die Lymphe, das Blut, die Knochen, die Hoden, Haut oder Muskel oder das blutbildende System betrifft bzw. darin vorhanden ist.

14. Antikörper oder Untersequenz davon zur Verwendung nach Anspruch 12, wobei die zelluläre hyperproliferative Erkrankung eine Neoplasie, einen Tumor oder Krebs umfasst.

15. Antikörper oder Untersequenz davon zur Verwendung nach Anspruch 14, wobei die Neoplasie, der Tumor oder der Krebs ein Sarkom, Karzinom, Adenokarzinom, Melanom, Myelom, Blastom, Gliom, Lymphom oder Leukämie umfasst.

16. Antikörper oder Untersequenz davon zur Verwendung nach Anspruch 15, wobei es sich bei dem Myelom um ein multiples Myelom handelt.

## Revendications

1. Glycoprotéine isolée ou purifiée, désignée sous le nom de Récepteur SAM-6 (SAM-6/R), ladite glycoprotéine SAM-6/R ayant une masse moléculaire apparente comprise dans la plage d'environ 80-82 kilodaltons (kDa) telle que déterminée par électrophorèse sur gel dénaturant, une séquence comprenant SEQ ID NO:1 ayant un fragment hydrate de carbone lié à l'azote (N) ou à l'oxygène (O), distinct de Grp78, un anticorps désigné sous le nom de SAM-6 se liant d'une manière spécifique à la glycoprotéine, et un traitement de la glycoprotéine SAM-6/R avec une O-glycosidase réduisant la liaison de l'anticorps SAM-6 à la glycoprotéine, l'anticorps SAM-6 comprenant :
(a) une séquence de la région variable de la chaîne lourde, la séquence de la région variable de la chaîne lourde comprenant la séquence d'acides aminés présentée dans SEQ ID NO:15, et
(b) une séquence de la région variable de la chaîne légère, la séquence de la région variable de la chaîne légère comprenant la séquence d'acides aminés présentée dans SEQ ID NO:13.

2. Glycoprotéine purifiée ou isolée selon la revendication 1, le traitement de la Glycoprotéine SAM-6/R avec une O-glycosidase réduisant la liaison d'un anticorps désigné sous le nom de SAM-6 à la glycoprotéine.

3. Glycoprotéine selon la revendication 1 ou 2, dans laquelle le fragment hydrate de carbone est lié à un résidu asparagine, sérine ou thréonine de SEQ ID NO:1.

4. Glycoprotéine selon la revendication 1 ou 2, dans laquelle l'anticorps SAM-6 tel que défini dans la revendication 1 se lie à une portion de la glycoprotéine comprenant le fragment hydrate de carbone lié à N ou à O, ou au fragment hydrate de carbone lié à N ou à O.

5. Glycoprotéine selon la revendication 1 ou 2, la glycoprotéine étant en outre caractérisée comme étant exprimée sur une lignée de cellules de carcinome pancréatique ou à une lignée cellulaire de carcinome gastrique, désignées respectivement sous les noms de BXPC-3 (Numéro de Dépôt ATCC CRL-1687) ou 23132/87 (Numéro de Dépôt DSMZ ACC 201).

6. Séquence d'acide nucléique capable de coder la glycoprotéine de la revendication 1 ou 2, ou sous-séquence de celle-là, capable d'avoir au moins un fragment hydrate de carbone lié à l'azote (N) ou à l'oxygène (O), distinct d'un fragment hydrate de carbone de Grp78, qui y est lié.

7. Séquence d'acide nucléique qui s'hybride spécifiquement aux séquences d'acides nucléiques de la revendication 6.

8. Cellule hôte transformée avec l'acide nucléique qui code pour la glycoprotéine de la revendication 1 ou 2.

9. Anticorps isolé ou purifié, ou sous-séquence de ce dernier, comprenant :
(a) une séquence de la région variable de la chaîne lourde, la séquence de la région variable de la chaîne lourde comprenant :
(i) une séquence d'acides aminés qui a une identité d'au moins 90 % avec la séquence présentée dans SEQ ID NO:18, et
(ii) une région déterminant la complémentarité (CDR) ayant une identité de 100 % avec la CDR3 (ARDRLAVAGRPFDY) présentée dans SEQ ID NO:17 ; et
(b) une séquence de la région variable de la chaîne légère, la séquence de la région variable de la chaîne légère comprenant une séquence d'acides aminés qui a une identité d'au moins 90 % avec la séquence présentée dans SEQ ID NO:13.

10. Anticorps isolé ou purifié ou sous-séquence de ce dernier selon la revendication 9, dans lequel les trois CDR de la région variable de la chaîne lourde comprennent CDR1, SYAMH, CDR2, VISYDGSNKYYADSVKG, et CDR3, ARDRLAVAGRPFDY ou consiste en ces derniers ; et les trois CDR de la région variable de la chaîne légère consistent en CDR1, SGDKLGDKYAC, CDR2, QDSKRPS, et CDR3, QAWDSSIVV.

11. Composition pharmaceutique comprenant la glycoprotéine SAM-6/R des revendications 1 ou 2, ou un anticorps ou une sous-séquence de ce dernier de la revendication 9 ou 10, et un support ou un excipient pharmaceutiquement acceptable.

12. Anticorps ou sous-séquence de ce dernier selon l'une quelconque des revendications 9 à 11 pour utilisation dans le traitement d'un trouble hyperprolifératif cellulaire.

13. Anticorps ou sous-séquence de ce dernier pour utilisation selon la revendication 12, le trouble hyperprolifératif cellulaire affectant au moins en partie le cerveau, la tête ou le cou, le sein, l'oesophage, la bouche, le nasopharynx, le nez ou les sinus, l'estomac, le duodénum, l'iléon, le jéjunum, le poumon, le foie, le pancréas, le rein, la glande surrénale, la thyroïde, la vessie, le côlon, le rectum, la prostate, l'utérus, le col de l'utérus, les ovaires, la moelle osseuse, la lymphe, le sang, les os, les testicules, la peau ou les muscles, ou le système hématopoïétique, ou y est présent.

14. Anticorps ou sous-séquence de ce dernier pour utilisation selon la revendication 12, le trouble hyperprolifératif cellulaire comprenant une néoplasie, une tumeur ou un cancer.

15. Anticorps ou sous-séquence de ce dernier pour utilisation selon la revendication 14, la néoplasie, la tumeur ou le cancer comprenant un sarcome, un carcinome, un adénocarcinome, un mélanome, un myélome, un blastome, un gliome, un lymphome ou une leucémie.

16. Anticorps ou sous-séquence de ce dernier pour utilisation selon la revendication 15, le myélome étant un myélome multiple.
